# EUROPEAN PATENT SPECIFICATION

(11) **EP 2 240 576 B1**
(45) Date of publication and mention of the grant of the patent: **03.10.2012**
(21) Application number: 09701217.3
(22) Date of filing: 09.01.2009
(51) Int. Cl.: C12N 9/12, C07K 14/195, C12N 15/62, C12Q 1/68

(54) **CREN7 CHIMERIC PROTEIN**
CHIMÄRES CREN7-PROTEIN
PROTÉINE CHIMÉRIQUE CREN7

(30) Priority: 11.01.2008 US 10812 P
(43) Date of publication of application: 20.10.2010
(73) Proprietor: Genesys Ltd, Surrey GU16 7PL (GB)
(72) Inventor: CLARK, Duncan, Roy, Camberley Surrey GU16 7PL (GB); WILKINSON, Martin, Camberley Surrey GU16 7PL (GB); MORANT, Nicholas, Camberley Surrey GU16 7PL (GB)
(74) Representative: Turner, Rhiannon Rosalind
(86) International application number: PCT/GB2009/000063
(87) International publication number: WO 2009/087394

(56) References cited:
- WO-A-01/92501
- WO-A-03/046149
- WO-A-2006/074233
- WO-A-2007/011891
- WANG Y ET AL: "A novel strategy to engineer DNA polymerases for enhanced processivity and improved performance in vitro" NUCLEIC ACIDS RESEARCH, OXFORD UNIVERSITY PRESS, SURREY, GB, vol. 32, no. 3, 1 January 2004 (2004-01-01), pages 1197-1207, XP002303419 ISSN: 0305-1048 cited in the application
- GUO LI ET AL: "Biochemical and structural characterization of Cren7, a novel chromatin protein conserved among Crenarchaea" NUCLEIC ACIDS RESEARCH, vol. 36, no. 4, 20 December 2007 (2007-12-20), pages 1129-1137, XP002519039 ISSN: 0305-1048 cited in the application
- CHRISTINE D HARDY ET AL: "Biochemical characterization of DNA-binding proteins from Pyrobaculum aerophilum and Aeropyrum pernix" EXTREMOPHILES ; LIFE UNDER EXTREME CONDITIONS, SPRINGER-VERLAG, TO, vol. 12, no. 2, 7 December 2007 (2007-12-07), pages 235-246, XP019593081 ISSN: 1433-4909
- DATABASE UniProt [Online] 20 May 2008 (2008-05-20), "RecName: Full=Chromatin protein Cren7;" XP002519040 retrieved from EBI accession no. UNIPROT:B1YCQ5 Database accession no. B1YCQ5
- DATABASE UniProt [Online] 22 July 2008 (2008-07-22), "RecName: Full=Chromatin protein Cren7;" XP002519041 retrieved from EBI accession no. UNIPROT:A8MCT2 Database accession no. A8MCT2
- DATABASE UniProt [Online] 22 July 2008 (2008-07-22), "RecName: Full=Chromatin protein Cren7;" XP002519042 retrieved from EBI accession no. UNIPROT:P0C836 Database accession no. P0C836
- ZHANG ZHENFENG ET AL: "Structural insights into the interaction of the crenarchaeal chromatin protein Cren7 with DNA", MOLECULAR MICROBIOLOGY, vol. 76, no. 3, May 2010 (2010-05), pages 749-759, ISSN: 0950-382X

## Description

### Field of invention

The present invention relates to improved nucleic acid modifying enzymes such as nucleic acid polymerases, and their use.

### Background

Nucleic acid modifying enzymes, especially thermostable DNA polymerases, have become an important tool for the molecular biologist. Various approaches for enhancing the activity of naturally-found DNA polymerases have been reported. For example, Motz et al. (2002, J. Biol. Chem. 277: 16179-16188) found that a proliferating cell nuclear antigen homologue from *Archaeoglobus fulgidus,* when fused to the classical PCR enzyme *Taq* DNA polymerase from *Thermus aquaticus,* stimulated processivity of the DNA polymerase. Davidson et al. (2003, Nucleic Acids Res. 31: 4702-4709) inserted the T3 DNA polymerase thioredoxin binding domain into the distantly related *Taq* DNA polymerase and showed that this improved the processivity and fidelity of the *Taq* DNA polymerase. Wang et al. (2004, Nucleic Acids Res. 32: -1197-1207; see also WO01/9250 WO2004/037979 and US2007/0141591) improved the processivity of DNA polymerases (or mutants thereof) by fusing the polymerase domain to a double-stranded DNA binding protein Sso7d from *Sulfolobus solfataricus,* Sso7d-like proteins, or mutants thereof. Meanwhile, Pavlov et al. (2002, Proc. Natl. Acad. Sci. USA 99: 13510-13515) taught that fusion of a number of helix-hairpin-helix motifs derived from DNA topoisomerase V to each of various DNA polymerases conferred salt resistance and increased processivity.

The present invention provides an alternative approach to enhancing nucleic acid modifying enzyme activity.

### Summary of invention

According to the present invention there is provided a chimeric protein comprising or consisting essentially of a nucleic acid modifying enzyme domain having nucleic acid modifying activity joined with a Cren7 enhancer domain or a variant thereof, in which the Cren7 enhancer domain enhances the activity of the nucleic acid modifying enzyme domain compared with a corresponding protein lacking the Cren7 enhancer domain (i.e., an identical protein comprising the nucleic acid modifying enzyme domain but lacking the Cren7 enhancer domain), wherein the nucleic acid modifying ezyme is a nucleic acid polymerase.

The Cren7 protein family is highly conserved in Crenarchael organisms, as suggested recently in Guo et al. (2007, Nucleic Acids Research Advance Access published 20 December 2007, 1-9). These workers suggested that the protein of SEQ ID NO: 1 below (labelled by them as "Cren7") is a putative major chromatin protein with a function in DNA supercoiling and compaction. The inventors have surprisingly found that this domain is useful to enhance the properties of a nucleic acid modifying enzyme domain and there was no suggestion of this in the publication by Guo *et al.*

This domain classification has been adopted as standard in the art for use with databases of protein sequences, such that the skilled person is able to search such databases, using a known Cren7 sequence such as SEQ ID NO:1 (for example by means of a BLASTP homology search) to determine whether a given organism expresses or is capable of expressing a Cren7 protein. See, for example:
http://expasy.org/cgi-bin/get-similar?name=Cren7%20family;
http://www.uniprot.org/uniprot/?query=family:%22Cren7+family%22; or
http://www.uniprot.org/uniprodQ97ZE3 (all accessed on 6 January 2009).

Therefore, according to the present invention, the Cren7 enhancer domain is preferably a Cren7 enhancer protein from a Crenarchaeal organism (i.e., an organism from the Phylum *Crenarchaeota),* which may be selected from an organism within the Class *Thermoprotei.* The organism may be in the Order *Thermoproteales* (such as *Caldivirga maquilingensis; Pyrobaculum islandicum; Pyrobaculum arsenaticum; Pyrobaculum aerophilum; Pyrobaculum calidifontis;* and *Thermoproteus neutrophilus),* or within the Order *Sulfolobales* (such as *Sulfolobus solfataricus; Sulfolobus acidocaldarius; Sulfolobus shibatae; Sulfolobus tokodaii;* and *Metallosphaera sedula)* or within the Order Desulfurococcales (such as *Staphylothermus marinus; Hyperthermus butylicus; Aeropyrum pernix;* and *Ignicoccus hospitalis).*

A variant of the Cren7 enhancer domain may be a structural variant, for example, having a percentage sequence similarity to a known Cren7 enhancer protein as set out in Tables 1 and 2 below, determined using the MatGAT program and the alignment matrix BLOSUM50 (Table 1) or BLOSUM62 (Table 2). MatGAT is a type of sequence comparison software which does not rely on comparison to a single sequence but can compare all similar proteins within a specified group. The software has been described by Campanella et al. (BMC Bioinformatics (2003) 4: 29) and is available at http://www.bitincka.com/ledion/matgat/ (as accessed on 6 January 2009). For example, a Cren7 enhancer domain may have at least 29% sequence identity to SEQ ID NO:13, determined using the MatGAT program and the alignment matrix BLOSUM62 (see Table 2), or at least 33% sequence identity to SEQ ID NO:1 determined using the MatGAT program and the alignment matrix BLOSUM50 (see Table 1). When using either alignment matrix as referred to throughout this specification, the default program parameters were used, namely, First Gap 12, Extending Gap 2.

Alternatively, a variant of the Cren7 enhancer domain may be a structural variant, for example, having at least 35% sequence identity to the *Sulfolobus solfataricus* Cren7 enhancer protein (SEQ ID NO: 1) when determined using BLASTP with SEQ ID NO:1 as the base sequence. A Cren7 enhancer domain may, for example, have at least 40%, 45%, 50%, 55%, 60%, 65%, 70%, 75%, 80%, 85%, 90%, 95%, 96%, 97%, 98% or even 99% sequence identity, to the *Sulfolobus solfataricus* Cren7 enhancer protein (SEQ ID NO: 1), when determined using BLASTP with SEQ ID NO:1 as the base sequence. This means that SEQ ID NO:1 is the sequence against which the percentage identity is determined. The BLAST software is publicly available at http://blast.ncbi.nlm.nih.govBlast.cgi (accessible on 6 January 2009). Different levels of percentage identity may be determined when using the BLASTP software if a sequence other than SEQ ID NO:1 is used as the base sequence.

The variant of the Cren7 enhancer domain used in the invention is a functional variant having at leart 40% sequence identity with the Cren7 enhancer protein of SEQ ID NO:1.

**Table 1: MatGAT comparison of sequences SEQ ID NO:1-14, 59 & 60**

| (BLOSUM50 alignment matrix) Light grey shows % identity; dark grey shows % similarity. | | | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| 1 | P_islandicum (SEQ ID N0:10) | | 78.3 | 65.6 | 81.7 | 35.9 | 33.9 | 33.9 | 39.1 | 42.2 | 37.5 | 31.7 | 78.3 | 30.6 | 37.5 | 39.7 | 57.1 |
| 2 | P_arsenaticum (SEQ ID NO:11) | 96.6 | | 73.0 | 81.4 | 35.9 | 38.1 | 38.1 | 35.5 | 39.3 | 33.9 | 32.8 | 86.4 | 35.9 | 33.9 | 40.6 | 51.6 |
| 3 | P_calidifontis (SEQ ID NO:13) | 84.1 | 87.3 | | 74.6 | 34.3 | 32.8 | 32.8 | 36.9 | 36.4 | 34.8 | 32.4 | 73.0 | 34.3 | 34.8 | 38.5 | 54.0 |
| 4 | P_aerophitum (SEQ ID NO:12) | 94.9 | 96.6 | 88.9 | | 34.4 | 36.5 | 36.5 | 33.9 | 38.7 | 33.9 | 29.7 | 81.4 | 35.9 | 33.9 | 37.7 | 54.8 |
| 5 | A_pemix (SEQ ID NO:7) | 48.4 | 48.4 | 43.8 | 48.4 | | 78.1 | 79.7 | 59.4 | 64.1 | 65.6 | 45.5 | 32.8 | 65.6 | 64.1 | 64.1 | 35.8 |
| 6 | H_buytlicus_0818 (SEQ ID NO:5) | 45.2 | 48.4 | 44.4 | 48.4 | 89.1 | | 98.4 | 66.1 | 67.7 | 74.6 | 50.0 | 34.9 | 76.2 | 73.0 | 74.2 | 39.7 |
| 7 | H_buytlicus 1128 (SEQ ID NO:6) | 45.2 | 48.4 | 44.4 | 48.4 | 89.1 | 100.0 | | 64.5 | 66.1 | 73.0 | 48.4 | 34.9 | 74.6 | 71.4 | 72.6 | 38.2 |
| 8 | M_sedula (SEQ ID NO:3) | 55.9 | 54.2 | 46.0 | 54.2 | 73.4 | 75.8 | 75.8 | | 83.1 | 81.7 | 43.8 | 35.5 | 66.1 | 83.3 | 84.7 | 38.5 |
| 9 | S_acidocaldarius (SEQ ID NO:2) | 54.2 | 52.5 | 47.6 | 54.2 | 78.1 | 79.0 | 79.0 | 19.1 | | 80.0 | 44.4 | 39.3 | 62.9 | 80.0 | 83.1 | 40.0 |
| 10 | S_solfataricus (SEQ ID NO:1) | 51.7 | 50.0 | 42.9 | 50.0 | 75.0 | 82.3 | 82.3 | 88.3 | 86.7 | | 43.8 | 33.9 | 72.6 | 98.3 | 85.0 | 38.5 |
| 11 | I_hospitalis (SEQ ID NO:9) | 52.5 | 50.8 | 44.4 | 49.2 | 57.8 | 59.7 | 59.7 | 55.9 | 61.0 | 53.3 | | 31.3 | 43.8 | 41.5 | 46.0 | 38.8 |
| 12 | T_neutrophilus (SEQ ID NO:14) | 93.2 | 94.9 | 85.7 | 94.9 | 45.3 | 45.2 | 45.2 | 52.5 | 50.8 | 48.3 | 45.8 | | 31.3 | 33.9 | 39.3 | 54.8 |
| 13 | S_marinus (SEQ ID NO:4) | 41.9 | 45.2 | 42.9 | 46.8 | 79.7 | 87.1 | 87.1 | 79.0 | 80.6 | 85.5 | 53.2 | 45.2 | | 74.2 | 72.6 | 36.4 |
| 14 | S_shibatae (SEQ ID NO:59) | 51.1 | 50.0 | 42.9 | 50.0 | 73.4 | 80.6 | 80.6 | 90.0 | 86.7 | 98.3 | 51.7 | 48.3 | 87.1 | | 85.0 | 38.5 |
| 15 | S_tokodaii (SEQ ID NO:60) | 55.9 | 57.6 | 47.6 | 54.2 | 78.1 | 79.0 | 79.0 | 88.1 | 89.8 | 90.0 | 57.9 | 54.2 | 80.6 | 90.0 | | 43.8 |
| 16 | C_maquilingensis (SEQ ID NO:8) | 73.8 | 73.8 | 71.4 | 75.4 | 53.1 | 51.6 | 51.6 | 52.5 | 52.5 | 50.8 | 57.4 | 73.8 | 46.8 | 50.8 | 54.1 | |

**Table 2: MatGAT comparison of sequences SEQ ID NO:1-14, 59 & 60**

| (BLOSUM62 alignment matrix) Light grey shows % identity; dark grey shows % similarity. | | | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| 1 | P_islandicum (SEQ ID NO:10) | | 78.3 | 65.6 | 81.7 | 35.9 | 33.9 | 33.9 | 36.7 | 40.0 | 33.3 | 31.7 | 78.3 | 30.6 | 33.3 | 39.7 | 57.1 |
| 2 | P_arsenaticum (SEQ ID N0:11) | 96.6 | | 73.0 | 81.4 | 34.4 | 36.5 | 38.5 | 35.5 | 39.3 | 33.9 | 32.8 | 86.4 | 34.9 | 33.9 | 36.7 | 51.6 |
| 3 | P_calidifontis (SEQ ID NO:13) | 84.1 | 87.3 | | 74.6 | 31.3 | 29.9 | 29.9 | 36.9 | 33.8 | 34.8 | 30.9 | 73.0 | 29.9 | 34.8 | 35.9 | 54.0 |
| 4 | P_aerophilum (SEO ID NO:12) | 94.9 | 96.6 | 87.3 | | 31.3 | 33.3 | 33.3 | 35.5 | 38.7 | 33.9 | 31.3 | 81.4 | 31.7 | 33.9 | 36.7 | 54.8 |
| 5 | A_pemix (SEQ ID NO:7) | 48.4 | 46.9 | 42.2 | 46.9 | | 78.1 | 79.7 | 59.4 | 64.1 | 65.6 | 43.9 | 32.8 | 65.6 | 64.1 | 64.1 | 34.8 |
| 6 | H_buytlicus_0878 (SEQ ID NO:5) | 45.2 | 46,8 | 42.9 | 46.8 | 89.1 | | 98.4 | 66.1 | 67.7 | 74.6 | 48.4 | 34.9 | 76.2 | 73.0 | 74.2 | 37.9 |
| 7 | H_buyflicus_1128 (SEQ ID NO:6) | 45.2 | 46.8 | 42.9 | 46.8 | 89.1 | 100.0 | | 64.5 | 66.1 | 73.0 | 46.9 | 34.9 | 74.6 | 71.4 | 72.6 | 36.4 |
| 8 | M_sedula (SEQ ID NO:3) | 49.2 | 54.2 | 46.0 | 52.5 | 73.4 | 75.8 | 75.8 | | 83.1 | 81.7 | 42.9 | 37.1 | 66.1 | 83.3 | 84.7 | 37.5 |
| 9 | S_acidocaldarius (SEQ ID NO:2) | 49.2 | 52.5 | 44.4 | 52.5 | 78.1 | 79.0 | 79.0 | 88.8 | | 80.0 | 44.4 | 41.0 | 62.9 | 80.0 | 83.1 | 37.5 |
| 10 | S_solfataricus (SEQ ID NO:1) | 41.7 | 50.0 | 42.9 | 50.0 | 75.0 | 82.3 | 82.3 | 88.3 | 86.7 | | 42.2 | 34.4 | 72.6 | 98,3 | 85.0 | 38.5 |
| 11 | I_hospitalis (SEQ ID NO:9) | 52.5 | 50.8 | 41.3 | 47.5 | 56.3 | 58.1 | 58.1 | 54.2 | 61.0 | 51.7 | | 31.3 | 43.8 | 42.2 | 43.5 | 35.8 |
| 12 | T_neutrophilus (SEQ ID NO:14) | 93.2 | 94.9 | 85.7 | 94.9 | 45.3 | 45.2 | 45.2 | 52.5 | 50.8 | 45.0 | 45.8 | | 30.2 | 33.9 | 38.3 | 54.8 |
| 13 | S_marinus (SEQ ID NO:4) | 41.9 | 41.9 | 42.9 | 41.9 | 79.7 | 87.1 | 87.1 | 79.0 | 80.6 | 85.5 | 53.2 | 41.9 | | 74.2 | 72.6 | 36.4 |
| 14 | S_shibatae (SEQ ID NO:59) | 41.7 | 50.0 | 42.9 | 50.0 | 73.4 | 80.6 | 80.6 | 90.0 | 86.7 | 98.3 | 51.7 | 48.3 | 87.1 | | 85.0 | 38.5 |
| 15 | S_tokodaii (SEQ ID NO:60) | 55.9 | 50.8 | 46.0 | 50.8 | 78.1 | 79.0 | 79.0 | 88.1 | 89.8 | 90.0 | 52.6 | 50.8 | 80.6 | 90.0 | | 43.8 |
| 16 | C_maquilingensis (SEQ ID NO:8) | 73.8 | 73.8 | 71.4 | 75.4 | 50.0 | 46.8 | 46.8 | 45.9 | 50.8 | 49.2 | 54.1 | 73.8 | 45.2 | 49.2 | 52.5 | |

The chimeric protein may comprise one, two or more Cren7 enhancer domains or variants thereof; where there are two or more, each may be the same or different to one or more other Cren7 enhancer domains or variants thereof comprised within the same chimeric protein.

The *Sulfolobus solfataricus* Cren7 enhancer protein has the amino acid sequence:
MSSGKKPVKV KTPAGKEAEL VPEKVWALAP KGRKGVKIGL FKDPETGKYF RHKLPDDYP I (SEQ ID NO: 1).

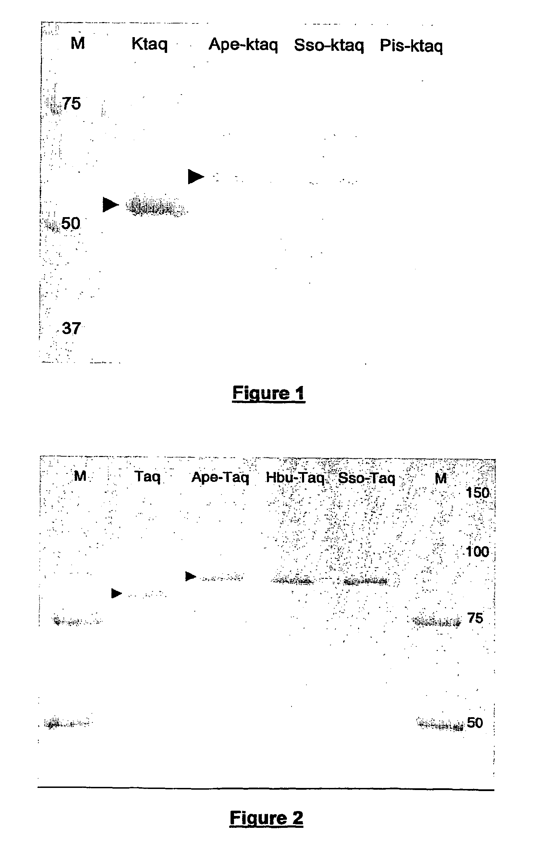

As mentioned above, the present inventors have identified that members of a new class of enhancer domain enhance the activity of nucleic acid modifying enzymes. As already described, these domains have since been designated as "Cren7" domains. These Cren7 enhancer domains are structurally and functionally similar to one another, for example to the *Sulfolobus solfataricus* Cren7 enhancer protein of SEQ ID NO: 1. An illustration of such similarity is set out in Figure 1B of the publication by Guo et al. (2007, Nucleic Acids Research Advance Access published 20 December 2007, 1-9).

In one aspect of the invention, the Cren7 enhancer domain or variant thereof has DNA binding activity which functions to enhance the activity of the nucleic acid modifying enzyme joined therewith. Without being bound by theory, it is considered that the Cren7 enhancer domains of the invention and variants thereof are non-sequence-specific double stranded DNA binding proteins which function to enhance nucleic acid modifying enzymes in a similar way to Sso7d-like proteins (see above). However, the Cren7 enhancer proteins of the present invention, such as the *Sulfolobus solfataricus* Cren7 enhancer protein of SEQ ID NO: 1, as well as variants of such proteins, have no significant amino acid similarity or identity to the known Sso7d-like proteins.

A BLASTP analysis against Sso7d or related protein Sac7d does not identify SEQ ID NO:1 and an analysis against SEQ ID NO:1 does not identify Sso7d or Sac7d, indicating that sequence homology is so low as to be undetectable. Table 3 below shows a MatGAT comparison of Cren7 enhancer domains according to the invention and Sso7d, Sac7d and related protein Ssh7d, demonstrating the low percentage sequence identity of these sequences in comparison with the Cren7 enhancer domains according to the invention. These comparisons were determined using the MatGAT program and the alignment matrix BLOSUM50, with default program parameters used, namely, First Gap 12, Extending Gap 2.

As used herein, the term "chimeric protein" means a protein or polypeptide comprising two or more heterologous domains which are not found in the same relationship to one another in nature.

The domains may be positioned in any arrangement relative to one another. For example, the chimeric protein may comprise a nucleic acid modifying enzyme domain joined at its 3' end to the 5' end of an Cren7 enhancer domain or variant thereof, or the nucleic acid modifying enzyme domain may be joined at its 5' end to the 3' end of an Cren7 enhancer domain or variant thereof. In another embodiment, the chimeric protein may comprise a nucleic acid modifying enzyme domain joined at its 5' end to an Cren7 enhancer domain or variant thereof and at its 3' end to another Cren7 enhancer domain or variant thereof, the Cren7 enhancer domains or variants thereof being the same as or different to one another. The skilled person will appreciate that the chimeric protein may comprise several Cren7 enhancer domains or variants thereof, each of which may the same or different to one or more other Cren7 enhancer domains or variants thereof comprised within the chimeric protein.

The term "joined" means functionally connecting protein domains which have been functionally connected using any method known in the art. By way of non-limiting example, the domains may be recombinantly fused as a single fusion protein, with or without intervening domains or residues, or the domains may be functionally connected by intein-mediated fusion, non-covalent association, and covalent bonding, including disulfide bonding, hydrogen bonding, electrostatic bonding, and conformational bonding such as antibody-antigen or biotin-avidin associations.

**Table 3: MatGAT comparison of sequences SEQ ID NO:1-14, 59 & 60 and Sso7d, Sac7d, Ssh7d**

| (BLOSUM50 alignment matrix) Light grey shows % identity; dark grey shows % similarity. | | | | | | | | | | | | | | | | | | | | |
|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 |
| 1 | P_islandicum (SEQ ID NO:10) | | 78.3 | 65.6 | 81.7 | 35.9 | 33.9 | 33.9 | 36.7 | 40.0 | 33.3 | 31.7 | 78.3 | 30.6 | 33.3 | 39.7 | 57.1 | 13.4 | 13.4 | 10.8 |
| 2 | P_arsenaticum (SEQ ID NO:11) | 96.6 | | 73.0 | 81.4 | 34.4 | 36.5 | 36.5 | 35.5 | 39.3 | 33.9 | 32.8 | 86.4 | 34.9 | 33.9 | 36.7 | 51.6 | 16.4 | 16.4 | 15.6 |
| 3 | P_calidifontis (SEQ ID NO:13) | 84.1 | 87.3 | | 74.6 | 31.3 | 29.9 | 29.9 | 36.9 | 33.8 | 34.8 | 30.9 | 73.0 | 29.9 | 34.8 | 35.9 | 54.0 | 19.1 | 19.1 | 12.3 |
| 4 | P_aerophilum (SEQ ID NO:12) | 94.9 | 96.6 | 87.3 | | 31.3 | 33.3 | 33.3 | 35.5 | 38.7 | 33.9 | 31.3 | 81.4 | 31.7 | 33.9 | 36.7 | 54.8 | 19.4 | 19.4 | 15.6 |
| 5 | A pernix (SEQ ID NO:7) | 48.4 | 46.9 | 42.2 | 46.9 | | 78.1 | 79.7 | 59.4 | 64.1 | 65.6 | 43.9 | 32.8 | 65.6 | 64.1 | 64.1 | 34.8 | 24.7 | 24.7 | 22.7 |
| 6 | H_buytlicus_0878 (SEQ ID NO:5) | 45.2 | 46.8 | 42.9 | 46.8 | 89.1 | | 98.4 | 66.1 | 67.7 | 74.6 | 48.4 | 34.9 | 76.2 | 73.0 | 74.2 | 37.9 | 19.7 | 19.7 | 26.0 |
| 7 | H_buytlicus_1128 (SEQ ID NO:6) | 45.2 | 46.8 | 42.9 | 46.8 | 89.1 | 100.0 | | 64.5 | 66.1 | 73.0 | 46.9 | 34.9 | 74.6 | 71.4 | 72.6 | 36.4 | 19.7 | 19.7 | 26.0 |
| 8 | sedula (SEQ ID NO:3) | 49.2 | 54.2 | 46.0 | 52.5 | 73.4 | 76.8 | 75.8 | | 83.1 | 81.7 | 42.9 | 37.1 | 66.1 | 83.3 | 84.7 | 37.5 | 20.3 | 20.3 | 17.6 |
| 9 | S_acidocaldarius (SEQ ID NO:2) | 49.2 | 52.5 | 44.4 | 52.5 | 78.1 | 79.0 | 79.0 | 89.8 | | 80.0 | 44.4 | 41.0 | 62.9 | 80.0 | 83.1 | 37.5 | 15.9 | 15.9 | 14.7 |
| 10 | S_solfataricus (SEQ ID NO:1) | 41.7 | 50.0 | 42.9 | 50.0 | 75.0 | 82.3 | 82.3 | 88.3 | 86.7 | | 42.2 | 34.4 | 72.6 | 98.3 | 85.0 | 38.6 | 14.7 | 14.7 | 14.9 |
| 11 | I_hospitalis (SEQ ID NO:9) | 52.5 | 50.8 | 41.3 | 47.5 | 56.3 | 58.1 | 58.1 | 54.2 | 61.0 | 51.7 | | 31.3 | 43.8 | 42.2 | 43.5 | 35.8 | 23.9 | 23.9 | 21.5 |
| 21 | T_neutrophilus (SEQ ID NO:14) | 93.2 | 94.9 | 85.7 | 94.9 | 45.3 | 45.2 | 45.2 | 52.5 | 50.8 | 45.0 | 45.8 | | 30.2 | 33.9 | 38.3 | 54.8 | 16.7 | 16.7 | 16.4 |
| 13 | S_marinus (SEQ ID NO:4) | 41.9 | 41.9 | 42.9 | 41.9 | 79.7 | 87.1 | 87.1 | 79.0 | 80.6 | 85.5 | 53.2 | 41.9 | | 74.2 | 72.6 | 36.4 | 20.6 | 20.6 | 26.0 |
| 14 | S_shibatae (SEQ ID NO:59) | 41.7 | 50.0 | 42.9 | 50.0 | 73.4 | 80.6 | 80.6 | 90.0 | 86.7 | 98.3 | 51.7 | 48.3 | 87.1 | | 85.0 | 38.5 | 14.7 | 14.7 | 14.9 |
| 15 | S_tokodaii (SEQ ID NO:60) | 55.9 | 50.8 | 46.0 | 50.8 | 78.1 | 79.0 | 79.0 | 88.1 | 89.8 | 90.0 | 52.6 | 50.8 | 80.6 | 90.0 | | 43.8 | 18.2 | 18.2 | 20.0 |
| 16 | C_maquilingensis (SEQ ID NO:8) | 73.8 | 73.8 | 71.4 | 75.4 | 50.0 | 46.8 | 46.8 | 45.9 | 50.8 | 49.2 | 54.1 | 73.8 | 45.2 | 49.2 | 52.5 | | 16.7 | 16.7 | 17.2 |
| 17 | Sso7d | 37.9 | 34.8 | 39.4 | 34.8 | 40.9 | 31.8 | 31.8 | 28.8 | 31.8 | 27.3 | 33.3 | 30.3 | 30.3 | 27.3 | 30.3 | 37.9 | | 100.0 | 79.1 |
| 18 | Sac7d | 37.9 | 34.8 | 39.4 | 34.8 | 40.9 | 31.8 | 31.8 | 28.8 | 31:8 | 27.3 | 33.3 | 30.3 | 30.3 | 27.3 | 30.3 | 37.9 | 100.0 | | 79.1 |
| 19 | Ssh7d | 37.5 | 34.4 | 35.9 | 34.4 | 42.2 | 37.5 | 37.5 | 35.9 | 39.1 | 34.4 | 32.8 | 35.9 | 39.1 | 34.4 | 34.4 | 40.6 | 87.9 | 87.9 | |

The term "domain" as used herein means a unit of a protein or protein complex with a defined function. Thus, the nucleic acid modifying enzyme domain has nucleic acid modifying activity, whilst the Cren7 enhancer domain or variant thereof enhances the activity of the nucleic acid modifying enzyme. Each domain may comprise a polypeptide sequence or subsequence, or a unit having a plurality of polypeptide sequences where the unit has a defined function.

The term "efficiency" as used herein refers to the ability of the nucleic acid modifying enzyme domain to perform its catalytic function under specific reaction conditions. Typically, "efficiency" as defined herein may be indicated by the amount of modified bases generated by the nucleic acid modifying enzyme domain per binding to a nucleic acid.

"Enhanced" in the context of the nucleic acid modifying enzyme domain means improving the activity of the domain by increasing the amount of enzyme product per unit enzyme per unit time.

The Cren7 enhancer domain of the present invention, or variant thereof, may increase the processivity of the nucleic acid modifying enzyme domain, where the enzyme is a processive enzyme.

"Processivity" as used herein means the ability of the nucleic acid modifying enzyme domain to remain attached to its nucleic acid substrate and perform multiple modification reactions. Improved processivity typically means that the nucleic acid nucleic acid modifying enzyme domain can modify relatively longer tracts of nucleic acid.

The nucleic acid modifying enzyme domain may be a functional domain of a processive enzyme that interacts with nucleic acid, such as a nucleic acid polymerase domain, for example a DNA polymerase domain. Alternatively, the nucleic acid modifying enzyme domain may be an RNA polymerase domain with RNA polymerase activity, a reverse transcriptase domain with reverse transcriptase activity, a methylase domain with methylase activity, a 3' exonuclease domain with 3' exonuclease activity, a gyrase domain with gyrase activity, a topoisomerase domain with topoisomerase activity, or any functional domain of any other processive enzyme that interacts with nucleic acid.

The nucleic acid modifying enzyme domain may be a ligase domain with ligase activity, an alkaline phosphatase domain with alkaline phosphatise activity, or a nucleic acid kinase domain with nucleic acid kinase activity.

As used herein, a "nucleic acid polymerase" refers to any enzyme that catalyzes polynucleotide synthesis by addition of nucleotide units to a nucleotide chain using a nucleic acid such as DNA as a template. The term includes any variants and recombinant functional derivatives of naturally occurring nucleic acid polymerases, whether derived by genetic modification or chemical modification or other methods known in the art.

The Cren7 enhancer domain or variant thereof may, alternatively or additionally for each property, improve one or more of the following properties of a nucleic acid polymerase domain according to the invention: extension time; salt tolerance; amplification efficiency; and amplification fidelity. For example, the chimeric protein may improve salt tolerance compared with nucleic acid modifying enzyme domain alone by about 20-fold, for example up to 10-fold, or 5-fold. Where the nucleic acid modifying enzyme is a nucleic acid polymerase, the Cren7 enhancer domain or variant thereof may, for example, allow amplification by the nucleic acid polymerase domain at a salt concentration equivalent to up to 200mM KCl, such as up to 150mM, 140mM, 130mM or up to 100mM KCl.

Enhancement of the above-mentioned properties of nucleic acid polymerases by the Cren7 enhancer domain or variant thereof provides a substantial improvement and increased application over non-chimeric polymerase. For example, with increased salt tolerance, PCR amplification from low quality of DNA template, such as DNA samples prepared from blood, food and plant sources, becomes more feasible.

The Cren7 enhancer domain of the invention may be one of the group comprising: *Sulfolobus solfataricus* Cren7 enhancer protein (SEQ ID NO: 1); *Sulfolobus acidocaldarius* Cren7 enhancer protein (SEQ ID NO: 2); *Metallosphaera sedula* Cren7 enhancer protein (SEQ ID NO: *3)*; *Staphylothermus marinus* Cren7 enhancer protein (SEQ ID NO: 4); *Hyperthermus butylicus* 0878 Cren7 enhancer protein (SEQ ID NO: *5); Hyperthermus butylicus* 1128 Cren7 enhancer protein (SEQ ID NO: *6)*; *Aeropyrum pernix* Cren7 enhancer protein (SEQ ID NO: 7); *Caldivirga maquilingensis* Cren7 enhancer protein (SEQ ID NO: 8); *Ignicoccus hospitalis* Cren7 enhancer protein (SEQ ID NO: 9); *Pyrobaculum islandicum* Cren7 enhancer protein (SEQ ID NO: 10); *Pyrobaculum arsenaticum* Cren7 enhancer protein (SEQ ID NO: 11); *Pyrobaculum aerophilum* Cren7 enhancer protein (SEQ ID NO: 12); *Pyrobaculum calidifontis* Cren7 enhancer protein (SEQ ID NO: 13); *Thermoproteus neutrophilus* Cren7 enhancer protein (SEQ ID NO: 14), *Sulfolobus shibatae* Cren7 enhancer protein (SEQ ID NO:59); and *Sulfolobus* tokodaii and Cren7 enhancer protein (SEQ ID NO:60). A variant of a Cren7 enhancer domain may be a functional or structural variant having at least 35% sequence identity (for example, at least 40%, 45%, 50%, 55%, 60%, 65%, 70%, 75%, 80%, 85%, 90%, 95%, 96%, 97%, 98% or even 99% sequence identity) with any of the Cren7 enhancer proteins of SEQ ID NO: 1-14, 59 or 60, determined using BLASTP with any of SEQ ID NO:1-14, 59 or 60, as appropriate, used as the base sequence.

In *Hyperthermus butylicus* it has been found that, unusually, two Cren7 proteins are expressed. The numbers used to differentiate the proteins (0878 and 1128) are the ORF (Open reading frame) numbers, as given in the genome, for each of the two copies.

The *Sulfolobus acidocaldarius* Cren7 enhancer protein has the amino acid sequence:

The *Metallosphaera sedula* Cren7 enhancer protein has the amino acid sequence:

The *Staphylothermus marinus* Cren7 enhancer protein has the amino acid sequence:

The *Hyperthermus butylicus* 0878 Cren7 enhancer protein has the amino acid sequence:

The *Hyperthermus butylicus* 1128 Cren7 enhancer protein has the amino acid sequence:

The *Aeropyrum pernix* Cren7 enhancer protein has the amino acid sequence:

The *Caldivirga maquilingensis* Cren7 enhancer protein has the amino acid sequence:

The *Ignicoccus hospitalis* Cren7 enhancer protein has the amino acid sequence:

The *Pyrobaculum islandicum* Cren7 enhancer protein has the amino acid sequence:

The *Pyrobaculum arsenaticum* Cren7 enhancer protein has the amino acid sequence:

The *Pyrobaculum aerophilum* Cren7 enhancer protein has the amino acid sequence:

The *Pyrobaculum calidifontis* Cren7 enhancer protein has the amino acid sequence:

The *Thermoproteus neutrophilus* Cren7 enhancer protein has the amino acid sequence:

The *Sulfolobus shibatae* Cren7 enhance protein has the amino acid sequence:

The *Sulfolobus tokodaii* Cren7 enhance protein has the amino acid sequence:

The majority of the Cren7 enhancer proteins for use in the invention are deemed to be "hypothetical proteins" in the prior art. However, the present application demonstrates that these proteins form a class of proteins with putative DNA binding activity that enhances nucleic acid modifying activity (such as DNA polymerase activity). This is now confirmed by the adoption of the Cren7 domain classification as standard in the art for use with databases of protein sequences, as discussed above.

The Cren7 enhancer domain, or variant thereof, for use in the present invention may comprise the conserved amino acid sequence:
G-X₁ -X₂-X₁ -X₁ -X₃-X₁ -P-X₁ -K-X₄-W-X₁ -L-X₁ -P-X₁-G-X₅-X₁ -G-V-X₁ -X₆-X₇-L-F-X₈-X₁-P-X₉-X₁₀-G-X₁₁-X₁-X₁₂-R-X₁-X₁-X₁₃ (SEQ ID NO: 15). Alternatively, the Cren7 enhancer domain, or variant thereof, of the present invention may comprise the conserved amino acid sequence:
   X₂-X₁-X₁-X₁-X₁₀-G-X₁-X₁-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-X₁₃ (SEQ ID NO:61).

In either case, independently,
X₁ is any amino acid,
X₂ is K, R or E,
X₃ is L or no amino acid,
X₄ is A, V or T,
X₅ is K or R,
X₆ is I or V,
X₇ is G or A,
X₈ is K, R or Q,
X₉ is D, N or E,
X₁₀ is any or no amino acid,
X₁₁ is K or H,
X₁₂ is I, V or F, and
X₁₃ is I, V or L.

In one embodiment, the Cren7 enhancer domain comprises or consists essentially of one of the group comprising: SEQ ID NO:1, SEQ ID NO: 6, SEQ ID NO: 7. A variant of the Cren7 enhancer domain may comprise or consist essentially of a functional or structural variant having at least 40% sequence identity with any of the Cren7 enhancer proteins SEQ ID NO:1, SEQ ID NO:6, SEQ ID NO:7.

The Cren7 enhancer domain or variant thereof of the present invention may have DNA binding activity.

The nucleic acid polymerase domain of the invention may comprise or consist essentially of a thermostable DNA polymerase or a functional mutant, variant or derivative thereof.

As used herein, "thermostable" DNA polymerase means a DNA polymerase which is relatively stable to heat and functions at high temperatures, for example 45-100°C, as compared, for example, to a non-thermostable form of DNA polymerase. For example, a thermostable DNA polymerase derived from thermophilic organisms such as *Pyrococcus furiosus* (Pfu; see for example Lundberg et al., 1991, Gene, 108: 1-6), *Methanococcus jannaschii*, *Archaeoglobus fulgidus* or P. *horikoshii* are more stable and active at elevated temperatures as compared to a DNA polymerase from E. *coli.* Representative thermostable polymerases include Pfu as well as polymerases extracted from the thermophilic bacteria *Thermus flavus, Thermus aquaticus, Thermus brockianus, Thermus ruber*, *Thermus thermophilus, Bacillus stearothermophilus, Thermus lacteus, Thermus rubens*, *Thermotoga maritima*, or from thermophilic archaea *Thermococcus litoralis*, and *Methanothermus fervidus*. Thermostable DNA polymerases for use in the present invention include Taq, KlenTaq, Tne, Tma, Pfu, Tfl, Tth, Stoffel fragment, VENT^{™} DNA polymerase, DEEPVENT^{™} DNA polymerase, KOD, Tgo, JDF3 and the like (see for example US5,436,149; US4,889,818; US4,965,188; US5,079,352; US5,614,365; US5,616,494; US5,374,553; US5,512,462; WO92/06188; WO92/06200; WO96/10640; Engelke et al., 1990, Anal. Biochem. 191: 396-400; Lawyer et al., 1993, PCR Meth. Appl. 2: 275-287; Flaman et al., 1994, Nuc. Acids Res. 22: 3259-3260).

Specific, non-limiting examples of chimeric proteins according to the invention which include an Cren7 enhancer domain and a thermostable DNA polymerase domain are:
(1) a chimeric protein in which the Cren7 enhancer domain from *Aeropyrum pernix* ("Ape") is joined with KlenTaq, Taq or Pfu thermostable DNA polymerase (such as the fusion proteins of SEQ ID NOs 16, 17 and 18, respectively, shown in the experimental section below);
(2) a chimeric protein in which the Cren7 enhancer domain from *Sulfolobus solfataricus* ("Sso") is joined with KlenTaq, Taq or Pfu thermostable DNA polymerase (such as the fusion proteins of SEQ ID NOs: 19, 20 and 21, respectively, shown in the experimental section below);
(3) a chimeric protein in which the Cren7 enhancer domain from *Pyrobaculum islandicum* ("Pis") is joined with KlenTaq (such as the fusion protein of SEQ ID NO: 22 shown in the experimental section below), Taq or Pfu; and
(4) a chimeric protein in which the Cren7 enhancer domain from *Hyperthermus butylicus* ("Hbu") joined with Taq or Pfu thermostable DNA polymerase (such as the fusion proteins of SEQ ID NOs: 23 and 24 respectively, shown in the experimental section below) or KlenTaq.

Alternatively, the nucleic acid polymerase domain of the invention may comprise or consist essentially of a mesophilic DNA polymerase or a functional mutant, variant or derivative thereof.

The mesophilic DNA polymerase may, for example, be T7 DNA polymerase, T5 DNA polymerase, T4 DNA polymerase, Klenow fragment DNA polymerase or DNA polymerase III (see for example US5,270,179; US5,047,342; Barnes, 1992, Gene 112: 29-35).

Alternatively, the nucleic acid polymerase domain of the invention may comprise or consist essentially of intermediate temperature DNA polymerases, namely, those which work optimally within the range 60-70°C, for example, at or around about 65°C. Examples are the polymerases obtainable from *Bacillus* or *Geobacillus* species.

The present invention encompasses variants of the Cren7 enhancer domains and nucleic acid modifying enzyme (for example, polymerase) domains. As used herein, a "variant" means a polypeptide in which the amino acid sequence differs from the base sequence from which it is derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type.

By "conservative substitution" is meant the substitution of an amino acid by another amino acid of the same class, in which the classes are defined as follows:

| Class | Amino acid examples |
|---|---|
| Nonpolar: | A, V, L, I, P, M, F, W |
| Uncharged polar: | G, S, T, C, Y, N, Q |
| Acidic: | D, E |
| Basic: | K, R, H. |

As is well known to those skilled in the art, altering the primary structure of a peptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptide's conformation.

Non-conservative substitutions are possible provided that these do not interrupt or interfere with the function of the DNA binding domain polypeptides.

Broadly speaking, fewer non-conservative substitutions will be possible without altering the biological activity of the polypeptides. Suitably, variants may be structural variants having at least 35% identical, 40% identical, 45% identical, 50% identical, 55% identical, 60% identical, 65% identical, for example at least 70% or 75% identical, such as at least 80%, 85%, 90%, 95%, 96%, 97%, 98% or even 99% identical to the base sequence.

Functional mutants, variants or derivatives of the above-mentioned chimeric proteins are also covered by the present invention. Such mutants, variants or derivatives are considered to be "functional" if they retain the same or similar properties and/or activity to the chimeric proteins of the invention as described herein. For example, where a chimeric protein is a DNA polymerase having a given activity, a variant is considered functional where the level of activity is at least 60%, preferably at least 70%, more preferably at least 80%, yet more preferably 90%, 95%, 96%, 97%, 98%, 99% or 100% that of the chimeric protein. The given activity may be determined by any standard measure, for example (in the case of a DNA polymerase), the number of bases of nucleotides of the template sequence which can be replicated in a given time period. The skilled person is routinely able to determine such properties and activities and examples of such methods are provided in the current specification. Functional mutants of the nucleic acid polymerases may include mutants which have been modified so as to remove other properties or activities not of interest, such as exonuclease activity.

Also provided according to the present invention is a composition comprising the chimeric protein as defined herein. Preferably the composition further comprises at least one component necessary or beneficial to activity of the nucleic acid modifying enzyme domain of the chimeric protein and/or the Cren7 enhancer domain. For example, where the nucleic acid modifying enzyme domain is a DNA polymerase, the composition may comprise a buffer and/or other reagents required to enable a polymerase chain reaction to be carried out.

According to a further aspect of the invention there is provided an isolated nucleic acid encoding the chimeric protein as defined herein.

For example, the nucleic acid may have the sequence of any one of SEQ ID NOs: 25-33 (which encode the chimeric protein of SEQ ID NOs: 16-24, respectively).

Using the standard genetic code, a nucleic acid encoding the polypeptides may readily be conceived and manufactured by the skilled person. The nucleic acid may be DNA or RNA, and where it is a DNA molecule, it may for example comprise a cDNA or genomic DNA.

The invention encompasses variant nucleic acids encoding the chimeric protein. The term "variant" in relation to a nucleic acid sequences means any substitution of, variation of, modification of, replacement of, deletion of, or addition of one or more nucleic acid(s) from or to a polynucleotide sequence, providing the resultant polypeptide sequence encoded by the polynucleotide exhibits at least the same properties as the polypeptide encoded by the basic sequence. The term therefore includes allelic variants and also includes a polynucleotide which substantially hybridises to the polynucleotide sequence of the present invention. Such hybridisation may occur at or between low and high stringency conditions. In general terms, low stringency conditions can be defined as hybridisation in which the washing step takes place in a 0.330-0.825 M NaCl buffer solution at a temperature of about 40-48°C below the calculated or actual melting temperature (Tₘ) of the probe sequence (for example, about ambient laboratory temperature to about 55°C), while high stringency conditions involve a wash in a 0.0165-0.0330 M NaCl buffer solution at a temperature of about 5-10°C below the calculated or actual T*ₘ* of the probe (for example, about 65°C). The buffer solution may, for example, be SSC buffer (0.15M NaCl and 0.015M tri-sodium citrate), with the low stringency wash taking place in 3 x SSC buffer and the high stringency wash taking place in 0.1 x SSC buffer. Steps involved in hybridisation of nucleic acid sequences have been described for example in Sambrook et al. (1989; Molecular Cloning, Cold Spring Harbor Laboratory Press, Cold Spring Harbor).

Typically, variants have 60% or more of the nucleotides in common with the nucleic acid sequence of the present invention, more typically 65%, 70%, 80%, 85%, or even 90%, 95%, 98% or 99% or greater sequence identity. The percentage sequence identity of nucleic acid sequences may be determined using, for example, the BLASTN software, available at http://blast.ncbi.nlm.nih.gov/Blast.cgi (accessible on 6 January 2009).

Variant nucleic acids of the invention may be codon-optimised for expression in a particular host cell.

Chimeric proteins and nucleic acids of the invention may be prepared synthetically using conventional synthesisers. Alternatively, they may be produced using recombinant DNA technology or isolated from natural sources followed by any chemical modification, if required. In these cases, a nucleic acid encoding the chimeric protein is incorporated into a suitable expression vector, which is then used to transform a suitable host cell, such as a prokaryotic cell such as *E*. *coli.* The transformed host cells are cultured and the protein isolated therefrom. Vectors, cells and methods of this type form further aspects of the present invention.

Sequence identity between nucleotide and amino acid sequences can be determined by comparing an alignment of the sequences. When an equivalent position in the compared sequences is occupied by the same amino acid or base, then the molecules are identical at that position. Scoring an alignment as a percentage of identity is a function of the number of identical amino acids or bases at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

Suitable computer programs for carrying out sequence comparisons are widely available in the commercial and public sector. In addition to the BLASTP, BLASTN and MatGAT programs discussed above, further examples include the Gap program (Needleman & Wunsch, 1970, J. Mol. Biol. 48: 443-453) and the FASTA program (Altschul et al., 1990, J. Mol. Biol. 215: 403-410). Gap and FASTA are available as part of the Accelrys GCG Package Version 11.1 (Accelrys, Cambridge, UK), formerly known as the GCG Wisconsin Package. The FASTA program can alternatively be accessed publicly from the European Bioinformatics Institute (http://www.ebi.ac.uk/fasta as accessed on 6 January 2009) and the University of Virginia (http://fasta.biotech.virginia.edu/fasta_www/cgi) or (http://fasta.bioch.virginia.edu/fasta_www2/fasta_list2.shtml as accessed on 6 January 2009). FASTA may be used to search a sequence database with a given sequence or to compare two given sequences (see http://fasta.bioch.virginia.edu/fasta_www/cgi/search_frm2.cgi as accessed on 6 January 2009). Typically, default parameters set by the computer programs should be used when comparing sequences. The default parameters may change depending on the type and length of sequences being compared. A sequence comparison using the FASTA program may use default parameters of Ktup = 2, Scoring matrix = Blosum50, gap = -10 and ext =-2.

The invention also provides a vector comprising the isolated nucleic acid as defined herein and a host cell transformed with such a vector, or transformed with the isolated nucleic acid as defined herein.

The invention also provides a kit comprising the chimeric protein as defined herein, and/or the composition as defined herein, and/or the isolated nucleic acid as defined herein, and/or the vector as defined herein, and/or a host cell transformed as described herein, together with packaging materials therefor.

In a further aspect there is provided a method of modifying a nucleic acid, comprising:
(1) contacting the nucleic acid with the chimeric protein as defined herein under conditions which allow activity of the nucleic acid modifying enzyme (nucleic acid polymerase) domain; and
(2) permitting the nucleic acid modifying enzyme domain to modify the nucleic acid.

A further method according to the invention is one of catalysing the synthesis of a polynucleotide from a target nucleic acid, comprising the steps of:
(1) providing a chimeric protein comprising a nucleic acid polymerase as defined herein; and
(2) contacting the target nucleic acid with the chimeric protein under conditions which allow the addition by the chimeric protein of nucleotide units to a nucleotide chain using the target nucleic acid, thereby synthesising the polynucleotide.

In another aspect of the invention there is provided a method of amplifying a sequence of a target nucleic acid using a thermocycling reaction, comprising the steps of:
(1) contacting the target nucleic acid with a chimeric protein comprising a nucleic acid polymerase as defined herein; and
(2) incubating the target nucleic acid with the chimeric protein under thermocycling reaction conditions which allow amplification of the target nucleic acid.

### Brief Description of Figures

Particular non-limiting embodiments of the present invention will now be described with reference to the following figures, in which:
Figure 1 shows a sodium dodecyl sulphate (SDS) polyacrylamide gel electrophoresis (PAGE) gel in which KlenTaq and various KlenTaq fusion proteins have been separated;
Figure 2 shows an SDS PAGE gel in which Taq and various Taq fusion proteins have been separated;
Figure 3 shows an SDS PAGE gel in which Pfu and various Pfu fusion proteins have been separated;
Figure 4 shows an agarose gel of PCR reaction samples evidencing the amplification efficiency of the Sso enhancer domain-KlenTaq fusion protein ("Sso-ktaq-enhancer") compared with KlenTaq DNA polymerase ("Ktaq") in the presence of differently sized DNA templates as indicated;
Figure 5 shows an agarose gel of PCR reaction samples evidencing the amplification efficiency of the Hbu Cren7 enhancer domain-Pfu fusion protein ("Hbu-Pfu-enhancer") compared with Pfu DNA polymerase ("Pfu") in the presence of differently sized DNA templates as indicated;
Figure 6 shows an agarose gel of PCR reaction samples evidencing the amplification efficiency of Pfu DNA polymerase in the presence of increasing amounts of KCl as shown in mM;
Figure 7 shows an agarose gel of PCR reaction samples evidencing the amplification efficiency of Ape Cren7 enhancer domain-Pfu fusion protein in the presence of increasing amounts of KCl as shown in mM, as compared with the results of Figure 6;
Figure 8 shows an agarose gel of PCR reaction samples evidencing the amplification efficiency of KlenTaq DNA polymerase in the presence of increasing amounts of KCl as shown in mM;
Figure 9 shows an agarose gel of PCR reaction samples evidencing the amplification efficiency of Ape Cren7 enhancer domain-KlenTaq fusion protein in the presence of increasing amounts of KCl as shown in mM, as compared with the results of Figure 8;
Figure 10 shows qPCR results using Taq polymerase (A) and Ape Cren7 enhancer domain-Taq fusion polymerase (B); and
Figure 11 shows qPCR results using Taq polymerase (A), Ape Cren 7 enhancer domain-Taq fusion polymerase (B) and Sso Cren7 enhancer domain-Taq fusion polymerase (C).

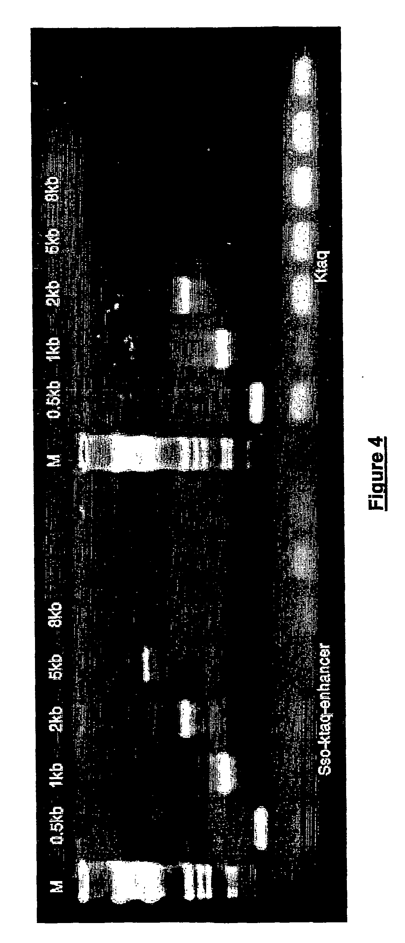

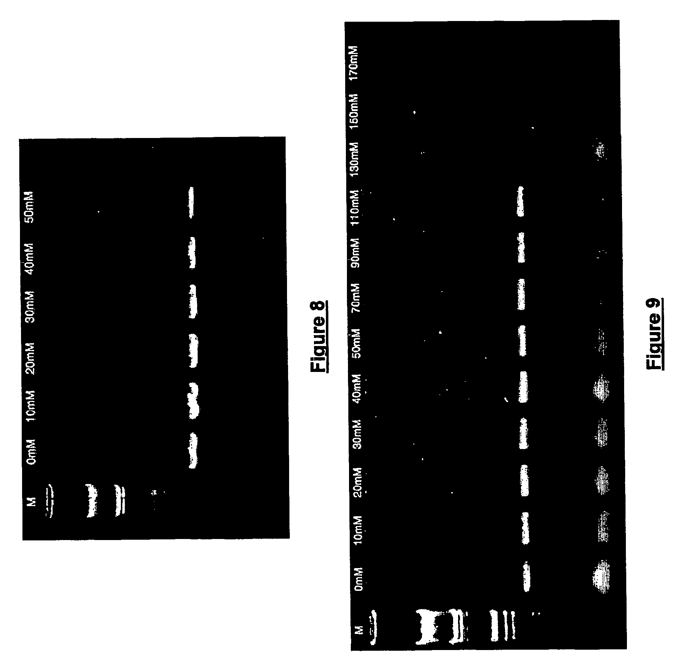

In Figures 1-3, lanes marked "M" represent molecular weight markers, with values given in kDa. In Figures 4-9, lanes marked "M" are DNA molecular weight markers.

### Examples

### 1. Construction of Cren7 enhancer domain-KlenTaq DNA polymerase fusion proteins

The entire *Aeropyrum pernix* ("Ape") Cren7 enhancer domain gene was amplified by PCR from genomic DNA using the following primer set:
*Upper*
   5'-GATATCCATATGAGCCAGAAGCAACTACCA-3'(SEQ ID NO: 34)
*Lower*
   5'-GAATTCCATATGGGTACCCCCGGTCTCGGGGTAGTCGT-3' (SEQ ID NO: 35).

The forward and reverse primers contained an *Nde*I site. The reverse primer in addition coded for a small linker peptide (sequence Gly-Thr-His) between the end of the Cren7 enhancer domain gene and the ATG of the *Nde*I site.

The PCR reactions contained *Pfu* DNA polymerase reaction buffer (20mM Tris-HCl (pH 8.8), 2mM MgSO₄, 10mM KCl, 10mM (NH₄)₂SO₄, 1% Triton^{®} X-100), 200µM each dNTP, 0.5µM forward and reverse primers, 100ng genomic DNA and 0.05u/µl of a mixture of *Taq* and *Pfu* (20:1 ratio) DNA polymerases.

The cycling protocol was 94°C for 60s; 25 cycles of 94°C for 10s and 72°C for 15s.

The entire *Sulfolobus solfataricus* ("Sso") Cren7 enhancer domain gene was similarly amplified by PCR from genomic DNA with the following primer set:
*Uppers*
   5'-GATATCCATATGAGTTCGGGTAAAAAACC-3' (SEQ ID NO: 36)
*Lower*
   5'-GAATTCCATATGGGTACCTATTGGATAATCATCTGGTA-3' (SEQ ID NO: 37).

The entire *Pyrobaculum islandicum* ("Pis") Cren7 enhancer domain gene was similarly amplified by PCR from genomic DNA with the following primer set:
*Upper*
   5'-GATATCCATATGGAAGAGGTCTTAGATCGT-3' (SEQ ID NO: 38)
*Lower*
   5'-GAATTCCATATGGGTAACGCTAGGCGGCAATTTCATTA-3' (SEQ ID NO: 39).

An expression vector pTTQ18KTAQ was obtained by cloning KlenTaq (see US5,616,494 and Barnes, 1992, cited above) into the vector pTTQ18 (Stark, 1987, Gene 51: 255-267) following codon-optimisation of the DNA polymerase using standard techniques for expression in *E*. *coli* cells. The amplified Pis Cren7 enhancer domain genes were digested with *NdeI* and cloned into pTTQ18KTAQ. Ligated DNA was used to transform *E*. *coli* cells TOP10F' (Invitrogen) and transformants plated on a Kanamycin plate. In plasmid mini-prep screening, approximately one out of ten was found to contain the correct size and orientated insert. *E*. *coli* cells carrying a sequenced insert plasmid were induced by addition of IPTG for 4 hours. Cells were lysed by sonication. The clarified lysate was then heat treated at 70°C for 30 min to inactivate the endogenous polymerases.

Cren7 enhancer domain-KlenTaq DNA polymerase fusions ("KlenTaq fusions") were subjected to electrophoresis in an 8% SDS PAGE gel. A major band of about 70kDa was detected as compared to 62kDa for similarly induced KlenTaq DNA polymerase without Cren7 enhancer domain, as shown in Figure 1. This correlates with the predicted molecular weights of around 70kDa for the chimeric protein and around 62kDa for the non-chimeric KlenTaq DNA polymerase.

The Ape Cren7 enhancer domain-KlenTaq fusion protein cloned as described above has following DNA sequence (5'-3'): and a corresponding amino acid sequence:

The Sso Cren7 enhancer domain-KlenTaq fusion protein cloned as described above has the following DNA sequence (5'-3'): and a corresponding amino acid sequence:

The Pis Cren7 enhancer domain-KlenTaq fusion protein cloned as described above has the following DNA sequence (5'-3'): and a corresponding amino acid sequence:

### 2. Construction of Cren7 enhancer domain-Taq DNA polymerase fusion proteins

The entire Ape Cren7 enhancer domain gene was amplified by PCR from genomic DNA using the following primer set:
*Upper*
   5'-GATATCCATATGAGCCAGAAGCAACTACCA-3' (SEQ ID NO: 34)
*Lower*
   5'-ATCCCCGAATTCATGGTAACACCACCCCCGGTCTCGGGGTAGTCG-3' (SEQ ID NO: 40).

The forward primer contained an *Nde*I site and reverse primers contained an *Eco*RI site. The reverse primer, in addition, coded for small linker peptide (sequence Gly-Gly-Val-Thr [SEQ ID NO: 41]) between the end of the Cren7 enhancer domain gene and the G of the EcoR I site.

The PCR reactions contained *Pfu* DNA polymerase reaction buffer, 200µM each dNTP, 0.5µM forward and reverse primers, 100ng genomic DNA and 0.05u/µl of a mixture of *Taq* and *Pfu* (20:1 ratio) DNA polymerases.

The cycling protocol was 94°C for 60s; 25 cycles of 94°C for 10s and 72°C for 15s.

The entire Sso Cren7 enhancer domain gene was similarly amplified by PCR from genomic DNA with the following primer set:
*Upper*
   5'-GATATCCATATGAGTTCGGGTAAAAAACC-3' (SEQ ID NO: 36)
*Lower*
   5'-ATCCCCGAATTCATGGTAACACCACCTATTGGATAATCATCTGGT-3' (SEQ ID NO: 42).

The entire Hbu Cren7 enhancer domain gene 1128 was similarly amplified by PCR from genomic DNA with the following primer set:
*Upper*
   5'-GATATCCATATGGCGTGTGAGAAGCCTGTT-3' (SEQ ID NO: 43)
*Lower*
   5'-ATCCCCGAATTCATGGTAACACCACCGCTGCAGATTGGGTAGTCG-3' SEQ ID NO: 44).

The amplified Cren7 enhancer domain gene was digested with *Nde*I and *Eco*RI cloned into an expression vector, pTTQ18TAQ, which encodes Taq DNA polymerase (see Engelke et al., 1990, cited above, for Taq DNA polymerase sequence). The ligated DNA was used to transform *E. coli* cells TOP10F' and transformants plated on an Ampicillin plate. In plasmid mini-prep screening, approximately eight out of ten were found to contain the correct size insert.

*E. coli* cells carrying sequenced insert plasmid were induced by addition of IPTG for 4hours. Cells were lysed by sonication. The clarified lysate was then heat treated at 70°C for 30 min to inactivate the endogenous polymerases.

In more detail, a single colony was inoculated into 10mls LB (+antibiotic at 50µg/ml) and grown overnight at 37°C, with shaking at 275rpm. 5mls of this primary culture was transferred to a 2 litre capacity shakeflask containing 900mls of TB and 100mls TB salt solution. Antibiotic was added to 50µg/ml. The culture was incubated at 37°C (275rpm) for -4hrs (until a reading of OD₆₀₀1 was reached). IPTG (1mM final) was added to the culture to induce protein expression for 4hrs. Cells were harvested by centrifugation (2000xg for 15mins) and frozen at -80°C.

(Autoclaved Luria Broth (LB): 10g tryptone, 5g yeast extract, 5g NaCl, in 1 Litre dH₂O; Autoclaved Terrific Broth (TB): 12g tryptone, 24g yeast extract, 4mls glycerol in 900mls dH₂O;
Autoclaved TB Salt Solution: 0.17M KH₂PO₄, 0.72M K₂HPO₄)

Cren7 enhancer domain-Taq DNA polymerase fusions ("Taq fusions") were subjected to electrophoresis in a 8% SDS PAGE gel. A major band of about 94kDa was detected as compared to 88kDa for similarly induced non-fusion *Taq* DNA polymerase, as shown in Figure 2. This correlates with the predicted molecular weights of around 101kDa for the chimeric protein and around 93kDa for the non-chimeric *Taq* DNA polymerase; DNA polymerases are known to sometimes run slightly faster than expected on SDS PAGE gels, so that their apparent molecular weight is smaller than predicted.

The Ape Cren7 enhancer domain-Taq fusion protein cloned as described above has the following DNA sequence (5'-3'): and a corresponding amino acid sequence:

The Sso Cren7 enhancer domain-Taq fusion protein cloned as described above has the following nucleotide sequence (5'-3'): and a corresponding amino acid sequence:

The Hbu Cren7 enhancer domain-Taq fusion protein cloned as described above has the following nucleotide sequence (5'-3'): and a corresponding amino acid sequence:

### 3. Construction of Cren7 enhancer domain-Pfu DNA polymerase proteins

The entire Ape Cren7 enhancer domain gene was amplified by PCR from genomic DNA using the following primer set:
*Upper*
   5'-GAATTCGGTACCCATAGCCAGAAGCAACTA-3' (SEQ ID NO: 45)
*Lower*
   5'-GAATTCGTCGACTTACCCGGTCTCGGGGTA-3' (SEQ ID NO: 46).

The forward primer contained a *Kpn*I site and reverse primers contained a stop codon followed by a *Sal*I site.

The PCR reactions contained *Pfu* DNA polymerase reaction buffer, 200µM each dNTP, 0.5µM forward and reverse primers, 100ng genomic DNA and 0.05u/µl of a mixture of *Taq* and *Pfu* (20:1 ratio) DNA polymerases.

The cycling protocol was 94°C for 60s; 25 cycles of 94°C for 10s and 72°C for 15s.

The entire Sso Cren7 enhancer domain gene was similarly amplified by PCR from genomic DNA with the following primer set:
*Upper*
   5'-GAATTCGGTACCCATATGAGTTCGGGTAAA-3' (SEQ ID NO: 47)
*Lower*
   5'-GAATTCGTCGACTTATATTGGATAATCATC-3' (SEQ ID NO: 48).

The entire Hbu Cren7 enhancer domain gene was similarly amplified by PCR from genomic DNA with the following primer set:
*Upper*
   5'-GAATTCGGTACCCATATGGCGTGTGAGAAG-3' (SEQ ID NO: 49)
*Lower*
   5'-GAATTCGTCGACTTAGCTGCAGATTGGGTA-3' (SEQ ID NO: 50).

Plasmid pET21a (Novagen) was used to produce plasmid pET21aPFU carrying the *Pfu* DNA polymerase gene (see Lu & Erickson, 1997, Protein Expr. Purif. 11: 179-184) under the control of the T7 promoter. This pET21aPFU plasmid was modified to introduce a unique restriction site at the 3' end of the *Pfu* gene. The resulting plasmid (pET21aPfuKpn) expresses a *Pfu* polymerase (PfuKpn) with three additional amino acids (Gly-Thr-His) at its C-terminus. No functional difference was observed between *PfuKpn* and commercial *Pfu* polymerase (Stratagene).

The amplified Cren7 enhancer domain genes were digested with *Kpn*I and *Sal*I cloned into the expression vector, pET21aPfuKpn. The ligated DNA was used to transform *E.coli* cells TOP10F' and transformants plated on an Ampicillin plate. In plasmid mini-prep screening, approximately eight out of ten was found to contain the correct size insert for Ape and Sso constructs.

Only one Hbu correct construct was found due to inadvertently missing the presence of two internal *Kpn*I sites within the gene.

Plasmids from single sequenced clones were isolated and transformed into *E*. *coli* BL21(DE3)pLYSS (Novagen; see also Studier et al., 1990, Methods Enzymol. 185: 60-89 and US4,952,496). *E. coli* cells carrying insert plasmid were induced by addition of IPTG for 4hours. Cells were lysed by sonication. The clarified lysate was then heat treated at 70°C for 30 min to inactivate the endogenous polymerases.

In more detail, a single colony was inoculated into 10mls LB (+antibiotic at 50µg/ml) and grown overnight at 37°C, with shaking at 275rpm. 5mls of this primary culture was transferred to a 2 litre capacity shake flask containing 900mls of TB and 100mls TB salt solution. Antibiotic was added to 50ug/ml. The culture was incubated at 37°C (275rpm) for ~4hrs (until a reading of OD₆₀₀1.0 was reached). IPTG (1mM final) was added to the culture to induce protein expression for 4hrs. Cells were harvested by centrifugation (2000xg for 15mins) and frozen at -80°C.

(Autoclaved Luria Broth (LB): 10g tryptone, 5g yeast extract, 5g NaCl, in 1 Litre dH₂O; Autoclaved Terrific Broth (TB): 12g tryptone, 24g yeast extract, 4mls glycerol in 900mls dH₂O;
Autoclaved TB Salt Solution: 0.17M KH₂PO₄, 0.72M K₂HPO₄)

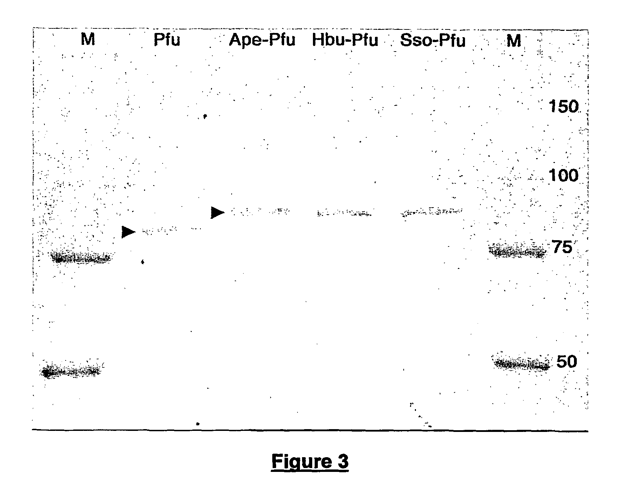

Cren7 enhancer domain-Pfu DNA polymerase fusions ("Pfu fusions") were subjected to electrophoresis in an 8% SDS PAGE gel. A major band of about 96kDa was detected as compared to 88kDa for similarly induced non-fusion *Pfu* DNA polymerase, as shown in Figure 3. This correlates with the predicted molecular weights of around 97kDa for the chimeric protein and around 89kDa for the non-chimeric *Pfu* DNA polymerase; DNA polymerases are known to sometimes run slightly faster than expected on SDS PAGE gels, so that their apparent molecular weight is smaller than predicted.

The Ape Cren7 enhancer domain-Pfu fusion protein cloned as described above has the following nucleotide sequence (5'-3'): and a corresponding amino acid sequence:

The Sso Cren7 enhancer domain-Pfu fusion protein cloned as described above has the following DNA sequence (5'-3'): and a corresponding amino acid sequence:

The Hbu Cren7 enhancer domain-Pfu fusion protein cloned as described above has the following DNA sequence (5'-3'): and a corresponding amino acid sequence:

### 4. Purification of DNA polymerases-Cren7 enhancer domain fusions.

5ml of an overnight culture was inoculated into 1 litre of LB +antibiotic (50µg/ml). After incubation at 37°C until an OD600 of 1.0, IPTG was added to 1mM final concentration to induce the Cren7 enhancer domain-DNA polymerase fusion production. After IPTG induction for 4 hours at 37°C, cells were harvested by centrifugation at 5000rpm for 15 min. Cell pellets were resuspended in lysis buffer. Cells were lysed by sonication. The lysate was then heat treated at 75°C for 30mins, cooled to 4°C and polyethyleneimine added to 1% final concentration. Cell debris, heat denatured proteins and precipitated nucleic acids were removed by centrifugation at 20,000g for 30 min. The solution was then dialysed against 20mM Tris-HCl pH7.5 and 50mM NaCl. The proteins were loaded onto a Heparin Sepharose^{®} column, unbound proteins washed off and the polymerase eluted with a NaCl gradient. Elution of the Cren7 enhancer domain-DNA polymerase fusions occurred at approximately 0.3M NaCl, 0.02M Tris-HCl.

The purified Cren7 enhancer domain-DNA polymerase fusions were subjected to electrophoresis in a 8% SDS PAGE gel. A single band of about 70kDa for each of the corresponding fusions was detected as compared to 62kDa for KlenTaq alone, 89kDa for each of the corresponding fusions as compared to 83kDa for Pfu alone, and 100kDa for each of the corresponding fusions as compared to 94kDa for Taq alone.

### 5. Further method of purification of DNA polymerases-Cren7 enhancer fusions

All steps were performed at 4°C.

1 litre's worth of -80°C frozen cell pastes were resuspended in 50ml of Lysis Buffer (50 mM Trizma, 2mM EDTA, 50mM NaCl, pH 8.0) and made 0.15mg/ml lysozyme). After 30 min at 4°C brought to 100ml total volume with Lysis Buffer in 100ml blue capped bottle. Lysate was sonicated for 2mins to reduce viscosity.

Sodium chloride was added to a final concentration of 0.25M (1.5g) and placed in an 80°C pre-heated water bath and brought to temperature (approx 15mins).

The mixture was held at 80°C for an additional 45 min to precipitate host proteins.

The heat treated lysate was cooled on ice and 10% polyethyleneimine was added to a final concentration of 0.3%.

After overnight incubation, cell debris, heat denatured proteins and polymin P precipitated nucleic acids were pelleted at 10,000rpm for 30 min at 4°C.

5µl loaded onto 8% SDS protein gel.

AmmSO₄ was added to 70% to precipitate the enzyme and left overnight at 4°C. The mixture was centrifuged at 10,000rpm for 30mins, the pellet re-suspended in 10ml buffer and dialysed against same buffer overnight. Any precipitate was removed by centrifugation at 10,000xg for 10 minutes and the supernatant was loaded onto Heparin column in 20mM Trizma (pH 8.0), 1mM EDTA, 0.05% Tween-20, 0.1M NaCl.

After washing with Column Buffer plus 100mM NaCl until the A₂₈₀ returned to background, the enzyme was eluted from the Heparin-Sepharose column using a 10 CV 0-60% linear gradient (0 =100mM NaCl buffer, 100% =1500mM NaCl buffer). The major peak eluting from the affinity column was polymerase. Roughly 60-80 fractions were collected and the column stopped once enzyme eluted.

Each fraction was 3 ml. Aliquots from the fractions were analyzed by SDS-PAGE as indicated in the figures.

Peak fractions were pooled into dialysis tubing, concentrated against solid PEG6000 to 3-4ml then dialysed against Pfe storage buffer; Taq-Cren7 constructs precipitate out if the buffer is not pH 8.5 and 200mM KCl. It was decided to store all Cren7 fusions in Pfe storage buffer.

### 6. Extension time in PCR

This example demonstrates that the Cren7 enhancer domain-DNA polymerase fusions using Sso and Hbu Cren7 enhancer domains amplify larger fragments during PCR compared to unmodified polymerases.

DNA polymerases equivalent to 1.25u of non-fusion DNA polymerases were tested in an extension efficiency assay by PCR of lambda DNA with a variety of primers.

Lambda DNA was used as the template to assess the relative efficiency of each polymerase in a PCR. For extension efficiency comparison, a set of primers (see Table 4) was used to amplify amplicons of 0.5, 1, 2, 5, 8, 10 and 12kb in size from the template in a 50µl reaction. Upon completion of the PCR, 5 µl of the PCR was mixed with loading dye, loaded onto a 1% agarose gel and the gel stained with ethidium bromide.

**Table 4. Primers used for testing extension efficiency.**

| Primer | Amplicon Size (kb)^{*1} | Sequence (5'-3') | SEQ ID NO: |
|---|---|---|---|
| L30350F | - | CCTGCTCTGCCGCTTCACGC | 51 |
| L71-0.5R | 0.5 | TCCGGATAAAAACGTCGATGACATTTGC | 52 |
| L71-1R | 1 | GATGACGCATCCTCACGATAATATCCGG | 53 |
| L72-2R | 2 | CCATGATTCAGTGTGCCCGTCTGG | 54 |
| L72-5R | 5 | CGAACGTCGCGCAGAGAAACAGG | 55 |
| L72-8R | 8 | GCCTCGTTGCGTTTGTTTGCACG | 56 |
| L71-10R | 10 | GCACAGAAGCTATTATGCGTCCCCAGG | 57 |
| L71-12R | 12 | TCTTCCTCGTGCATCGAGCTATTCGG | 58 |

| | | | |
|---|---|---|---|
| *¹ Lambda DNA amplicon size when using L30350F and each R primer. | | | |

The PCR reactions contained 20mM Tris-HCl (pH 8.8), 2mM MgSO₄, 10mM KCl (50mM KCl for the fusion enzymes), 10mM (NH₄)₂SO₄, 1% Triton^{®} X-100, 200µM each dNTP, 0.5µM forward and reverse primers, 130pg/µl lambda DNA and 1.25u of enzyme under test.

The cycling protocol was 95°C for 20s; 20 cycles of 94°C for 5s and 72°C for 2 min.

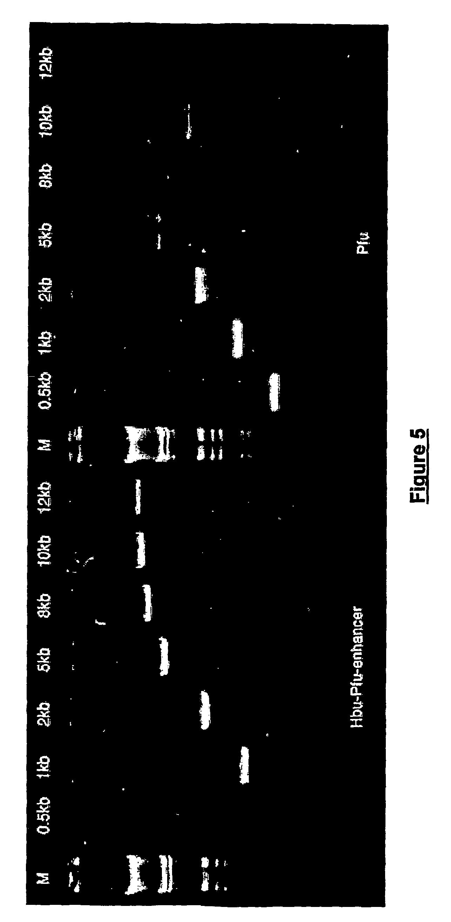

The results are shown in Figure 4 (for Sso KlenTaq fusion) and Figure 5 (for Hbu Pfu fusion).

It is clear that the Hbu-Pfu fusion was able to amplify all fragments, including the 12 kb fragment. In contrast, Pfu polymerase amplified only up to the 5 kb fragment.

Similarly, the Sso-KlenTaq fusion amplified up to the 8kb fragments, whereas KlenTaq polymerase amplified only up to the 2 kb fragment with a 2 min extension time.

Thus, the presence of Cren7 enhancer domain in the fusion proteins result in longer amplification products in PCR reactions compared to the unmodified protein.

### 7. Salt-tolerance in PCR

The binding of polymerase to a primed DNA template is sensitive to the ionic strength of the reaction buffer due to electrostatic interactions, which is stronger in low salt concentration and weaker in high. This example demonstrates that the Cren7 enhancer domain-DNA polymerase fusions exhibit improved performance in PCR reactions containing elevated KCI concentrations. Without wishing to be bound by theory, it is believed that the presence of the Cren7 enhancer domain in the fusion proteins stabilises the binding interaction of the polymerase to DNA template.

Lambda DNA (130pg) was used as a template in PCR reactions with primers L30350F and L71-1R (see Table 1 above). The concentration of KCI was varied from 10mM to 150mM, while all other components of the reaction buffer were unchanged. The PCR reaction was carried out using a cycling program of 94°C for 3 min, 20 cycles of 94°C for 30s, 55°C for 30s, and 72°C for 30s, followed by 72°C for 10 min. Upon completion of the reaction, 5µl of the PCR reaction products were also analysed in on an agarose gel to verify that amplicons of expected length were generated.

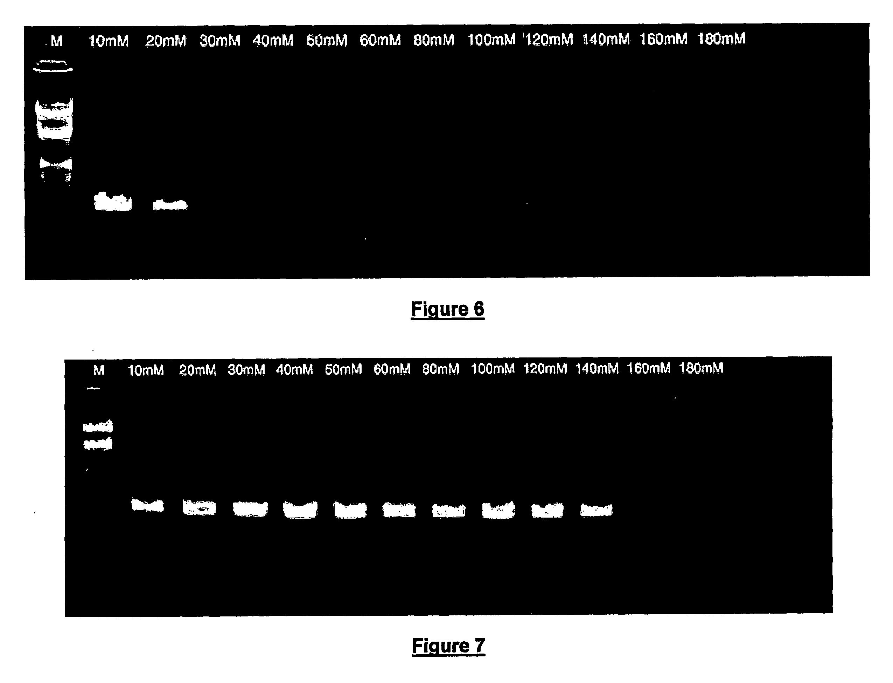

The effects of KCl concentration on the PCR efficiency of Pfu alone versus that of the Ape-Pfu fusion protein, and of KlenTaq alone versus that of the Ape-KlenTaq fusion protein are shown in Figures 6, 7, 8 and 9, respectively.

Unmodified Pfu showed a preference for KCl concentration below 20mM. In contrast, the Ape Cren7 enhancer domain-Pfu fusion protein gave maximum activity in 30-120mM KCl.

Unmodified KlenTaq showed a preference for KCl concentration below 50mM. In contrast, the Ape Cren7 enhancer domain-KlenTaq fusion protein gave maximum activity in 30-90mM KCl.

Thus, the Cren7 enhancer domain-DNA polymerase fusion proteins were more tolerant of elevated KCl concentration in comparison to their counterpart DNA polymerase lacking the Cren7 enhancer domain. This feature of the fusion proteins will allow PCR amplification from low quality of DNA template, e.g., DNA samples prepared from, but not limited to, blood, food and plant sources.

### 8. Use of fusion proteins in qPCR; TaqMan^{®} probe testing

Quantitative PCR experiments were carried out using the following Taq polymerases:
Wild type Taq polymerase
Chimeric Ape Cren7-Taq polymerase

All enzymes were converted for hotstart by chemical modification with anhydride exactly as described in US patent 5,677,152. The instrument used for all tests was Cepheid SmartCycler^{®}

A 142bp mitochondrial target was amplified using the following primers:
Forward: 5' CCA CTG TAA AGC TAA CTT AGC ATT AAC C 3' (SEQ ID NO:62)
Reverse: 5' GTG ATG AGG AAT AGT GTA AGG AGT ATG G 3' (SEQ ID NO:63)
Probe, carrying a FAM fluorescent label at its 5' end and a TAMRA fluorescent label at its 3' end: 5' CCA ACA CCT CTT TAC AGT GAA ATG CCC CA 3' (SEQ ID NO:64)

The amplification conditions for wt Taq polymerase were:
50mM Tris-HCl pH 8.0, 300nM primers, 100nM probe, 5mM MgCl₂, 200µM dNTPs, 1.25u DNA polymerase, 30ng human chromosomal DNA.

The amplification conditions for Ape Cren7-Taq polymerase were:
50mM Tris-HCl pH 8.0, 80mM KCl, 300nM primers, 100nM probe, 5mM MgCl₂, 200µM dNTPs, 1.25u DNA polymerase, 30ng human chromosomal DNA.

Cycling conditions:
95°C for 5mins initial enzyme activation
45 cycles of:

| | |
|---|---|
| 95°C | 3secs |
| 60°C | variable |

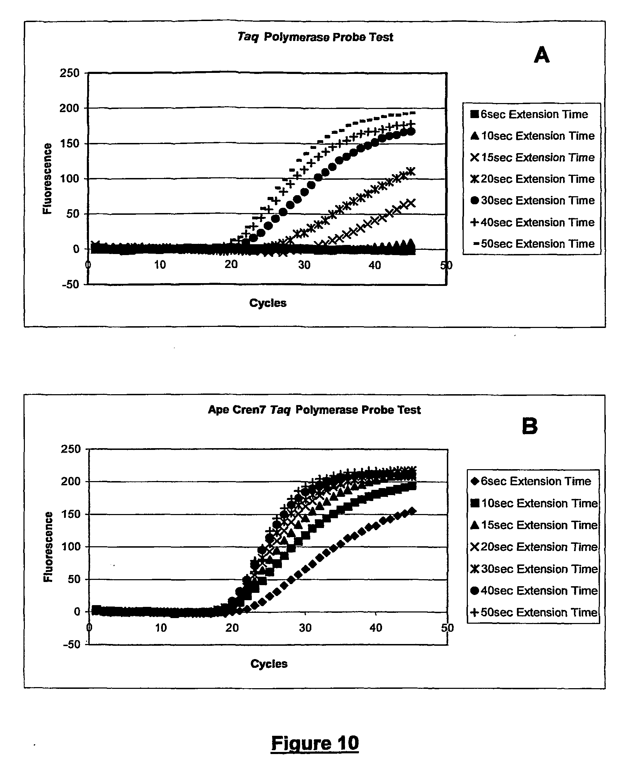

Extension times tested were 50, 40, 30, 20, 15, 10 and 6 seconds. The results in Figure 10 show that the Cren7 fusion construct required a much lower extension time to gain a similar result, i.e., 10 seconds using the fusion polymerase is equivalent to 30-40 seconds using wild type Taq. This demonstrates that the fusion polymerase has a faster rate of progression than the wild type polymerase.

### 9. Use of fusion proteins in qPCR; SYBR^{®} Green I testing

Quantitative PCR experiments were carried out using the following Taq polymerases:
Wild type Taq polymerase
Chimeric Ape Cren7-Taq polymerase
Chimeric Sso Cren7-Taq polymerase

All enzymes were converted for hotstart by chemical modification with anhydride exactly as described in US 5677152. The instrument used for all tests was Cepheid SmartCycler^{®}

A 142bp mitochondrial target was amplified using the following primers:
Forward: 5' CCA CTG TAA AGC TAA CTT AGC ATT AAC C 3' (SEQ ID NO:62)
Reverse: 5' GTG ATG AGG AAT AGT GTA AGG AGT ATG G 3' (SEQ ID NO:63)

The amplification conditions for wt Taq polymerase were:
50mM Tris-HCl pH 8.0, 300nM primers, 0.5x SYBR^{®} Green I, 5mM MgCl₂, 200µM dNTPs, 1.25u DNA polymerase, 30ng human chromosomal DNA.

The amplification conditions used for Ape and Sso Cren7-Taq polymerases were:
15mM Tris-HCl pH 8.3, 80mM (100mM for Sso) KCl, 300nM primers, 0.5x SYBR^{®} Green I, 5mM MgCl₂, 200µM dNTPs, 1.25u DNA polymerase, 30ng human chromosomal DNA.

Cycling conditions:
95°C for 5mins initial enzyme activation
45 cycles of:

| | |
|---|---|
| 95°C | 3secs |
| 60°C | variable |

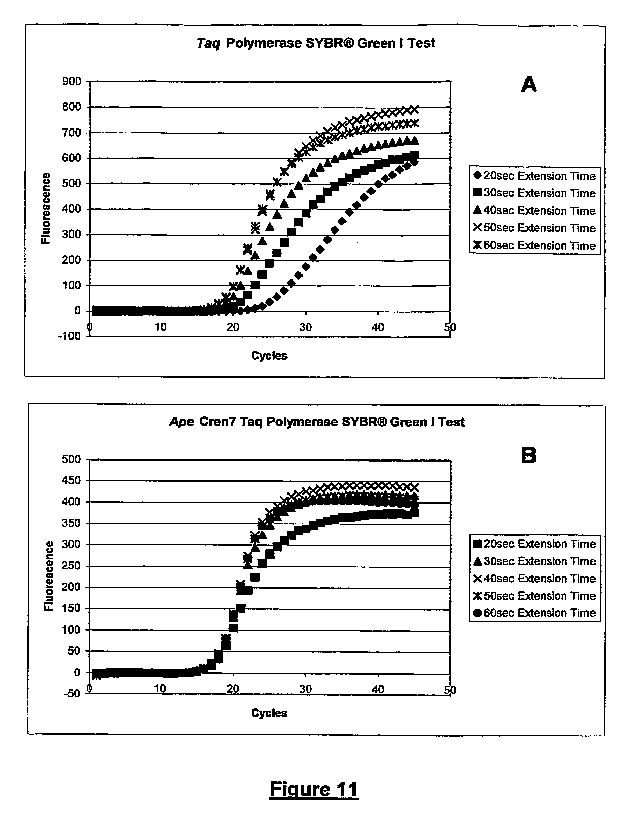

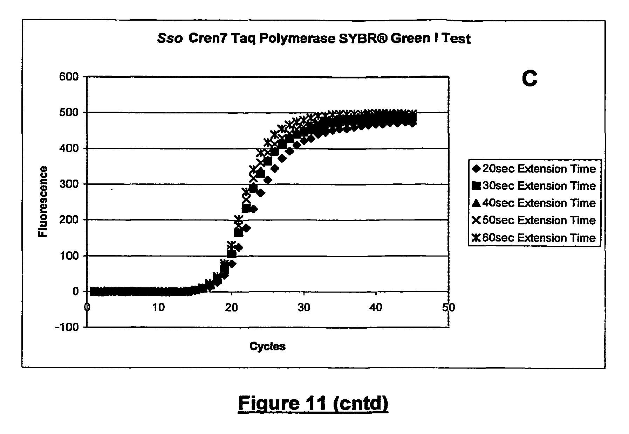

Extension times tested were 60, 50, 40, 30 and 20 seconds. The results in Figure 11 show that the Cren7 fusion constructs require a much lower extension time for a similar result, i.e., 20 seconds using the fusion constructs is equivalent to 50 seconds using wild type Taq. The reactions plateau earlier when using the fusion constructs. Again, the results show that the fusion polymerases have a faster rate of progression than the wild type polymerase.

## Claims

1. A chimeric protein comprising a nucleic acid polymerase domain joined with a Cren7 enhancer domain or variant thereof, in which the Cren7 enhancer domain or variant thereof enhances the activity of the nucleic acid polymerase domain compared with a corresponding protein lacking the Cren7 enhancer domain or variant thereof, wherein the Cren7 enhancer domain variant is a functional variant having at least 40% sequence identity with the Cren7 enhancer protein of SEQ ID NO: 1.

2. The protein according to claim 1, in which the Cren7 enhancer domain or variant thereof comprises the conserved amino acid sequence:
G-X₁-X₂-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-X₁₃ (SEQ ID NO: 15); or comprises the conserved amino acid sequence:
X₂-X₁-X₁-X₁-X₁₀-G-X₁-X₁-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-Xₜ₃ (SEQ ID NO:61).
where X₁ is any amino acid, X₂ is K, R or E, X₃ is L or no amino acid, X₄ is A, V or T, X₅ is K or R, X₆ is I or V, X₇ is G or A, X₈ is K, R or Q, X₉ is D, N or E, X₁₀ is any or no amino acid, X₁₁ is K or H, X₁₂ is I, V or F, and X₁₃ is I, V or L.

3. The protein according to any preceding claim, in which the Cren7 enhancer domain is one of the group comprising: *Sulfolobus solfataricus* Cren7 enhancer protein (SEQ ID NO: 1); *Sulfolobus acidocaldarius* Cren7 enhancer protein (SEQ ID NO: 2); *Metallosphaera sedula* Cren7 enhancer protein (SEQ ID NO: 3); *Staphylothermus marinus* Cren7 enhancer protein (SEQ ID NO: 4); *Hyperthermus butylicus* 0878 Cren7 enhancer protein (SEQ ID NO: 5); *Hyperthermus butylicus* 1128 Cren7 enhancer protein (SEQ ID NO: 6); *Aeropyrum pernix* Cren7 enhancer protein (SEQ ID NO: 7); *Caldivirga maquilingensis* Cren7 enhancer protein (SEQ ID NO: 8); *Ignicoccus hospitalis* Cren7 enhancer protein (SEQ ID NO: 9); *Pyrobaculum islandicum* Cren7 enhancer protein (SEQ ID NO: 10); *Pyrobaculum arsenaticum* Cren7 enhancer protein (SEQ ID NO: 11); *Pyrobaculum aerophilum* Cren7 enhancer protein (SEQ ID NO: 12); *Pyrobaculum calidifontis* Cren7 enhancer protein (SEQ ID NO: 13); *Thermoproteus neutrophilus* Cren7 enhancer protein (SEQ ID NO: 14), *Sulfolobus shibatae* Cren7 enhancer protein (SEQ ID NO:59); and *Sulfolobus tokodaii* Cren7 enhancer protein (SEQ ID NO:60).

4. The protein according to any preceding claim, in which the Cren7 enhancer domain is one of the group comprising: *Sulfolobus solfataricus* Cren7 enhancer protein (SEQ ID NO: 1); *Hyperthermus butylicus* 1128 Cren7 enhancer protein (SEQ ID NO: 6); *Aeropyrum pernix* Cren7 enhancer protein (SEQ ID NO: 7) or in which the Cren7 enhancer domain is a functional variant of any of the Cren7 enhancer proteins of SEQ ID NOs: 1, 6 or 7.

5. The protein according to any preceding of claim, in which the nucleic acid polymerase domain comprises or consists of a thermostable DNA polymerase or a functional mutant, variant or derivative thereof, or of a mesophilic DNA polymerase or a functional mutant, variant or derivative thereof, or of an intermediate temperature DNA polymerase or a functional mutant, variant or derivative thereof.

6. The protein according to claim 5, in which the protein is a fusion protein having the sequence of any one of SEQ ID NOs 16-24, or a functional mutant, variant or derivative thereof.

7. A composition comprising the chimeric protein as defined in any of claims 1-6.

8. An isolated nucleic acid encoding the chimeric protein as defined in any of claims 1-6.

9. A vector comprising the isolated nucleic acid as defined in claim 8.

10. A host cell transformed with the nucleic acid of claim 8 or the vector of claim 9.

11. A kit comprising the chimeric protein as defined in any of claims 1-6, and/or the composition of claim 7, and/or the isolated nucleic acid of claim 8, and/or the vector of claim 9, and/or the host cell of claim 10, together with packaging materials therefor.

12. A method of modifying a nucleic acid, comprising:
a. contacting the nucleic acid with the chimeric protein as defined in any of claims 1-6 under conditions which allow activity of the nucleic acid polymerase domain; and
b. permitting the nucleic acid polymerase domain to modify the nucleic acid.

13. A method of catalysing the synthesis of a polynucleotide from a target nucleic acid, comprising the steps of:
a. providing a chimeric protein as defined in any of claims 1-6; and
b. contacting the target nucleic acid with the chimeric protein under conditions which allow the addition by the chimeric protein of nucleotide units to a nucleotide chain using the target nucleic acid, thereby synthesising the polynucleotide.

14. A method of amplifying a sequence of a target nucleic acid using a thermocycling reaction, comprising the steps of:
a. contacting the target nucleic acid with a chimeric protein as defined in claim 1 ; and
b. incubating the target nucleic acid with the chimeric protein under thermocycling reaction conditions which allow amplification of the target nucleic acid.

## Patentansprüche

1. Chimäres Protein, umfassend eine Nucleinsäure-Polymerase-Domäne, die mit einer Cren7-Enhancer-Domäne oder einer Variante davon verbunden ist, bei dem die Cren7-Enhancer-Domäne oder die Variante davon die Aktivität der Nucleinsäure-Polymerase-Domäne verstärkt, verglichen mit einem entsprechenden Protein, bei dem die Cren7-Enhancer-Domäne oder eine Variante davon fehlt, wobei die Cren7-Enhancer-Domänen-Variante eine funktionelle Variante mit einer mindestens 40 %igen Sequenzidentität zum Cren7-Enhancer-Protein von SEQ ID NO: 1 ist.

2. Protein nach Anspruch 1, bei dem die Cren7-Enhancer-Domäne oder Variante davon die folgende konservierte Aminosäuresequenz umfasst:
G-X₁-X₂-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-X₁₃ (SEQ ID NO: 15)
oder die folgende konservierte Aminosäuresequenz umfasst:
X₂-X₁-X₁-X₁-X₁₀-G-X₁-X₁-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-X₁₃ (SEQ ID NO:61),
wobei X₁ eine beliebige Aminosäure bedeutet, X₂ K, R oder E bedeutet, X₃ L oder keine Aminosäure bedeutet, X₄ A, V oder T bedeutet, X₅ K oder R bedeutet, X₆ I oder V bedeutet, X₇ G oder A bedeutet, X₈ K, R oder Q bedeutet, X₉- D, N oder E bedeutet, X₁₀ eine beliebige oder keine Aminosäure bedeutet, X₁₁ K oder H bedeutet, X₁₂ I, V oder F bedeutet und X₁₃ I, V oder L bedeutet.

3. Protein nach einem der vorstehenden Ansprüche, bei dem die Cren7-Enhancer-Domäne eine Domäne aus der folgenden Gruppe ist:
Sulfolobus solfataricus-Cren7-Enhancer-Protein (SEQ ID NO: 1); Sulfolobus acidocaldarius-Cren7-Enhancer-Protein (SEQ ID NO: 2); Metallosphaera sedula-Cren7-Enhancer-Protein (SEQ ID NO: 3); Staphylothermus marinus-Cren7-Enhancer-Protein (SEQ ID NOs: 4); Hyperthermus butylicus 0878-Cren7-Enhancer-Protein (SEQ ID NO: 5); Hyperthermus butylicus 1128-Cren7-Enhancer-Protein (SEQ ID NOs: 6); Aeropyrum pernix-Cren7-Enhancer-Protein (SEQ ID NO: 7); Caldivirga maquilingensis-Cren7-Enhancer-Protein (SEQ ID NO: 8); Ignicoccus hospitalis-Cren7-Enhancer-Protein (SEQ ID NO: 9); Pyrobaculum islandicum-Cren7-Enhancer-Protein (SEQ ID NO: 10); Pyrobaculum arsenaticum-Cren7-Enhancer-Protein (SEQ ID NO: 11); Pyrobaculum aerophilum-Cren7-Enhancer-Protein (SEQ ID NO: 12); Pyrobaculum calidifontis-Cren7-Enhancer-Protein (SEQ ID NO: 13); Thermoproteus neutrophilus-Cren7-Enhancer-Protein (SEQ ID NO: 14), Sulfolobus shibatae-Cren7-Enhancer-Protein (SEQ ID NO: 59); und Sulfolobus tokodaii-Cren7-Enhancer-Protein (SEQ ID NO: 60).

4. Protein nach einem der vorstehenden Ansprüche, bei dem die Cren7-Enhancer-Domäne eine Domäne aus der folgenden Gruppe ist: Sulfolobus solfataricus-Cren7-Enhancer-Protein (SEQ ID NO: 1); Hyperthermus butylicus 1128-Cren7-Enhancer-Protein (SEQ ID NO: 6); Aeropyrum pernix-Cren7-Enhancer-Protein (SEQ ID NOs: 7), oder bei dem die Cren7-Enhancer-Domäne eine funktionelle Variante von einem der Cren7-Enhancer-Proteine von SEQ ID NO: 1, 6 oder 7 ist.

5. Protein nach einem der vorstehenden Ansprüche, bei dem die Nucleinsäure-Polymerase-Domäne eine thermostabile DNA-Polymerase oder eine funktionelle Mutante, Variante oder Derivat davon umfasst oder daraus besteht oder eine mesophile DNA-Polymerase oder eine funktionelle Mutante, Variante oder Derivat davon umfasst oder daraus besteht oder eine Mitteltemperatur-DNA-Polymerase oder eine funktionelle Mutante, Variante oder Derivat davon umfasst oder daraus besteht.

6. Protein nach Anspruch 5, bei dem das Protein ein Fusionsprotein mit einer der Sequenzen von SEQ ID NO: 16-24 oder eine funktionelle Mutante, Variante oder Derivat davon ist.

7. Zusammensetzung, umfassend das chimäre Protein gemäß der Definition in einem der Ansprüche 1 bis 6.

8. Isolierte Nucleinsäure, die für das chimäre Protein gemäß der Definition in einem der Ansprüche 1 bis 6, kodiert.

9. Vektor, umfassend die isolierte Nucleinsäure gemäß der Definition in Anspruch 8.

10. Wirtszelle, die mit der Nucleinsäure gemäß Anspruch 8 oder mit dem Vektor gemäß Anspruch 9 transformiert ist.

11. Kit, umfassend das chimäre Protein gemäß der Definition in einem der Ansprüche 1 bis 6 und/oder die Zusammensetzung gemäß Anspruch 7 und/oder die isolierte Nucleinsäure gemäß Anspruch 8 und/oder den Vektor gemäß Anspruch 9 und/oder die Wirtszelle gemäß Anspruch 10 zusammen mit Packungsmaterialien hierfür.

12. Verfahren zum Modifizieren einer Nucleinsäure, umfassend die folgenden Stufen:
a. man bringt Nucleinsäure mit dem chimären Protein gemäß der Definition in einem der Ansprüche 1 bis 6 unter Bedingungen in Kontakt, die es der Nucleinsäure-Polymerase-Domäne ermöglichen, ihre Aktivität zu entfalten; und
b. man ermöglicht es der Nucleinsäure-Polymerase-Domäne, die Nucleinsäure zu modifizieren.

13. Verfahren zum Katalysieren der Synthese eines Polynucleotids aus einer Zielnucleinsäure, umfassend die folgenden Stufen:
a. man stellt ein chimäres Protein gemäß der Definition in einem der Ansprüche 1 bis 6 bereit; und
b. man bringt die Zielnucleinsäure mit dem chimären Protein unter Bedingungen in Kontakt, die die Addition von Nucleotideinheiten an ein Nucleotid durch das chimäre Protein unter Verwendung der Zielnucleinsäure ermöglichen, wodurch das Polynucleotid synthetisiert wird.

14. Verfahren zum Amplifizieren einer Sequenz einer Zielnucleinsäure unter Anwendung einer Thermocyclisierungsreaktion, umfassend die folgenden Stufen:
a. man bringt die Zielnucleinsäure mit einem chimären Protein gemäß der Definition in Anspruch 1 in Kontakt; und
b. man inkubiert die Zielnucleinsäure mit dem chimären Protein unter Thermocyclisierungs-Reaktionsbedingungen, die die Amplifikation der Zielnucleinsäure ermöglichen.

## Revendications

1. Protéine chimérique comprenant un domaine d'acide nucléique polymérase joint à un domaine amplificateur Cren7 ou une variante de celui-ci, dans laquelle le domaine amplificateur Cren7 ou une variante de celui-ci renforce l'activité du domaine d'acide nucléique polymérase par rapport à une protéine correspondante dépourvue du domaine amplificateur Cren7 ou une variante de celui-ci, la variante du domaine amplificateur Cren7 étant une variante fonctionnelle ayant au moins 40 % d'identité de séquence avec la protéine amplificateur Cren7 de SÉQ ID N° : 1.

2. Protéine selon la revendication 1, dans laquelle le domaine amplificateur Cren7 ou une variante de celui-ci comprend la séquence d'acides aminés conservée :
G-X₁-X₂-X₁-X₁-X₃-X₁-P-X₁-K-X₄-W-X₁-L-X₁-P-X₁-G-X₅-X₁-G-V-X₁-X₆-X₇-L-F-X₈-X₁-P-X₉-X₁₀-G-X₁₁-X₁-X₁₂-R-X₁-X₁-X₁₃ (SÉQ ID N° : 15) ; ou comprend la séquence d'acides aminés conservée :
X₂-X₁-X₁-X₁-X₁₀-G-X₁-X₁-X₁-X₁-X₃-X₁-P-X₁-K-X₄-W-X₁-L-X₁-P-X₁-G-X₁-X₁-G-V-X₁-X₆-X₇-L-F-X₈-X₁-P-X₉-X₁₀-G-X₁₁-X₁-X₁₂-R-X₁-X₁-X₁₃ (SEQ ID N° : 61),
où X₁ est un acide aminé, X₂ est K, R ou E, X₃ est L ou aucun acide aminé, X₄ est A, V ou T, X₅ est K ou R, X₆ est I ou V, X₇ est G ou A, X₈ est K, R ou Q, X₉ est D, N ou E, X₁₀ est un acide aminé ou n'est pas un acide aminé, X₁₁ est K ou H, X₁₂ est I, V ou F et X₁₃ est I, V ou L.

3. Protéine selon l'une quelconque des revendications précédentes, dans laquelle le domaine amplificateur Cren7 est l'un du groupe comprenant : la protéine amplificateur Cren7 de Sulfolobus solfataricus (SÉQ ID N° : 1) ; la protéine amplificatrice Cren7 de Sulfolobus acidocaldarius (SÉQ ID N° : 2) ; la protéine amplificatrice Cren7 de Metallosphaera sedula (SÉQ ID N° : 3) ; la protéine amplificatrice Cren7 de Staphylothermus marinus (SÉQ ID N° : 4) ; la protéine amplificatrice Cren7 de Hyperthermus butylicus 0878 (SÉQ ID N° : 5) ; la protéine amplificatrice Cren7 de Hyperthermus butylicus 1128 (SÉQ ID N° : 6) ; la protéine amplificatrice Cren7 de Aeropyrum pernix (SÉQ ID N° : 7) ; la protéine amplificatrice Cren7 de Caldivirga maquilingensis (SÉQ ID N° : 8) ; la protéine amplificatrice Cren7 de Ignicoccus hospitalis (SÉQ ID N° : 9) ; la protéine amplificatrice Cren7 de Pyrobaculum islandicum (SÉQ ID N° : 10) ; la protéine amplificatrice Cren7 de Pyrobaculum arsenaticum (SÉQ ID N° : 11) ; la protéine amplificatrice Cren7 de Pyrobaculum aerophilum (SÉQ ID N° : 12) ; la protéine amplificatrice Cren7 de Pyrobaculum calidifontis (SÉQ ID N° : 13) ; la protéine amplificatrice Cren7 de Thermoproteus neutrophilus (SÉQ ID N° : 14), la protéine amplificatrice Cren7 de Sulfolobus shibatae (SÉQ ID N° : 59) ; et la protéine amplificatrice Cren7 de Sulfolobus tokodaii (SÉQ ID N° : 60).

4. Protéine selon l'une quelconque des revendications précédentes, dans laquelle le domaine amplificateur Cren7 est l'un du groupe comprenant la protéine amplificatrice Cren7 de Sulfolobus solfataricus (SÉQ ID N° : 1) ; la protéine amplificatrice Cren7 de Hyperthermus butylicus 1128 (SÉQ ID N° : 6) ; la protéine amplificatrice Cren7 de Aeropyrum pernix (SÉQ ID N° : 7) ou dans laquelle le domaine amplificateur Cren7 est une variante fonctionnelle de l'une quelconque des protéines amplificatrices Cren7 de SÉQ ID N° : 1, 6 ou 7.

5. Protéine selon l'une quelconque des revendications précédentes, dans laquelle le domaine d'acide nucléique polymérase comprend ou consiste en une ADN polymérase thermostable ou un mutant fonctionnel, une variante ou un dérivé de celle-ci ou une ADN polymérase mésophile ou un mutant fonctionnel, une variante ou un dérivé de celle-ci ou d'une ADN polymérase de température intermédiaire ou un mutant fonctionnel, une variante ou un dérivé de celle-ci.

6. Protéine selon la revendication 5, la protéine étant une protéine de fusion ayant la séquence selon l'une quelconque des SÉQ ID N° 16 à 24 ou un mutant, une variante ou un dérivé fonctionnel de celle-ci.

7. Composition comprenant la protéine chimérique telle que définie dans l'une quelconque des revendications 1 à 6.

8. Acide nucléique isolé codant pour la protéine chimérique telle que définie dans l'une quelconque des revendications 1 à 6.

9. Vecteur comprenant l'acide nucléique isolé tel que défini dans la revendication 8.

10. Cellule hôte transformée avec l'acide nucléique selon la revendication 8 ou le vecteur selon la revendication 9.

11. kit comprenant la protéine chimérique telle que définie dans l'une quelconque des revendications 1 à 6, et/ou composition selon la revendication 7 et/ou acide nucléique isolé selon la revendication 8 et/ou vecteur selon la revendication 9 et/ou cellule hôte selon la revendication 10, conjointement avec des matériaux de conditionnement à cette fin.

12. Procédé de modification d'un acide nucléique comprenant :
a. la mise en contact de l'acide nucléique avec la protéine chimérique telle que définie dans l'une quelconque des revendications 1 à 6, dans des conditions qui permettent l'activité du domaine de l'acide nucléique polymérase ; et
b. le fait de permettre au domaine d'acide nucléique de modifier l'acide nucléique.

13. Procédé de catalyse de la synthèse d'un polynucléotide d'un acide nucléique cible comprenant les étapes consistant à :
a. fournir une protéine chimérique telle que définie dans l'une quelconque des revendications 1 à 6 : et
b. mettre en contact l'acide nucléique cible avec la protéine chimérique dans des conditions qui permettent l'addition par la protéine chimérique d'unités de nucléotide à une chaîne de nucléotide en utilisant l'acide nucléique cible, synthétisant ainsi le polynucléotide.

14. Procédé d'amplification d'une séquence d'un acide nucléique cible utilisant une réaction de thermocyclage, comprenant les étapes consistant à :
a. mettre en contact l'acide nucléique cible avec une protéine chimérique telle que définie dans la revendication 1 ; et
b. incuber l'acide nucléique cible avec la protéine chimérique dans des conditions réactionnelles de thermocyclage qui permettent l'amplification de l'acide nucléique cible.


### SEQUENCE LISTING

<110> Genesys Ltd
   Clark, Duncan
   wilkinson, Martin
   Morant, Nicholas
<120> Chimeric protein
<130> P1420PC00
<150> 61/010,812
   <151> 2008-01-11
<160> 64
<170> PatentIn version 3.5
<210> 1
   <211> 60
   <212> PRT
   <213> sulfolobus solfataricus
<400> 1
   Met Ser Ser Gly Lys Lys Pro Val Lys Val Lys Thr Pro Ala Gly Lys
   1               5                   10                  15
   Glu Ala Glu Leu Val Pro Glu Lys Val Trp Ala Leu Ala Pro Lys Gly
               20                  25                  30
   Arg Lys Gly Val Lys Ile Gly Leu Phe Lys Asp Pro Glu Thr Gly Lys
           35                  40                  45
   Tyr Phe Arg His Lys Leu Pro Asp Asp Tyr Pro Ile
       50                  55                  60
<210> 2
   <211> 59
   <212> PRT
   <213> sulfolobus acidocaldarius
<400> 2
   Met Ser Glu Lys Lys Arg Val Arg Val Arg Thr Pro Gly Gly Lys Glu
   1               5                   10                  15
   Leu Glu Leu Thr Pro Glu Lys Thr Trp Val Leu Ala Pro Lys Gly Arg
               20                  25                  30
   Lys Gly Val Lys Ile Gly Leu Phe Lys Asp Pro Glu Ser Gly Lys Tyr
           35                  40                  45
   Phe Arg His Lys Leu Pro Asp Asp Tyr Pro Val
       50                  55
<210> 3
   <211> 59
   <212> PRT
   <213> Metallosphaera sedula
<400> 3
   Met Thr Tyr Lys Lys Ala Val Lys Ile Lys Thr Pro Gly Gly Lys Glu
   1               5                   10                  15
   Ala Glu Leu Ala Pro Glu Lys Ala Trp Thr Leu Ala Pro Lys Gly Arg
               20                  25                  30
   Lys Gly Val Lys Ile Gly Leu Phe Lys Asp Pro Glu Ser Gly Lys Tyr
           35                  40                  45
   Phe Arg His Lys Leu Pro Asp Asp Tyr Pro Val
       50                  55
<210> 4
   <211> 61
   <212> PRT
   <213> Staphylothermus marinus
<400> 4
   Met Ala Ala Cys Lys Asp Ala Val Lys Val Lys Thr Leu Ser Gly Lys
   1               5                   10                  15
   Glu Val Glu Leu Val Pro Lys Lys Val Trp Gln Leu Ser Pro Lys Gly
               20                  25                  30
   Arg Lys Gly Val Lys Val Gly Leu Phe Gln Asp Pro Glu Thr Gly Lys
           35                  40                  45
   Tyr Phe Ala Lys Val Pro Asp Asp Tyr Pro Ile Cys Gly
       50                  55                  60
<210> 5
   <211> 61
   <212> PRT
   <213> Hyperthermus butylicus
<400> 5
   Met Ala Cys Glu Lys Pro Val Lys Val Arg Asp Pro Thr Thr Gly Lys
   1               5                   10                  15
   Glu Val Glu Leu Val Pro Ile Lys Val Trp Gln Leu Ala Pro Lys Gly
               20                  25                  30
   Arg Lys Gly Val Lys Ile Gly Leu Phe Lys Ser Pro Glu Thr Gly Lys
           35                  40                  45
   Tyr Phe Ala Lys Val Pro Asp Asp Tyr Pro Ile Cys Ser
       50                  55                  60
<210> 6
   <211> 62
   <212> PRT
   <213> Hyperthermus butylicus
<400> 6
   Met Ala Cys Glu Lys Pro Val Lys Val Arg Asp Pro Thr Thr Gly Lys
   1               5                   10                  15
   Glu Val Glu Leu Val Pro Ile Lys Val Trp Gln Leu Ala Pro Arg Gly
               20                  25                  30
   Arg Lys Gly Val Lys Ile Gly Leu Phe Lys Ser Pro Glu Thr Gly Lys
           35                  40                  45
   Tyr Phe Arg Ala Lys Val Pro Asp Asp Tyr Pro Ile Cys Ser
       50                  55                  60
<210> 7
   <211> 63
   <212> PRT
   <213> Aeropyrum pernix
<400> 7
   Met Ser Gln Lys Gln Leu Pro Pro Val Lys Val Arg Asp Pro Thr Thr
   1               5                   10                  15
   Gly Lys Glu Val Glu Leu Thr Pro Ile Lys Val Trp Lys Leu Ser Pro
               20                  25                  30
   Arg Gly Arg Arg Gly Val Lys Ile Gly Leu Phe Lys Ser Pro Glu Thr
           35                  40                  45
   Gly Lys Phe Arg Ala Lys Val Pro Asp Asp Tyr Pro Glu Thr Gly
       50                  55                  60
<210> 8
   <211> 76
   <212> PRT
   <213> Caldivirga maquilingensis
<400> 8
   Met Leu Phe Met Phe Ile Ser His Tyr Ala Val Tyr Leu Leu Thr Gly
   1               5                   10                  15
   Met Ala Val Asn Val Gln Gln Tyr Leu Asn Lys Glu Tyr Glu Val Glu
               20                  25                  30
   Cys Asp Gly Gln Met Val Arg Leu Lys Pro Val Lys Ala Trp Val Leu
           35                  40                  45
   Gln Pro Gly Arg Lys Gly Val Val Ile Gly Leu Phe Lys Cys Pro Asn
       50                  55                  60
   Gly Lys Thr Leu Arg Lys Ala Ile Gly Lys Ile Glu
   65                  70                  75
<210> 9
   <211> 55
   <212> PRT
   <213> Ignicoccus hospitalis
<400> 9
   Met Pro Lys Cys Pro Lys Cys Gly Ala Glu Val Lys Glu Pro Ile Lys
   1               5                   10                  15
   Thr Trp Val Leu Ala Pro Lys Gly Arg Lys Gly Val Ile Ile Gly Leu
               20                  25                  30
   Phe Arg Cys Pro Asn Gly His Tyr Phe Arg Ala Lys Val Gly Glu Ala
           35                  40                  45
   Pro Pro Lys Lys Glu Ala Ala
       50                  55
<210> 10
   <211> 59
   <212> PRT
   <213> Pyrobaculum islandicum
<400> 10
   Met Glu Glu Val Leu Asp Arg Glu Tyr Glu Val Glu Tyr Gly Gly Arg
   1 5 10 15
   Lys Tyr Arg Leu Lys Pro Val Lys Ala Trp Val Leu Gln Pro Pro Gly
   20 25 30
   Lys Pro Gly Val Val Ile Ala Leu Phe Lys Leu Pro Asp Gly Lys Thr
   35 40 45
   Ile Arg Lys Val Ile Met Lys Leu Pro Pro Ser
   50 55
<210> 11
   <211> 59
   <212> PRT
   <213> Pyrobaculum arsenaticum
<400> 11
   Met Ala Glu Glu Ile Leu Asn Arg Glu Tyr Glu Val Glu Tyr Gly Gly
   1 5 10 15
   Lys Arg Tyr Ile Leu Arg Pro Ile Lys Ala Trp Val Leu Gln Pro Pro
   20 25 30
   Gly Lys Pro Gly Val Val Val Ala Leu Phe Arg Leu Pro Asp Gly Lys
   35 40 45
   Thr Val Arg Lys Val Val Met Lys Leu Pro Pro
   50 55
<210> 12
   <211> 59
   <212> PRT
   <213> Pyrobaculum aerophilum
<400> 12
   Met Ala Glu Glu Ile Leu Asn Arg Glu Tyr Glu Val Glu Tyr Glu Gly
   1 5 10 15
   Arg Lys Tyr Phe Leu Arg Pro Val Lys Ala Trp Val Leu Gln Pro Pro
   20 25 30
   Gly Lys Pro Gly Val Val Val Ala Leu Phe Lys Leu Pro Asn Gly Lys
   35 40 45
   Ser Ile Arg Lys Val Ile Met Arg Leu Pro Pro
   50 55
<210> 13
   <211> 63
   <212> PRT
   <213> Pyrobaculum calidifontis
<400> 13
   Met Asp Gln Asp Val Ala Glu Glu Ile Leu Asn Lys Glu Tyr Glu Val
   1               5                   10                  15
   Val Tyr Glu Gly Lys Arg Phe Leu Leu Lys Pro Ala Lys Ala Trp Val
               20                  25                  30
   Leu Gln Pro Pro Gly Lys Pro Gly Val Ile Val Ala Leu Phe Lys Leu
           35                  40                  45
   Pro Asn Gly Lys Thr Val Arg Lys Val Ile Ala Arg Leu Pro Pro
       50                  55                  60
<210> 14
   <211> 59
   <212> PRT
   <213> Thermoproteus neutrophilus
<400> 14
   Met Ala Glu Glu Ile Leu Asn Arg Glu Tyr Glu Val Glu Tyr Gly Gly
   1               5                   10                  15
   Lys Arg Tyr Trp Leu Arg Pro Ser Lys Ala Trp Val Leu Gln Pro Pro
               20                  25                  30
   Gly Lys Pro Gly Val Val Ile Ala Leu Phe Lys Leu Pro Asp Gly Arg
           35                  40                  45
   Thr Val Arg Lys Ala Ile Met Arg Leu Pro Pro
       50                  55
<210> 15
   <211> 40
   <212> PRT
   <213> Artificial
<220>
   <223> Consensus sequence
<220>
   <221> misc_feature
   <222> (2)..(2)
   <223> xaa can be any naturally occurring amino acid
<220>
   <221> misc_feature
   <222> (3)..(3)
   <223> xaa can be Lys, Arg or Glu
<220>
   <221> misc_feature
   <222> (4)..(5)
   <223> xaa can be any naturally occurring amino acid
<220>
   <221> misc_feature
   <222> (6)..(6)
   <223> xaa can be Leu or no amino acid
<220>
   <221> misc_feature
   <222> (7)..(7)
   <223> xaa can be any naturally occurring amino acid
<220>
   <221> misc_feature
   <222> (9)..(9)
   <223> xaa can be any naturally occurring amino acid
<220>
   <221> misc_feature
   <222> (11)..(11)
   <223> xaa can be Ala, val or Thr
<220>
   <221> misc_feature
   <222> (13)..(13)
   <223> xaa can be any naturally occurring amino acid
<220>
   <221> misc_feature
   <222> (15)..(15)
   <223> xaa can be any naturally occurring amino acid
<220>
   <221> misc_feature
   <222> (17)..(17)
   <223> xaa can be any naturally occurring amino acid
<220>
   <221> misc_feature
   <222> (19)..(19)
   <223> xaa can be Lys or Arg
<220>
   <221> misc_feature
   <222> (20)..(20)
   <223> xaa can be any naturally occurring amino acid
<220>
   <221> misc_feature
   <222> (23)..(23)
   <223> xaa can be any naturally occurring amino acid
<220>
   <221> misc_feature
   <222> (24)..(24)
   <223> xaa can be Ile or val
<220>
   <221> misc_feature
   <222> (25)..(25)
   <223> Xaa can be Gly or Ala
<220>
   <221> misc_feature
   <222> (28)..(28)
   <223> xaa can be Lys, Arg or Gln
<220>
   <221> misc_feature
   <222> (29)..(29)
   <223> xaa can be any naturally occurring amino acid
<220>
   <221> misc_feature
   <222> (31)..(31)
   <223> xaa can be Asp, Asn or Glu
<220>
   <221> misc_feature
   <222> (32)..(32)
   <223> xaa can be any naturally occurring amino acid or no amino acid
<220>
   <221> misc_feature
   <222> (34)..(34)
   <223> Xaa can be Lys or His
<220>
   <221> misc_feature
   <222> (35)..(35)
   <223> Xaa can be any naturally occurring amino acid
<220>
   <221> misc_feature
   <222> (36)..(36)
   <223> Xaa can be Ile, val or Phe
<220>
   <221> misc_feature
   <222> (38)..(39)
   <223> Xaa can be any naturally occurring amino acid
<220>
   <221> misc_feature
   <222> (40)..(40)
   <223> Xaa can be Ile, val or Leu
<400> 15
   Gly Xaa Xaa Xaa Xaa Xaa Xaa Pro Xaa Lys Xaa Trp Xaa Leu Xaa Pro
   1 5 10 15
   Xaa Gly Xaa Xaa Gly Val Xaa Xaa Xaa Leu Phe Xaa Xaa Pro Xaa Xaa
   20 25 30
   Gly Xaa Xaa Xaa Arg Xaa Xaa Xaa
   35 40
<210> 16
   <211> 621
   <212> PRT
   <213> chimeric protein
<400> 16
   Met Ser Gln Lys Gln Leu Pro Pro Val Lys Val Arg Asp Pro Thr Thr
   1 5 10 15
   Gly Lys Glu Val Glu Leu Thr Pro Ile Lys Val Trp Lys Leu Ser Pro
   20 25 30
   Arg Gly Arg Arg Gly Val Lys Ile Gly Leu Phe Lys Ser Pro Glu Thr
   35 40 45
   Gly Lys Tyr Phe Arg Ala Lys Val Pro Asp Asp Tyr Pro Glu Thr Gly
   50 55 60
   Gly Thr His Met Gly Leu Leu His Glu Phe Gly Leu Leu Glu Ser Pro
   65 70 75 80
   Lys Ala Leu Glu Glu Ala Pro Trp Pro Pro Pro Glu Gly Ala Phe Val
   85 90 95
   Gly Phe Val Leu Ser Arg Lys Glu Pro Met Trp Ala Asp Leu Leu Ala
   100 105 110
   Leu Ala Ala Ala Arg Gly Gly Arg Val His Arg Ala Pro Glu Pro Tyr
   115 120 125
   Lys Ala Leu Arg Asp Leu Lys Glu Ala Arg Gly Leu Leu Ala Lys Asp
   130 135 140
   Leu Ser Val Leu Ala Leu Arg Glu Gly Leu Gly Leu Pro Pro Gly Asp
   145 150 155 160
   Asp Pro Met Leu Leu Ala Tyr Leu Leu Asp Pro Ser Asn Thr Thr Pro
   165 170 175
   Glu Gly Val Ala Arg Arg Tyr Gly Gly Glu Trp Thr Glu Glu Ala Gly
   180 185 190
   Glu Arg Ala Ala Leu Ser Glu Arg Leu Phe Ala Asn Leu Trp Gly Arg
   195 200 205
   Leu Glu Gly Glu Glu Arg Leu Leu Trp Leu Tyr Arg Glu Val Glu Arg
   210 215 220
   Pro Leu Ser Ala Val Leu Ala His Met Glu Ala Thr Gly Val Arg Leu
   225 230 235 240
   Asp Val Ala Tyr Leu Arg Ala Leu Ser Leu Glu Val Ala Glu Glu Ile
   245 250 255
   Ala Arg Leu Glu Ala Glu Val Phe Arg Leu Ala Gly His Pro Phe Asn
   260 265 270
   Leu Asn Ser Arg Asp Gln Leu Glu Arg Val Leu Phe Asp Glu Leu Gly
   275 280 285
   Leu Pro Ala Ile Gly Lys Thr Glu Lys Thr Gly Lys Arg Ser Thr Ser
   290 295 300
   Ala Ala Val Leu Glu Ala Leu Arg Glu Ala His Pro Ile Val Glu Lys
   305 310 315 320
   Ile Leu Gln Tyr Arg Glu Leu Thr Lys Leu Lys Ser Thr Tyr Ile Asp
   325 330 335
   Pro Leu Pro Asp Leu Ile His Pro Arg Thr Gly Arg Leu His Thr Arg
   340 345 350
   Phe Asn Gln Thr Ala Thr Ala Thr Gly Arg Leu Ser Ser Ser Asp Pro
   355 360 365
   Asn Leu Gln Asn Ile Pro Val Arg Thr Pro Leu Gly Gln Arg Ile Arg
   370 375 380
   Arg Ala Phe Ile Ala Glu Glu Gly Trp Leu Leu Val Ala Leu Asp Tyr
   385 390 395 400
   Ser Gln Ile Glu Leu Arg Val Leu Ala His Leu Ser Gly Asp Glu Asn
   405 410 415
   Leu Ile Arg Val Phe Gln Glu Gly Arg Asp Ile His Thr Glu Thr Ala
   420 425 430
   Ser Trp Met Phe Gly Val Pro Arg Glu Ala Val Asp Pro Leu Met Arg
   435 440 445
   Arg Ala Ala Lys Thr Ile Asn Phe Gly Val Leu Tyr Gly Met Ser Ala
   450 455 460
   His Arg Leu Ser Gln Glu Leu Ala Ile Pro Tyr Glu Glu Ala Gln Ala
   465 470 475 480
   Phe Ile Glu Arg Tyr Phe Gln Ser Phe Pro Lys Val Arg Ala Trp Ile
   485 490 495
   Glu Lys Thr Leu Glu Glu Gly Arg Arg Arg Gly Tyr Val Glu Thr Leu
   500 505 510
   Phe Gly Arg Arg Arg Tyr Val Pro Asp Leu Glu Ala Arg Val Lys Ser
   515 520 525
   Val Arg Glu Ala Ala Glu Arg Met Ala Phe Asn Met Pro Val Gln Gly
   530 535 540
   Thr Ala Ala Asp Leu Met Lys Leu Ala Met Val Lys Leu Phe Pro Arg
   545 550 555 560
   Leu Glu Glu Met Gly Ala Arg Met Leu Leu Gln Val His Asp Glu Leu
   565 570 575
   Val Leu Glu Ala Pro Lys Glu Arg Ala Glu Ala Val Ala Arg Leu Ala
   580 585 590
   Lys Glu Val Met Glu Gly Val Tyr Pro Leu Ala Val Pro Leu Glu Val
   595 600 605
   Glu Val Gly Ile Gly Glu Asp Trp Leu Ser Ala Lys Glu
   610 615 620
<210> 17
   <211> 901
   <212> PRT
   <213> Artificial
<220>
   <223> Chimeric protein
<400> 17
   Met Ser Gln Lys Gln Leu Pro Pro Val Lys Val Arg Asp Pro Thr Thr
   1 5 10 15
   Gly Lys Glu Val Glu Leu Thr Pro Ile Lys Val Trp Lys Leu Ser Pro
   20 25 30
   Arg Gly Arg Arg Gly Val Lys Ile Gly Leu Phe Lys Ser Pro Glu Thr
   35 40 45
   Gly Lys Tyr Phe Arg Ala Lys Val Pro Asp Asp Tyr Pro Glu Thr Gly
   50 55 60
   Gly Gly Val Thr Met Asp Ser Gly Met Leu Pro Leu Phe Glu Pro Lys
   65 70 75 80
   Gly Arg Val Leu Leu Val Asp Gly His His Leu Ala Tyr Arg Thr Phe
   85 90 95
   His Ala Leu Lys Gly Leu Thr Thr Ser Arg Gly Glu Pro Val Gln Ala
   100 105 110
   Val Tyr Gly Phe Ala Lys Ser Leu Leu Lys Ala Leu Lys Glu Asp Gly
   115 120 125
   Asp Ala Val Ile Val Val Phe Asp Ala Lys Ala Pro Ser Phe Arg His
   130 135 140
   Glu Ala Tyr Gly Gly Tyr Lys Ala Gly Arg Ala Pro Thr Pro Glu Asp
   145 150 155 160
   Phe Pro Arg Gln Leu Ala Leu Ile Lys Glu Leu Val Asp Leu Leu Gly
   165 170 175
   Leu Ala Arg Leu Glu Val Pro Gly Tyr Glu Ala Asp Asp Val Leu Ala
   180 185 190
   Ser Leu Ala Lys Lys Ala Glu Lys Glu Gly Tyr Glu Val Arg Ile Leu
   195 200 205
   Thr Ala Asp Lys Asp Leu Tyr Gln Leu Leu Ser Asp Arg Ile His Val
   210 215 220
   Leu His Pro Glu Gly Tyr Leu Ile Thr Pro Ala Trp Leu Trp Glu Lys
   225 230 235 240
   Tyr Gly Leu Arg Pro Asp Gln Trp Ala Asp Tyr Arg Ala Leu Thr Gly
   245 250 255
   Asp Glu Ser Asp Asn Leu Pro Gly Val Lys Gly Ile Gly Glu Lys Thr
   260 265 270
   Ala Arg Lys Leu Leu Glu Glu Trp Gly Ser Leu Glu Ala Leu Leu Lys
   275 280 285
   Asn Leu Asp Arg Leu Lys Pro Ala Ile Arg Glu Lys Ile Leu Ala His
   290 295 300
   Met Asp Asp Leu Lys Leu Ser Trp Asp Leu Ala Lys Val Arg Thr Asp
   305 310 315 320
   Leu Pro Leu Glu Val Asp Phe Ala Lys Arg Arg Glu Pro Asp Arg Glu
   325 330 335
   Arg Leu Arg Ala Phe Leu Glu Arg Leu Glu Phe Gly Ser Leu Leu His
   340 345 350
   Glu Phe Gly Leu Leu Glu Ser Pro Lys Ala Leu Glu Glu Ala Pro Trp
           355                 360                 365
   Pro Pro Pro Glu Gly Ala Phe Val Gly Phe Val Leu Ser Arg Lys Glu
       370                 375                 380
   Pro Met Trp Ala Asp Leu Leu Ala Leu Ala Ala Ala Arg Gly Gly Arg
   385                 390                 395                 400
   Val His Arg Ala Pro Glu Pro Tyr Lys Ala Leu Arg Asp Leu Lys Glu
                   405                 410                 415
   Ala Arg Gly Leu Leu Ala Lys Asp Leu Ser Val Leu Ala Leu Arg Glu
               420                 425                 430
   Gly Leu Gly Leu Pro Pro Gly Asp Asp Pro Met Leu Leu Ala Tyr Leu
           435                 440                 445
   Leu Asp Pro Ser Asn Thr Thr Pro Glu Gly Val Ala Arg Arg Tyr Gly
       450                 455                 460
   Gly Glu Trp Thr Glu Glu Ala Gly Glu Arg Ala Ala Leu Ser Glu Arg
   465                 470                 475                 480
   Leu Phe Ala Asn Leu Trp Gly Arg Leu Glu Gly Glu Glu Arg Leu Leu
                   485                 490                 495
   Trp Leu Tyr Arg Glu Val Glu Arg Pro Leu Ser Ala Val Leu Ala His
               500                 505                 510
   Met Glu Ala Thr Gly Val Arg Leu Asp Val Ala Tyr Leu Arg Ala Leu
           515                 520                 525
   Ser Leu Glu Val Ala Glu Glu Ile Ala Arg Leu Glu Ala Glu Val Phe
       530                 535                 540
   Arg Leu Ala Gly His Pro Phe Asn Leu Asn Ser Arg Asp Gln Leu Glu
   545                 550                 555                 560
   Arg Val Leu Phe Asp Glu Leu Gly Leu Pro Ala Ile Gly Lys Thr Glu
                   565                 570                 575
   Lys Thr Gly Lys Arg Ser Thr Ser Ala Ala Val Leu Glu Ala Leu Arg
               580                 585                 590
   Glu Ala His Pro Ile Val Glu Lys Ile Leu Gln Tyr Arg Glu Leu Thr
           595                 600                 605
   Lys Leu Lys Ser Thr Tyr Ile Asp Pro Leu Pro Asp Leu Ile His Pro
       610                 615                 620
   Arg Thr Gly Arg Leu His Thr Arg Phe Asn Gln Thr Ala Thr Ala Thr
   625 630 635 640
   Gly Arg Leu Ser Ser Ser Asp Pro Asn Leu Gln Asn Ile Pro Val Arg
   645 650 655
   Thr Pro Leu Gly Gln Arg Ile Arg Arg Ala Phe Ile Ala Glu Glu Gly
   660 665 670
   Trp Leu Leu Val Ala Leu Asp Tyr Ser Gln Ile Glu Leu Arg Val Leu
   675 680 685
   Ala His Leu Ser Gly Asp Glu Asn Leu Ile Arg Val Phe Gln Glu Gly
   690 695 700
   Arg Asp Ile His Thr Glu Thr Ala Ser Trp Met Phe Gly Val Pro Arg
   705 710 715 720
   Glu Ala Val Asp Pro Leu Met Arg Arg Ala Ala Lys Thr Ile Asn Phe
   725 730 735
   Gly Val Leu Tyr Gly Met Ser Ala His Arg Leu Ser Gln Glu Leu Ala
   740 745 750
   Ile Pro Tyr Glu Glu Ala Gln Ala Phe Ile Glu Arg Tyr Phe Gln Ser
   755 760 765
   Phe Pro Lys Val Arg Ala Trp Ile Glu Lys Thr Leu Glu Glu Gly Arg
   770 775 780
   Arg Arg Gly Tyr Val Glu Thr Leu Phe Gly Arg Arg Arg Tyr Val Pro
   785 790 795 800
   Asp Leu Glu Ala Arg Val Lys Ser Val Arg Glu Ala Ala Glu Arg Met
   805 810 815
   Ala Phe Asn Met Pro Val Gln Gly Thr Ala Ala Asp Leu Met Lys Leu
   820 825 830
   Ala Met Val Lys Leu Phe Pro Arg Leu Glu Glu Met Gly Ala Arg Met
   835 840 845
   Leu Leu Gln Val His Asp Glu Leu Val Leu Glu Ala Pro Lys Glu Arg
   850 855 860
   Ala Glu Ala Val Ala Arg Leu Ala Lys Glu Val Met Glu Gly Val Tyr
   865 870 875 880
   Pro Leu Ala Val Pro Leu Glu Val Glu Val Gly Ile Gly Glu Asp Trp
   885 890 895
   Leu Ser Ala Lys Glu
   900
<210> 18
   <211> 841
   <212> PRT
   <213> Artificial
<220>
   <223> Chimeric protein
<400> 18
   Met Ile Leu Asp Val Asp Tyr Ile Thr Glu Glu Gly Lys Pro Val Ile
   1               5                   10                  15
   Arg Leu Phe Lys Lys Glu Asn Gly Lys Phe Lys Ile Glu His Asp Arg
               20                  25                  30
   Thr Phe Arg Pro Tyr Ile Tyr Ala Leu Leu Arg Asp Asp Ser Lys Ile
           35                  40                  45
   Glu Glu Val Lys Lys Ile Thr Gly Glu Arg His Gly Lys Ile Val Arg
       50                  55                  60
   Ile Val Asp Val Glu Lys Val Glu Lys Lys Phe Leu Gly Lys Pro Ile
   65                  70                  75                  80
   Thr Val Trp Lys Leu Tyr Leu Glu His Pro Gln Asp Val Pro Thr Ile
                   85                  90                  95
   Arg Glu Lys Val Arg Glu His Pro Ala Val Val Asp Ile Phe Glu Tyr
               100                 105                 110
   Asp Ile Pro Phe Ala Lys Arg Tyr Leu Ile Asp Lys Gly Leu Ile Pro
           115                 120                 125
   Met Glu Gly Glu Glu Glu Leu Lys Ile Leu Ala Phe Asp Ile Glu Thr
       130                 135                 140
   Leu Tyr His Glu Gly Glu Glu Phe Gly Lys Gly Pro Ile Ile Met Ile
   145                 150                 155                 160
   Ser Tyr Ala Asp Glu Asn Glu Ala Lys Val Ile Thr Trp Lys Asn Ile
                   165                 170                 175
   Asp Leu Pro Tyr Val Glu Val Val Ser Ser Glu Arg Glu Met Ile Lys
               180                 185                 190
   Arg Phe Leu Arg Ile Ile Arg Glu Lys Asp Pro Asp Ile Ile Val Thr
           195                 200                 205
   Tyr Asn Gly Asp Ser Phe Asp Phe Pro Tyr Leu Ala Lys Arg Ala Glu
       210                 215                 220
   Lys Leu Gly Ile Lys Leu Thr Ile Gly Arg Asp Gly Ser Glu Pro Lys
   225                 230                 235                 240
   Met Gln Arg Ile Gly Asp Met Thr Ala Val Glu Val Lys Gly Arg Ile
                   245                 250                 255
   His Phe Asp Leu Tyr His Val Ile Thr Arg Thr Ile Asn Leu Pro Thr
               260                 265                 270
   Tyr Thr Leu Glu Ala Val Tyr Glu Ala Ile Phe Gly Lys Pro Lys Glu
           275                 280                 285
   Lys Val Tyr Ala Asp Glu Ile Ala Lys Ala Trp Glu Ser Gly Glu Asn
       290                 295                 300
   Leu Glu Arg Val Ala Lys Tyr Ser Met Glu Asp Ala Lys Ala Thr Tyr
   305                 310                 315                 320
   Glu Leu Gly Lys Glu Phe Leu Pro Met Glu Ile Gln Leu Ser Arg Leu
                   325                 330                 335
   Val Gly Gln Pro Leu Trp Asp Val Ser Arg Ser Ser Thr Gly Asn Leu
               340                 345                 350
   Val Glu Trp Phe Leu Leu Arg Lys Ala Tyr Glu Arg Asn Glu Val Ala
           355                 360                 365
   Pro Asn Lys Pro Ser Glu Glu Glu Tyr Gln Arg Arg Leu Arg Glu Ser
       370                 375                 380
   Tyr Thr Gly Gly Phe Val Lys Glu Pro Glu Lys Gly Leu Trp Glu Asn
   385                 390                 395                 400
   Ile Val Tyr Leu Asp Phe Arg Ala Leu Tyr Pro Ser Ile Ile Ile Thr
                   405                 410                 415
   His Asn Val Ser Pro Asp Thr Leu Asn Leu Glu Gly Cys Lys Asn Tyr
               420                 425                 430
   Asp Ile Ala Pro Gln Val Gly His Lys Phe Cys Lys Asp Ile Pro Gly
           435                 440                 445
   Phe Ile Pro Ser Leu Leu Gly His Leu Leu Glu Glu Arg Gln Lys Ile
       450                 455                 460
   Lys Thr Lys Met Lys Glu Thr Gln Asp Pro Ile Glu Lys Ile Leu Leu
   465                 470                 475                 480
   Asp Tyr Arg Gln Lys Ala Ile Lys Leu Leu Ala Asn Ser Phe Tyr Gly
                   485                 490                 495
   Tyr Tyr Gly Tyr Ala Lys Ala Arg Trp Tyr Cys Lys Glu Cys Ala Glu
   500 505 510
   Ser Val Thr Ala Trp Gly Arg Lys Tyr Ile Glu Leu Val Trp Lys Glu
   515 520 525
   Leu Glu Glu Lys Phe Gly Phe Lys Val Leu Tyr Ile Asp Thr Asp Gly
   530 535 540
   Leu Tyr Ala Thr Ile Pro Gly Gly Glu Ser Glu Glu Ile Lys Lys Lys
   545 550 555 560
   Ala Leu Glu Phe Val Lys Tyr Ile Asn Ser Lys Leu Pro Gly Leu Leu
   565 570 575
   Glu Leu Glu Tyr Glu Gly Phe Tyr Lys Arg Gly Phe Phe Val Thr Lys
   580 585 590
   Lys Arg Tyr Ala Val Ile Asp Glu Glu Gly Lys Val Ile Thr Arg Gly
   595 600 605
   Leu Glu Ile Val Arg Arg Asp Trp Ser Glu Ile Ala Lys Glu Thr Gln
   610 615 620
   Ala Arg Val Leu Glu Thr Ile Leu Lys His Gly Asp Val Glu Glu Ala
   625 630 635 640
   Val Arg Ile Val Lys Glu Val Ile Gln Lys Leu Ala Asn Tyr Glu Ile
   645 650 655
   Pro Pro Glu Lys Leu Ala Ile Tyr Glu Gln Ile Thr Arg Pro Leu His
   660 665 670
   Glu Tyr Lys Ala Ile Gly Pro His Val Ala Val Ala Lys Lys Leu Ala
   675 680 685
   Ala Lys Gly Val Lys Ile Lys Pro Gly Met Val Ile Gly Tyr Ile Val
   690 695 700
   Leu Arg Gly Asp Gly Pro Ile Ser Asn Arg Ala Ile Leu Ala Glu Glu
   705 710 715 720
   Tyr Asp Pro Lys Lys His Lys Tyr Asp Ala Glu Tyr Tyr Ile Glu Asn
   725 730 735
   Gln Val Leu Pro Ala Val Leu Arg Ile Leu Glu Gly Phe Gly Tyr Arg
   740 745 750
   Lys Glu Asp Leu Arg Tyr Gln Lys Thr Arg Gln Val Gly Leu Thr Ser
   755 760 765
   Trp Leu Asn Ile Lys Lys Ser Gly Thr His Ser Gln Lys Gln Leu Pro
   770 775 780
   Pro Val Lys Val Arg Asp Pro Thr Thr Gly Lys Glu Val Glu Leu Thr
   785 790 795 800
   Pro Ile Lys Val Trp Lys Leu Ser Pro Arg Gly Arg Arg Gly Val Lys
   805 810 815
   Ile Gly Leu Phe Lys Ser Pro Glu Thr Gly Lys Tyr Phe Arg Ala Lys
   820 825 830
   Val Pro Asp Asp Tyr Pro Glu Thr Gly
   835 840
<210> 19
   <211> 617
   <212> PRT
   <213> Artificial
<220>
   <223> Chimeric protein
<400> 19
   Met Ser Ser Gly Lys Lys Pro Val Lys Val Lys Thr Pro Ala Gly Lys
   1 5 10 15
   Glu Ala Glu Leu Val Pro Glu Lys Val Trp Ala Leu Ala Pro Lys Gly
   20 25 30
   Arg Lys Gly Val Lys Ile Gly Leu Phe Lys Asp Pro Glu Thr Gly Lys
   35 40 45
   Tyr Phe Arg His Lys Leu Pro Asp Asp Tyr Pro Ile Gly Thr His Met
   50 55 60
   Gly Leu Leu His Glu Phe Gly Leu Leu Glu Ser Pro Lys Ala Leu Glu
   65 70 75 80
   Glu Ala Pro Trp Pro Pro Pro Glu Gly Ala Phe Val Gly Phe Val Leu
   85 90 95
   Ser Arg Lys Glu Pro Met Trp Ala Asp Leu Leu Ala Leu Ala Ala Ala
   100 105 110
   Arg Gly Gly Arg Val His Arg Ala Pro Glu Pro Tyr Lys Ala Leu Arg
   115 120 125
   Asp Leu Lys Glu Ala Arg Gly Leu Leu Ala Lys Asp Leu Ser Val Leu
   130 135 140
   Ala Leu Arg Glu Gly Leu Gly Leu Pro Pro Gly Asp Asp Pro Met Leu
   145                 150                 155                 160
   Leu Ala Tyr Leu Leu Asp Pro Ser Asn Thr Thr Pro Glu Gly Val Ala
                   165                 170                 175
   Arg Arg Tyr Gly Gly Glu Trp Thr Glu Glu Ala Gly Glu Arg Ala Ala
               180                 185                 190
   Leu Ser Glu Arg Leu Phe Ala Asn Leu Trp Gly Arg Leu Glu Gly Glu
           195                 200                 205
   Glu Arg Leu Leu Trp Leu Tyr Arg Glu Val Glu Arg Pro Leu Ser Ala
       210                 215                 220
   Val Leu Ala His Met Glu Ala Thr Gly Val Arg Leu Asp Val Ala Tyr
   225                 230                 235                 240
   Leu Arg Ala Leu Ser Leu Glu Val Ala Glu Glu Ile Ala Arg Leu Glu
                   245                 250                 255
   Ala Glu Val Phe Arg Leu Ala Gly His Pro Phe Asn Leu Asn Ser Arg
               260                 265                 270
   Asp Gln Leu Glu Arg Val Leu Phe Asp Glu Leu Gly Leu Pro Ala Ile
           275                 280                 285
   Gly Lys Thr Glu Lys Thr Gly Lys Arg Ser Thr Ser Ala Ala Val Leu
       290                 295                 300
   Glu Ala Leu Arg Glu Ala His Pro Ile Val Glu Lys Ile Leu Gln Tyr
   305                 310                 315                 320
   Arg Glu Leu Thr Lys Leu Lys Ser Thr Tyr Ile Asp Pro Leu Pro Asp
                   325                 330                 335
   Leu Ile His Pro Arg Thr Gly Arg Leu His Thr Arg Phe Asn Gln Thr
               340                 345                 350
   Ala Thr Ala Thr Gly Arg Leu Ser Ser Ser Asp Pro Asn Leu Gln Asn
           355                 360                 365
   Ile Pro Val Arg Thr Pro Leu Gly Gln Arg Ile Arg Arg Ala Phe Ile
       370                 375                 380
   Ala Glu Glu Gly Trp Leu Leu Val Ala Leu Asp Tyr Ser Gln Ile Glu
   385                 390                 395                 400
   Leu Arg Val Leu Ala His Leu Ser Gly Asp Glu Asn Leu Ile Arg Val
                   405                 410                 415
   Phe Gln Glu Gly Arg Asp Ile His Thr Glu Thr Ala Ser Trp Met Phe
   420 425 430
   Gly Val Pro Arg Glu Ala Val Asp Pro Leu Met Arg Arg Ala Ala Lys
   435 440 445
   Thr Ile Asn Phe Gly Val Leu Tyr Gly Met Ser Ala His Arg Leu Ser
   450 455 460
   Gln Glu Leu Ala Ile Pro Tyr Glu Glu Ala Gln Ala Phe Ile Glu Arg
   465 470 475 480
   Tyr Phe Gln Ser Phe Pro Lys Val Arg Ala Trp Ile Glu Lys Thr Leu
   485 490 495
   Glu Glu Gly Arg Arg Arg Gly Tyr Val Glu Thr Leu Phe Gly Arg Arg
   500 505 510
   Arg Tyr Val Pro Asp Leu Glu Ala Arg Val Lys Ser Val Arg Glu Ala
   515 520 525
   Ala Glu Arg Met Ala Phe Asn Met Pro Val Gln Gly Thr Ala Ala Asp
   530 535 540
   Leu Met Lys Leu Ala Met Val Lys Leu Phe Pro Arg Leu Glu Glu Met
   545 550 555 560
   Gly Ala Arg Met Leu Leu Gln Val His Asp Glu Leu Val Leu Glu Ala
   565 570 575
   Pro Lys Glu Arg Ala Glu Ala Val Ala Arg Leu Ala Lys Glu Val Met
   580 585 590
   Glu Gly Val Tyr Pro Leu Ala Val Pro Leu Glu Val Glu Val Gly Ile
   595 600 605
   Gly Glu Asp Trp Leu Ser Ala Lys Glu
   610 615
<210> 20
   <211> 897
   <212> PRT
   <213> Artificial
<220>
   <223> chimeric protein
<400> 20
   Met Ser Ser Gly Lys Lys Pro Val Lys Val Lys Thr Pro Ala Gly Lys
   1 5 10 15
   Glu Ala Glu Leu Val Pro Glu Lys Val Trp Ala Leu Ala Pro Lys Gly
   20 25 30
   Arg Lys Gly Val Lys Ile Gly Leu Phe Lys Asp Pro Glu Thr Gly Lys
           35                  40                  45
   Tyr Phe Arg His Lys Leu Pro Asp Asp Tyr Pro Ile Gly Gly Val Thr
       50                  55                  60
   Met Asp Ser Gly Met Leu Pro Leu Phe Glu Pro Lys Gly Arg Val Leu
   65                  70                  75                  80
   Leu Val Asp Gly His His Leu Ala Tyr Arg Thr Phe His Ala Leu Lys
                   85                  90                  95
   Gly Leu Thr Thr Ser Arg Gly Glu Pro Val Gln Ala Val Tyr Gly Phe
               100                 105                 110
   Ala Lys Ser Leu Leu Lys Ala Leu Lys Glu Asp Gly Asp Ala Val Ile
           115                 120                 125
   Val Val Phe Asp Ala Lys Ala Pro Ser Phe Arg His Glu Ala Tyr Gly
       130                 135                 140
   Gly Tyr Lys Ala Gly Arg Ala Pro Thr Pro Glu Asp Phe Pro Arg Gln
   145                 150                 155                 160
   Leu Ala Leu Ile Lys Glu Leu Val Asp Leu Leu Gly Leu Ala Arg Leu
                   165                 170                 175
   Glu Val Pro Gly Tyr Glu Ala Asp Asp Val Leu Ala Ser Leu Ala Lys
               180                 185                 190
   Lys Ala Glu Lys Glu Gly Tyr Glu Val Arg Ile Leu Thr Ala Asp Lys
           195                 200                 205
   Asp Leu Tyr Gln Leu Leu Ser Asp Arg Ile His Val Leu His Pro Glu
       210                 215                 220
   Gly Tyr Leu Ile Thr Pro Ala Trp Leu Trp Glu Lys Tyr Gly Leu Arg
   225                 230                 235                 240
   Pro Asp Gln Trp Ala Asp Tyr Arg Ala Leu Thr Gly Asp Glu Ser Asp
                   245                 250                 255
   Asn Leu Pro Gly Val Lys Gly Ile Gly Glu Lys Thr Ala Arg Lys Leu
               260                 265                 270
   Leu Glu Glu Trp Gly Ser Leu Glu Ala Leu Leu Lys Asn Leu Asp Arg
           275                 280                 285
   Leu Lys Pro Ala Ile Arg Glu Lys Ile Leu Ala His Met Asp Asp Leu
       290                 295                 300
   Lys Leu Ser Trp Asp Leu Ala Lys Val Arg Thr Asp Leu Pro Leu Glu
   305 310 315 320
   Val Asp Phe Ala Lys Arg Arg Glu Pro Asp Arg Glu Arg Leu Arg Ala
   325 330 335
   Phe Leu Glu Arg Leu Glu Phe Gly Ser Leu Leu His Glu Phe Gly Leu
   340 345 350
   Leu Glu Ser Pro Lys Ala Leu Glu Glu Ala Pro Trp Pro Pro Pro Glu
   355 360 365
   Gly Ala Phe Val Gly Phe Val Leu Ser Arg Lys Glu Pro Met Trp Ala
   370 375 380
   Asp Leu Leu Ala Leu Ala Ala Ala Arg Gly Gly Arg Val His Arg Ala
   385 390 395 400
   Pro Glu Pro Tyr Lys Ala Leu Arg Asp Leu Lys Glu Ala Arg Gly Leu
   405 410 415
   Leu Ala Lys Asp Leu Ser Val Leu Ala Leu Arg Glu Gly Leu Gly Leu
   420 425 430
   Pro Pro Gly Asp Asp Pro Met Leu Leu Ala Tyr Leu Leu Asp Pro Ser
   435 440 445
   Asn Thr Thr Pro Glu Gly Val Ala Arg Arg Tyr Gly Gly Glu Trp Thr
   450 455 460
   Glu Glu Ala Gly Glu Arg Ala Ala Leu Ser Glu Arg Leu Phe Ala Asn
   465 470 475 480
   Leu Trp Gly Arg Leu Glu Gly Glu Glu Arg Leu Leu Trp Leu Tyr Arg
   485 490 495
   Glu Val Glu Arg Pro Leu Ser Ala Val Leu Ala His Met Glu Ala Thr
   500 505 510
   Gly Val Arg Leu Asp Val Ala Tyr Leu Arg Ala Leu Ser Leu Glu Val
   515 520 525
   Ala Glu Glu Ile Ala Arg Leu Glu Ala Glu Val Phe Arg Leu Ala Gly
   530 535 540
   His Pro Phe Asn Leu Asn Ser Arg Asp Gln Leu Glu Arg Val Leu Phe
   545 550 555 560
   Asp Glu Leu Gly Leu Pro Ala Ile Gly Lys Thr Glu Lys Thr Gly Lys
   565 570 575
   Arg Ser Thr Ser Ala Ala Val Leu Glu Ala Leu Arg Glu Ala His Pro
   580 585 590
   Ile Val Glu Lys Ile Leu Gln Tyr Arg Glu Leu Thr Lys Leu Lys Ser
   595 600 605
   Thr Tyr Ile Asp Pro Leu Pro Asp Leu Ile His Pro Arg Thr Gly Arg
   610 615 620
   Leu His Thr Arg Phe Asn Gln Thr Ala Thr Ala Thr Gly Arg Leu Ser
   625 630 635 640
   Ser Ser Asp Pro Asn Leu Gln Asn Ile Pro Val Arg Thr Pro Leu Gly
   645 650 655
   Gln Arg Ile Arg Arg Ala Phe Ile Ala Glu Glu Gly Trp Leu Leu Val
   660 665 670
   Ala Leu Asp Tyr Ser Gln Ile Glu Leu Arg Val Leu Ala His Leu Ser
   675 680 685
   Gly Asp Glu Asn Leu Ile Arg Val Phe Gln Glu Gly Arg Asp Ile His
   690 695 700
   Thr Glu Thr Ala Ser Trp Met Phe Gly Val Pro Arg Glu Ala Val Asp
   705 710 715 720
   Pro Leu Met Arg Arg Ala Ala Lys Thr Ile Asn Phe Gly Val Leu Tyr
   725 730 735
   Gly Met Ser Ala His Arg Leu Ser Gln Glu Leu Ala Ile Pro Tyr Glu
   740 745 750
   Glu Ala Gln Ala Phe Ile Glu Arg Tyr Phe Gln Ser Phe Pro Lys Val
   755 760 765
   Arg Ala Trp Ile Glu Lys Thr Leu Glu Glu Gly Arg Arg Arg Gly Tyr
   770 775 780
   Val Glu Thr Leu Phe Gly Arg Arg Arg Tyr Val Pro Asp Leu Glu Ala
   785 790 795 800
   Arg Val Lys Ser Val Arg Glu Ala Ala Glu Arg Met Ala Phe Asn Met
   805 810 815
   Pro Val Gln Gly Thr Ala Ala Asp Leu Met Lys Leu Ala Met Val Lys
   820 825 830
   Leu Phe Pro Arg Leu Glu Glu Met Gly Ala Arg Met Leu Leu Gln Val
   835 840 845
   His Asp Glu Leu Val Leu Glu Ala Pro Lys Glu Arg Ala Glu Ala Val
   850 855 860
   Ala Arg Leu Ala Lys Glu Val Met Glu Gly Val Tyr Pro Leu Ala Val
   865 870 875 880
   Pro Leu Glu Val Glu Val Gly Ile Gly Glu Asp Trp Leu Ser Ala Lys
   885 890 895
   Glu
<210> 21
   <211> 838
   <212> PRT
   <213> Artificial
<220>
   <223> Chimeric protein
<400> 21
   Met Ile Leu Asp Val Asp Tyr Ile Thr Glu Glu Gly Lys Pro Val Ile
   1 5 10 15
   Arg Leu Phe Lys Lys Glu Asn Gly Lys Phe Lys Ile Glu His Asp Arg
   20 25 30
   Thr Phe Arg Pro Tyr Ile Tyr Ala Leu Leu Arg Asp Asp Ser Lys Ile
   35 40 45
   Glu Glu Val Lys Lys Ile Thr Gly Glu Arg His Gly Lys Ile Val Arg
   50 55 60
   Ile Val Asp Val Glu Lys Val Glu Lys Lys Phe Leu Gly Lys Pro Ile
   65 70 75 80
   Thr Val Trp Lys Leu Tyr Leu Glu His Pro Gln Asp Val Pro Thr Ile
   85 90 95
   Arg Glu Lys Val Arg Glu His Pro Ala Val Val Asp Ile Phe Glu Tyr
   100 105 110
   Asp Ile Pro Phe Ala Lys Arg Tyr Leu Ile Asp Lys Gly Leu Ile Pro
   115 120 125
   Met Glu Gly Glu Glu Glu Leu Lys Ile Leu Ala Phe Asp Ile Glu Thr
   130 135 140
   Leu Tyr His Glu Gly Glu Glu Phe Gly Lys Gly Pro Ile Ile Met Ile
   145 150 155 160
   Ser Tyr Ala Asp Glu Asn Glu Ala Lys Val Ile Thr Trp Lys Asn Ile
   165 170 175
   Asp Leu Pro Tyr Val Glu Val Val Ser Ser Glu Arg Glu Met Ile Lys
   180 185 190
   Arg Phe Leu Arg Ile Ile Arg Glu Lys Asp Pro Asp Ile Ile Val Thr
   195 200 205
   Tyr Asn Gly Asp Ser Phe Asp Phe Pro Tyr Leu Ala Lys Arg Ala Glu
   210 215 220
   Lys Leu Gly Ile Lys Leu Thr Ile Gly Arg Asp Gly Ser Glu Pro Lys
   225 230 235 240
   Met Gln Arg Ile Gly Asp Met Thr Ala Val Glu Val Lys Gly Arg Ile
   245 250 255
   His Phe Asp Leu Tyr His Val Ile Thr Arg Thr Ile Asn Leu Pro Thr
   260 265 270
   Tyr Thr Leu Glu Ala Val Tyr Glu Ala Ile Phe Gly Lys Pro Lys Glu
   275 280 285
   Lys Val Tyr Ala Asp Glu Ile Ala Lys Ala Trp Glu Ser Gly Glu Asn
   290 295 300
   Leu Glu Arg Val Ala Lys Tyr Ser Met Glu Asp Ala Lys Ala Thr Tyr
   305 310 315 320
   Glu Leu Gly Lys Glu Phe Leu Pro Met Glu Ile Gln Leu Ser Arg Leu
   325 330 335
   Val Gly Gln Pro Leu Trp Asp Val Ser Arg Ser Ser Thr Gly Asn Leu
   340 345 350
   Val Glu Trp Phe Leu Leu Arg Lys Ala Tyr Glu Arg Asn Glu Val Ala
   355 360 365
   Pro Asn Lys Pro Ser Glu Glu Glu Tyr Gln Arg Arg Leu Arg Glu Ser
   370 375 380
   Tyr Thr Gly Gly Phe Val Lys Glu Pro Glu Lys Gly Leu Trp Glu Asn
   385 390 395 400
   Ile Val Tyr Leu Asp Phe Arg Ala Leu Tyr Pro Ser Ile Ile Ile Thr
   405 410 415
   His Asn Val Ser Pro Asp Thr Leu Asn Leu Glu Gly Cys Lys Asn Tyr
   420 425 430
   Asp Ile Ala Pro Gln Val Gly His Lys Phe Cys Lys Asp Ile Pro Gly
           435                 440                 445
   Phe Ile Pro Ser Leu Leu Gly His Leu Leu Glu Glu Arg Gln Lys Ile
       450                 455                 460
   Lys Thr Lys Met Lys Glu Thr Gln Asp Pro Ile Glu Lys Ile Leu Leu
   465                 470                 475                 480
   Asp Tyr Arg Gln Lys Ala Ile Lys Leu Leu Ala Asn Ser Phe Tyr Gly
                   485                 490                 495
   Tyr Tyr Gly Tyr Ala Lys Ala Arg Trp Tyr Cys Lys Glu Cys Ala Glu
               500                 505                 510
   Ser Val Thr Ala Trp Gly Arg Lys Tyr Ile Glu Leu Val Trp Lys Glu
           515                 520                 525
   Leu Glu Glu Lys Phe Gly Phe Lys Val Leu Tyr Ile Asp Thr Asp Gly
       530                 535                 540
   Leu Tyr Ala Thr Ile Pro Gly Gly Glu Ser Glu Glu Ile Lys Lys Lys
   545                 550                 555                 560
   Ala Leu Glu Phe Val Lys Tyr Ile Asn Ser Lys Leu Pro Gly Leu Leu
                   565                 570                 575
   Glu Leu Glu Tyr Glu Gly Phe Tyr Lys Arg Gly Phe Phe Val Thr Lys
               580                 585                 590
   Lys Arg Tyr Ala Val Ile Asp Glu Glu Gly Lys Val Ile Thr Arg Gly
           595                 600                 605
   Leu Glu Ile Val Arg Arg Asp Trp Ser Glu Ile Ala Lys Glu Thr Gln
       610                 615                 620
   Ala Arg Val Leu Glu Thr Ile Leu Lys His Gly Asp Val Glu Glu Ala
   625                 630                 635                 640
   Val Arg Ile Val Lys Glu Val Ile Gln Lys Leu Ala Asn Tyr Glu Ile
                   645                 650                 655
   Pro Pro Glu Lys Leu Ala Ile Tyr Glu Gln Ile Thr Arg Pro Leu His
               660                 665                 670
   Glu Tyr Lys Ala Ile Gly Pro His Val Ala Val Ala Lys Lys Leu Ala
           675                 680                 685
   Ala Lys Gly Val Lys Ile Lys Pro Gly Met Val Ile Gly Tyr Ile Val
       690                 695                 700
   Leu Arg Gly Asp Gly Pro Ile Ser Asn Arg Ala Ile Leu Ala Glu Glu
   705 710 715 720
   Tyr Asp Pro Lys Lys His Lys Tyr Asp Ala Glu Tyr Tyr Ile Glu Asn
   725 730 735
   Gln Val Leu Pro Ala Val Leu Arg Ile Leu Glu Gly Phe Gly Tyr Arg
   740 745 750
   Lys Glu Asp Leu Arg Tyr Gln Lys Thr Arg Gln Val Gly Leu Thr Ser
   755 760 765
   Trp Leu Asn Ile Lys Lys Ser Gly Thr His Met Ser Ser Gly Lys Lys
   770 775 780
   Pro Val Lys Val Lys Thr Pro Ala Gly Lys Glu Ala Glu Leu Val Pro
   785 790 795 800
   Glu Lys Val Trp Ala Leu Ala Pro Lys Gly Arg Lys Gly Val Lys Ile
   805 810 815
   Gly Leu Phe Lys Asp Pro Glu Thr Gly Lys Tyr Phe Arg His Lys Leu
   820 825 830
   Pro Asp Asp Tyr Pro Ile
   835
<210> 22
   <211> 616
   <212> PRT
   <213> Artificial
<220>
   <223> Chimeric protein
<400> 22
   Met Glu Glu Val Leu Asp Arg Glu Tyr Glu Val Glu Tyr Gly Gly Arg
   1 5 10 15
   Lys Tyr Arg Leu Lys Pro Val Lys Ala Trp Val Leu Gln Pro Pro Gly
   20 25 30
   Lys Pro Gly Val Val Ile Ala Leu Phe Lys Leu Pro Asp Gly Lys Thr
   35 40 45
   Ile Arg Lys Val Ile Met Lys Leu Pro Pro Ser Val Thr His Met Gly
   50 55 60
   Leu Leu His Glu Phe Gly Leu Leu Glu Ser Pro Lys Ala Leu Glu Glu
   65 70 75 80
   Ala Pro Trp Pro Pro Pro Glu Gly Ala Phe Val Gly Phe Val Leu Ser
   85 90 95
   Arg Lys Glu Pro Met Trp Ala Asp Leu Leu Ala Leu Ala Ala Ala Arg
               100                 105                 110
   Gly Gly Arg Val His Arg Ala Pro Glu Pro Tyr Lys Ala Leu Arg Asp
           115                 120                 125
   Leu Lys Glu Ala Arg Gly Leu Leu Ala Lys Asp Leu Ser Val Leu Ala
       130                 135                 140
   Leu Arg Glu Gly Leu Gly Leu Pro Pro Gly Asp Asp Pro Met Leu Leu
   145                 150                 155                 160
   Ala Tyr Leu Leu Asp Pro Ser Asn Thr Thr Pro Glu Gly Val Ala Arg
                   165                 170                 175
   Arg Tyr Gly Gly Glu Trp Thr Glu Glu Ala Gly Glu Arg Ala Ala Leu
               180                 185                 190
   Ser Glu Arg Leu Phe Ala Asn Leu Trp Gly Arg Leu Glu Gly Glu Glu
           195                 200                 205
   Arg Leu Leu Trp Leu Tyr Arg Glu Val Glu Arg Pro Leu Ser Ala Val
       210                 215                 220
   Leu Ala His Met Glu Ala Thr Gly Val Arg Leu Asp Val Ala Tyr Leu
   225                 230                 235                 240
   Arg Ala Leu Ser Leu Glu Val Ala Glu Glu Ile Ala Arg Leu Glu Ala
                   245                 250                 255
   Glu Val Phe Arg Leu Ala Gly His Pro Phe Asn Leu Asn Ser Arg Asp
               260                 265                 270
   Gln Leu Glu Arg Val Leu Phe Asp Glu Leu Gly Leu Pro Ala Ile Gly
           275                 280                 285
   Lys Thr Glu Lys Thr Gly Lys Arg Ser Thr Ser Ala Ala Val Leu Glu
       290                 295                 300
   Ala Leu Arg Glu Ala His Pro Ile Val Glu Lys Ile Leu Gln Tyr Arg
   305                 310                 315                 320
   Glu Leu Thr Lys Leu Lys Ser Thr Tyr Ile Asp Pro Leu Pro Asp Leu
                   325                 330                 335
   Ile His Pro Arg Thr Gly Arg Leu His Thr Arg Phe Asn Gln Thr Ala
               340                 345                 350
   Thr Ala Thr Gly Arg Leu Ser Ser Ser Asp Pro Asn Leu Gln Asn Ile
           355                 360                 365
   Pro Val Arg Thr Pro Leu Gly Gln Arg Ile Arg Arg Ala Phe Ile Ala
   370 375 380
   Glu Glu Gly Trp Leu Leu Val Ala Leu Asp Tyr Ser Gln Ile Glu Leu
   385 390 395 400
   Arg Val Leu Ala His Leu Ser Gly Asp Glu Asn Leu Ile Arg Val Phe
   405 410 415
   Gln Glu Gly Arg Asp Ile His Thr Glu Thr Ala Ser Trp Met Phe Gly
   420 425 430
   Val Pro Arg Glu Ala Val Asp Pro Leu Met Arg Arg Ala Ala Lys Thr
   435 440 445
   Ile Asn Phe Gly Val Leu Tyr Gly Met Ser Ala His Arg Leu Ser Gln
   450 455 460
   Glu Leu Ala Ile Pro Tyr Glu Glu Ala Gln Ala Phe Ile Glu Arg Tyr
   465 470 475 480
   Phe Gln Ser Phe Pro Lys Val Arg Ala Trp Ile Glu Lys Thr Leu Glu
   485 490 495
   Glu Gly Arg Arg Arg Gly Tyr Val Glu Thr Leu Phe Gly Arg Arg Arg
   500 505 510
   Tyr Val Pro Asp Leu Glu Ala Arg Val Lys Ser Val Arg Glu Ala Ala
   515 520 525
   Glu Arg Met Ala Phe Asn Met Pro Val Gln Gly Thr Ala Ala Asp Leu
   530 535 540
   Met Lys Leu Ala Met Val Lys Leu Phe Pro Arg Leu Glu Glu Met Gly
   545 550 555 560
   Ala Arg Met Leu Leu Gln Val His Asp Glu Leu Val Leu Glu Ala Pro
   565 570 575
   Lys Glu Arg Ala Glu Ala Val Ala Arg Leu Ala Lys Glu Val Met Glu
   580 585 590
   Gly Val Tyr Pro Leu Ala Val Pro Leu Glu Val Glu Val Gly Ile Gly
   595 600 605
   Glu Asp Trp Leu Ser Ala Lys Glu
   610 615
<210> 23
   <211> 899
   <212> PRT
   <213> Artificial
<220>
   <223> Chimeric protein
<400> 23
   Met Ala Cys Glu Lys Pro Val Lys Val Arg Asp Pro Thr Thr Gly Lys
   1 5 10 15
   Glu Val Glu Leu Val Pro Ile Lys Val Trp Gln Leu Ala Pro Arg Gly
   20 25 30
   Arg Lys Gly Val Lys Ile Gly Leu Phe Lys Ser Pro Glu Thr Gly Lys
   35 40 45
   Tyr Phe Arg Ala Lys Val Pro Asp Asp Tyr Pro Ile Cys Ser Gly Gly
   50 55 60
   Val Thr Met Asp Ser Gly Met Leu Pro Leu Phe Glu Pro Lys Gly Arg
   65 70 75 80
   Val Leu Leu Val Asp Gly His His Leu Ala Tyr Arg Thr Phe His Ala
   85 90 95
   Leu Lys Gly Leu Thr Thr Ser Arg Gly Glu Pro Val Gln Ala Val Tyr
   100 105 110
   Gly Phe Ala Lys Ser Leu Leu Lys Ala Leu Lys Glu Asp Gly Asp Ala
   115 120 125
   Val Ile Val Val Phe Asp Ala Lys Ala Pro Ser Phe Arg His Glu Ala
   130 135 140
   Tyr Gly Gly Tyr Lys Ala Gly Arg Ala Pro Thr Pro Glu Asp Phe Pro
   145 150 155 160
   Arg Gln Leu Ala Leu Ile Lys Glu Leu Val Asp Leu Leu Gly Leu Ala
   165 170 175
   Arg Leu Glu Val Pro Gly Tyr Glu Ala Asp Asp Val Leu Ala Ser Leu
   180 185 190
   Ala Lys Lys Ala Glu Lys Glu Gly Tyr Glu Val Arg Ile Leu Thr Ala
   195 200 205
   Asp Lys Asp Leu Tyr Gln Leu Leu Ser Asp Arg Ile His Val Leu His
   210 215 220
   Pro Glu Gly Tyr Leu Ile Thr Pro Ala Trp Leu Trp Glu Lys Tyr Gly
   225 230 235 240
   Leu Arg Pro Asp Gln Trp Ala Asp Tyr Arg Ala Leu Thr Gly Asp Glu
   245 250 255
   Ser Asp Asn Leu Pro Gly Val Lys Gly Ile Gly Glu Lys Thr Ala Arg
   260 265 270
   Lys Leu Leu Glu Glu Trp Gly Ser Leu Glu Ala Leu Leu Lys Asn Leu
   275 280 285
   Asp Arg Leu Lys Pro Ala Ile Arg Glu Lys Ile Leu Ala His Met Asp
   290 295 300
   Asp Leu Lys Leu Ser Trp Asp Leu Ala Lys Val Arg Thr Asp Leu Pro
   305 310 315 320
   Leu Glu Val Asp Phe Ala Lys Arg Arg Glu Pro Asp Arg Glu Arg Leu
   325 330 335
   Arg Ala Phe Leu Glu Arg Leu Glu Phe Gly Ser Leu Leu His Glu Phe
   340 345 350
   Gly Leu Leu Glu Ser Pro Lys Ala Leu Glu Glu Ala Pro Trp Pro Pro
   355 360 365
   Pro Glu Gly Ala Phe Val Gly Phe Val Leu Ser Arg Lys Glu Pro Met
   370 375 380
   Trp Ala Asp Leu Leu Ala Leu Ala Ala Ala Arg Gly Gly Arg Val His
   385 390 395 400
   Arg Ala Pro Glu Pro Tyr Lys Ala Leu Arg Asp Leu Lys Glu Ala Arg
   405 410 415
   Gly Leu Leu Ala Lys Asp Leu Ser Val Leu Ala Leu Arg Glu Gly Leu
   420 425 430
   Gly Leu Pro Pro Gly Asp Asp Pro Met Leu Leu Ala Tyr Leu Leu Asp
   435 440 445
   Pro Ser Asn Thr Thr Pro Glu Gly Val Ala Arg Arg Tyr Gly Gly Glu
   450 455 460
   Trp Thr Glu Glu Ala Gly Glu Arg Ala Ala Leu Ser Glu Arg Leu Phe
   465 470 475 480
   Ala Asn Leu Trp Gly Arg Leu Glu Gly Glu Glu Arg Leu Leu Trp Leu
   485 490 495
   Tyr Arg Glu Val Glu Arg Pro Leu Ser Ala Val Leu Ala His Met Glu
   500 505 510
   Ala Thr Gly Val Arg Leu Asp Val Ala Tyr Leu Arg Ala Leu Ser Leu
   515 520 525
   Glu Val Ala Glu Glu Ile Ala Arg Leu Glu Ala Glu Val Phe Arg Leu
   530 535 540
   Ala Gly His Pro Phe Asn Leu Asn Ser Arg Asp Gln Leu Glu Arg Val
   545 550 555 560
   Leu Phe Asp Glu Leu Gly Leu Pro Ala Ile Gly Lys Thr Glu Lys Thr
   565 570 575
   Gly Lys Arg Ser Thr Ser Ala Ala Val Leu Glu Ala Leu Arg Glu Ala
   580 585 590
   His Pro Ile Val Glu Lys Ile Leu Gln Tyr Arg Glu Leu Thr Lys Leu
   595 600 605
   Lys Ser Thr Tyr Ile Asp Pro Leu Pro Asp Leu Ile His Pro Arg Thr
   610 615 620
   Gly Arg Leu His Thr Arg Phe Asn Gln Thr Ala Thr Ala Thr Gly Arg
   625 630 635 640
   Leu Ser Ser Ser Asp Pro Asn Leu Gln Asn Ile Pro Val Arg Thr Pro
   645 650 655
   Leu Gly Gln Arg Ile Arg Arg Ala Phe Ile Ala Glu Glu Gly Trp Leu
   660 665 670
   Leu Val Ala Leu Asp Tyr Ser Gln Ile Glu Leu Arg Val Leu Ala His
   675 680 685
   Leu Ser Gly Asp Glu Asn Leu Ile Arg Val Phe Gln Glu Gly Arg Asp
   690 695 700
   Ile His Thr Glu Thr Ala Ser Trp Met Phe Gly Val Pro Arg Glu Ala
   705 710 715 720
   Val Asp Pro Leu Met Arg Arg Ala Ala Lys Thr Ile Asn Phe Gly Val
   725 730 735
   Leu Tyr Gly Met Ser Ala His Arg Leu Ser Gln Glu Leu Ala Ile Pro
   740 745 750
   Tyr Glu Glu Ala Gln Ala Phe Ile Glu Arg Tyr Phe Gln Ser Phe Pro
   755 760 765
   Lys Val Arg Ala Trp Ile Glu Lys Thr Leu Glu Glu Gly Arg Arg Arg
   770 775 780
   Gly Tyr Val Glu Thr Leu Phe Gly Arg Arg Arg Tyr Val Pro Asp Leu
   785 790 795 800
   Glu Ala Arg Val Lys Ser Val Arg Glu Ala Ala Glu Arg Met Ala Phe
   805 810 815
   Asn Met Pro Val Gln Gly Thr Ala Ala Asp Leu Met Lys Leu Ala Met
   820 825 830
   Val Lys Leu Phe Pro Arg Leu Glu Glu Met Gly Ala Arg Met Leu Leu
   835 840 845
   Gln Val His Asp Glu Leu Val Leu Glu Ala Pro Lys Glu Arg Ala Glu
   850 855 860
   Ala Val Ala Arg Leu Ala Lys Glu Val Met Glu Gly Val Tyr Pro Leu
   865 870 875 880
   Ala Val Pro Leu Glu Val Glu Val Gly Ile Gly Glu Asp Trp Leu Ser
   885 890 895
   Ala Lys Glu
<210> 24
   <211> 840
   <212> PRT
   <213> Artificial
<220>
   <223> Chimeric protein
<400> 24
   Met Ile Leu Asp Val Asp Tyr Ile Thr Glu Glu Gly Lys Pro Val Ile
   1 5 10 15
   Arg Leu Phe Lys Lys Glu Asn Gly Lys Phe Lys Ile Glu His Asp Arg
   20 25 30
   Thr Phe Arg Pro Tyr Ile Tyr Ala Leu Leu Arg Asp Asp Ser Lys Ile
   35 40 45
   Glu Glu Val Lys Lys Ile Thr Gly Glu Arg His Gly Lys Ile Val Arg
   50 55 60
   Ile Val Asp Val Glu Lys Val Glu Lys Lys Phe Leu Gly Lys Pro Ile
   65 70 75 80
   Thr Val Trp Lys Leu Tyr Leu Glu His Pro Gln Asp Val Pro Thr Ile
   85 90 95
   Arg Glu Lys Val Arg Glu His Pro Ala Val Val Asp Ile Phe Glu Tyr
   100 105 110
   Asp Ile Pro Phe Ala Lys Arg Tyr Leu Ile Asp Lys Gly Leu Ile Pro
   115 120 125
   Met Glu Gly Glu Glu Glu Leu Lys Ile Leu Ala Phe Asp Ile Glu Thr
   130 135 140
   Leu Tyr His Glu Gly Glu Glu Phe Gly Lys Gly Pro Ile Ile Met Ile
   145 150 155 160
   Ser Tyr Ala Asp Glu Asn Glu Ala Lys Val Ile Thr Trp Lys Asn Ile
   165 170 175
   Asp Leu Pro Tyr Val Glu Val Val Ser Ser Glu Arg Glu Met Ile Lys
   180 185 190
   Arg Phe Leu Arg Ile Ile Arg Glu Lys Asp Pro Asp Ile Ile Val Thr
   195 200 205
   Tyr Asn Gly Asp Ser Phe Asp Phe Pro Tyr Leu Ala Lys Arg Ala Glu
   210 215 220
   Lys Leu Gly Ile Lys Leu Thr Ile Gly Arg Asp Gly Ser Glu Pro Lys
   225 230 235 240
   Met Gln Arg Ile Gly Asp Met Thr Ala Val Glu Val Lys Gly Arg Ile
   245 250 255
   His Phe Asp Leu Tyr His Val Ile Thr Arg Thr Ile Asn Leu Pro Thr
   260 265 270
   Tyr Thr Leu Glu Ala Val Tyr Glu Ala Ile Phe Gly Lys Pro Lys Glu
   275 280 285
   Lys Val Tyr Ala Asp Glu Ile Ala Lys Ala Trp Glu Ser Gly Glu Asn
   290 295 300
   Leu Glu Arg Val Ala Lys Tyr Ser Met Glu Asp Ala Lys Ala Thr Tyr
   305 310 315 320
   Glu Leu Gly Lys Glu Phe Leu Pro Met Glu Ile Gln Leu Ser Arg Leu
   325 330 335
   Val Gly Gln Pro Leu Trp Asp Val Ser Arg Ser Ser Thr Gly Asn Leu
   340 345 350
   Val Glu Trp Phe Leu Leu Arg Lys Ala Tyr Glu Arg Asn Glu Val Ala
   355 360 365
   Pro Asn Lys Pro Ser Glu Glu Glu Tyr Gln Arg Arg Leu Arg Glu Ser
   370 375 380
   Tyr Thr Gly Gly Phe Val Lys Glu Pro Glu Lys Gly Leu Trp Glu Asn
   385 390 395 400
   Ile Val Tyr Leu Asp Phe Arg Ala Leu Tyr Pro Ser Ile Ile Ile Thr
   405 410 415
   His Asn Val Ser Pro Asp Thr Leu Asn Leu Glu Gly Cys Lys Asn Tyr
   420 425 430
   Asp Ile Ala Pro Gln Val Gly His Lys Phe Cys Lys Asp Ile Pro Gly
   435 440 445
   Phe Ile Pro Ser Leu Leu Gly His Leu Leu Glu Glu Arg Gln Lys Ile
   450 455 460
   Lys Thr Lys Met Lys Glu Thr Gln Asp Pro Ile Glu Lys Ile Leu Leu
   465 470 475 480
   Asp Tyr Arg Gln Lys Ala Ile Lys Leu Leu Ala Asn Ser Phe Tyr Gly
   485 490 495
   Tyr Tyr Gly Tyr Ala Lys Ala Arg Trp Tyr Cys Lys Glu Cys Ala Glu
   500 505 510
   Ser Val Thr Ala Trp Gly Arg Lys Tyr Ile Glu Leu Val Trp Lys Glu
   515 520 525
   Leu Glu Glu Lys Phe Gly Phe Lys Val Leu Tyr Ile Asp Thr Asp Gly
   530 535 540
   Leu Tyr Ala Thr Ile Pro Gly Gly Glu Ser Glu Glu Ile Lys Lys Lys
   545 550 555 560
   Ala Leu Glu Phe Val Lys Tyr Ile Asn Ser Lys Leu Pro Gly Leu Leu
   565 570 575
   Glu Leu Glu Tyr Glu Gly Phe Tyr Lys Arg Gly Phe Phe Val Thr Lys
   580 585 590
   Lys Arg Tyr Ala Val Ile Asp Glu Glu Gly Lys Val Ile Thr Arg Gly
   595 600 605
   Leu Glu Ile Val Arg Arg Asp Trp Ser Glu Ile Ala Lys Glu Thr Gln
   610 615 620
   Ala Arg Val Leu Glu Thr Ile Leu Lys His Gly Asp Val Glu Glu Ala
   625 630 635 640
   Val Arg Ile Val Lys Glu Val Ile Gln Lys Leu Ala Asn Tyr Glu Ile
   645 650 655
   Pro Pro Glu Lys Leu Ala Ile Tyr Glu Gln Ile Thr Arg Pro Leu His
   660 665 670
   Glu Tyr Lys Ala Ile Gly Pro His Val Ala Val Ala Lys Lys Leu Ala
   675 680 685
   Ala Lys Gly Val Lys Ile Lys Pro Gly Met Val Ile Gly Tyr Ile Val
   690 695 700
   Leu Arg Gly Asp Gly Pro Ile Ser Asn Arg Ala Ile Leu Ala Glu Glu
   705 710 715 720
   Tyr Asp Pro Lys Lys His Lys Tyr Asp Ala Glu Tyr Tyr Ile Glu Asn
   725 730 735
   Gln Val Leu Pro Ala Val Leu Arg Ile Leu Glu Gly Phe Gly Tyr Arg
   740 745 750
   Lys Glu Asp Leu Arg Tyr Gln Lys Thr Arg Gln Val Gly Leu Thr Ser
   755 760 765
   Trp Leu Asn Ile Lys Lys Ser Gly Thr His Met Ala Cys Glu Lys Pro
   770 775 780
   Val Lys Val Arg Asp Pro Thr Thr Gly Lys Glu Val Glu Leu Val Pro
   785 790 795 800
   Ile Lys Val Trp Gln Leu Ala Pro Arg Gly Arg Lys Gly Val Lys Ile
   805 810 815
   Gly Leu Phe Lys Ser Pro Glu Thr Gly Lys Tyr Phe Arg Ala Lys Val
   820 825 830
   Pro Asp Asp Tyr Pro Ile Cys Ser
   835 840
<210> 25
   <211> 1866
   <212> DNA
   <213> Artificial
<220>
   <223> cloned construct
<400> 25
   atgagccaga agcaactacc acctgtgaag gtcagggacc cgactacagg caaggaggtc 60
   gagctaacgc caatcaaagt gtggaagcta tcgccgaggg ggaggagggg cgtcaagata 120
   ggtctcttca agagccccga gacgggcaag tacttcaggg ccaaggtgcc cgacgactac 180
   cccgagaccg gggtacccca tatgggtctg ctgcacgaat tcggtctgct ggaatctccg 240
   aaagcgctgg aagaagcgcc gtggccgccg ccggaaggtg cgttcgttgg tttcgttctg 300
   tctcgtaaag aaccgatgtg ggcggacctg ctggcgctgg cggcggcgcg tggtggtcgt     360
   gttcaccgtg cgccggaacc ttataaagcc ctcagggacc tgaaggaggc gcgggggctt     420
   ctcgccaaag acctgagcgt tctggccctg agggaaggcc ttggcctccc gcccggcgac     480
   gaccccatgc tcctcgccta cctcctggac ccttccaaca ccacccccga gggggtggcc     540
   cggcgctacg gcggggagtg gacggaggag gcgggggagc gggccgccct ttccgagagg     600
   ctcttcgcca acctgtgggg gaggcttgag ggggaggaga ggctcctttg gctttaccgg   660
   gaggtggaga ggcccctttc cgctgtcctg gcccacatgg aggccacggg ggtgcgcctg     720
   gacgtggcct atctcagggc cttgtccctg gaggtggccg aggagatcgc ccgcctcgag     780
   gccgaggtct tccgcctggc cggccacccc ttcaacctca actcccggga ccagctggaa     840
   agggtcctct ttgacgagct agggcttccc gccatcggca agacggagaa gaccggcaag     900
   cgctccacca gcgccgccgt cctggaggcc ctccgcgagg cccaccccat cgtggagaag     960
   atcctgcagt accgggagct caccaagctg aagagcacct acattgaccc cttgccggac    1020
   ctcatccacc ccaggacggg ccgcctccac acccgcttca accagacggc cacggccacg    1080
   ggcaggctaa gtagctccga tcccaacctc cagaacatcc ccgtccgcac cccgcttggg    1140
   cagaggatcc gccgggcctt catcgccgag gaggggtggc tattggtggc cctggactat    1200
   agccagatag agctcagggt gctggcccac ctctccggcg acgagaacct gatccgggtc    1260
   ttccaggagg ggcgggacat ccacacggag accgccagct ggatgttcgg cgtcccccgg    1320
   gaggccgtgg accccctgat gcgccgggcg gccaagacca tcaacttcgg ggtcctctac   1380
   ggcatgtcgg cccaccgcct ctcccaggag ctagccatcc cttacgagga ggcccaggcc    1440
   ttcattgagc gctactttca gagcttcccc aaggtgcggg cctggattga gaagaccctg    1500
   gaggagggca ggaggcgggg gtacgtggag accctcttcg gccgccgccg ctacgtgcca    1560
   gacctagagg cccgggtgaa gagcgtgcgg gaggcggccg agcgcatggc cttcaacatg    1620
   cccgtccagg gcaccgccgc cgacctcatg aagctggcta tggtgaagct cttccccagg    1680
   ctggaggaaa tgggggccag gatgctcctt caggtccacg acgagctggt cctcgaggcc    1740
   ccaaaagaga gggcggaggc cgtggcccgg ctggccaagg aggtcatgga gggggtgtat    1800
   cccctggccg tgcccctgga ggtggaggtg gggatagggg aggactggct ctccgccaag    1860
   gagtga                                                               1866
<210> 26
   <211> 2706
   <212> DNA
   <213> Artificial
<220>
   <223> cloned construct
<400> 26
   atgagccaga agcaactacc acctgtgaag gtcagggacc cgactacagg caaggaggtc       60
   gagctaacgc caatcaaagt gtggaagcta tcgccgaggg ggaggagggg cgtcaagata      120
   ggtctcttca agagccccga gacgggcaag tacttcaggg ccaaggtgcc cgacgactac    180
   cccgagaccg ggggtggtgt taccatgaat tcggggatgc tgcccctctt tgagcccaag    240
   ggccgggtcc tcctggtgga cggccaccac ctggcctacc gcaccttcca cgccctgaag    300
   ggcctcacca ccagccgggg ggagccggtg caggcggtct acggcttcgc caagagcctc    360
   ctcaaggccc tcaaggagga cggggacgcg gtgatcgtgg tctttgacgc caaggccccc    420
   tccttccgcc acgaggccta cggggggtac aaggcgggcc gggcccccac gccggaggac    480
   tttccccggc aactcgccct catcaaggag ctggtggacc tcctggggct ggcgcgcctc    540
   gaggtcccgg gctacgaggc ggacgacgtc ctggccagcc tggccaagaa ggcggaaaag    600
   gagggctacg aggtccgcat cctcaccgcc gacaaagacc tttaccagct cctttccgac    660
   cgcatccacg tcctccaccc cgaggggtac ctcatcaccc cggcctggct ttgggaaaag    720
   tacggcctga ggcccgacca gtgggccgac taccgggccc tgaccgggga cgagtccgac    780
   aaccttcccg gggtcaaggg catcggggag aagacggcga ggaagcttct ggaggagtgg    840
   gggagcctgg aagccctcct caagaacctg gaccggctga agcccgccat ccgggagaag    900
   atcctggccc acatggacga tctgaagctc tcctgggacc tggccaaggt gcgcaccgac    960
   ctgcccctgg aggtggactt cgccaaaagg cgggagcccg accgggagag gcttagggcc   1020
   tttctggaga ggcttgagtt tggcagcctc ctccacgagt tcggccttct ggaaagcccc   1080
   aaggccctgg aggaggcccc ctggcccccg ccggaagggg ccttcgtggg ctttgtgctt   1140
   tcccgcaagg agcccatgtg ggccgatctt ctggccctgg ccgccgccag ggggggccgg   1200
   gtccaccggg cccccgagcc ttataaagcc ctcagggacc tgaaggaggc gcgggggctt   1260
   ctcgccaaag acctgagcgt tctggccctg agggaaggcc ttggcctccc gcccggcgac   1320
   gaccccatgc tcctcgccta cctcctggac ccttccaaca ccacccccga gggggtggcc   1380
   cggcgctacg gcggggagtg gacggaggag gcgggggagc gggccgccct ttccgagagg   1440
   ctcttcgcca acctgtgggg gaggcttgag ggggaggaga ggctcctttg gctttaccgg     1500
   gaggtggaga ggcccctttc cgctgtcctg gcccacatgg aggccacggg ggtgcgcctg   1560
   gacgtggcct atctcagggc cttgtccctg gaggtggccg aggagatcgc ccgcctcgag   1620
   gccgaggtct tccgcctggc cggccacccc ttcaacctca actcccggga ccagctggaa   1680
   agggtcctct ttgacgagct agggcttccc gccatcggca agacggagaa gaccggcaag   1740
   cgctccacca gcgccgccgt cctggaggcc ctccgcgagg cccaccccat cgtggagaag   1800
   atcctgcagt accgggagct caccaagctg aagagcacct acattgaccc cttgccggac   1860
   ctcatccacc ccaggacggg ccgcctccac acccgcttca accagacggc cacggccacg   1920
   ggcaggctaa gtagctccga tcccaacctc cagaacatcc ccgtccgcac cccgcttggg   1980
   cagaggatcc gccgggcctt catcgccgag gaggggtggc tattggtggc cctggactat   2040
   agccagatag agctcagggt gctggcccac ctctccggcg acgagaacct gatccgggtc   2100
   ttccaggagg ggcgggacat ccacacggag accgccagct ggatgttcgg cgtcccccgg   2160
   gaggccgtgg accccctgat gcgccgggcg gccaagacca tcaacttcgg ggtcctctac 2220
   ggcatgtcgg cccaccgcct ctcccaggag ctagccatcc cttacgagga ggcccaggcc 2280
   ttcattgagc gctactttca gagcttcccc aaggtgcggg cctggattga gaagaccctg 2340
   gaggagggca ggaggcgggg gtacgtggag accctcttcg gccgccgccg ctacgtgcca 2400
   gacctagagg cccgggtgaa gagcgtgcgg gaggcggccg agcgcatggc cttcaacatg 2460
   cccgtccagg gcaccgccgc cgacctcatg aagctggcta tggtgaagct cttccccagg 2520
   ctggaggaaa tgggggccag gatgctcctt caggtccacg acgagctggt cctcgaggcc 2580
   ccaaaagaga gggcggaggc cgtggcccgg ctggccaagg aggtcatgga gggggtgtat 2640
   cccctggccg tgcccctgga ggtggaggtg gggatagggg aggactggct ctccgccaag 2700
   gagtga 2706
<210> 27
   <211> 2526
   <212> DNA
   <213> Artificial
<220>
   <223> cloned construct
<400> 27
   atgattttag atgtggatta cataactgaa gaaggaaaac ctgttattag gctattcaaa 60
   aaagagaacg gaaaatttaa gatagagcat gatagaactt ttagaccata catttacgct 120
   cttctcaggg atgattcaaa gattgaagaa gttaagaaaa taacggggga aaggcatgga 180
   aagattgtga gaattgttga tgtagagaag gttgagaaaa agtttctcgg caagcctatt 240
   accgtgtgga aactttattt ggaacatccc caagatgttc ccactattag agaaaaagtt 300
   agagaacatc cagcagttgt ggacatcttc gaatacgata ttccatttgc aaagagatac 360
   ctcatcgaca aaggcctaat accaatggag ggggaagaag agctaaagat tcttgccttc 420
   gatatagaaa ccctctatca cgaaggagaa gagtttggaa aaggcccaat tataatgatt 480
   agttatgcag atgaaaatga agcaaaggtg attacttgga aaaacataga tcttccatac 540
   gttgaggttg tatcaagcga gagagagatg ataaagagat ttctcaggat tatcagggag 600
   aaggatcctg acattatagt tacttataat ggagactcat tcgacttccc atatttagcg 660
   aaaagggcag aaaaacttgg gattaaatta accattggaa gagatggaag cgagcccaag 720
   atgcagagaa taggcgatat gacggctgta gaagtcaagg gaagaataca tttcgacttg 780
   tatcatgtaa taacaaggac aataaatctc ccaacataca cactagaggc tgtatatgaa 840
   gcaatttttg gaaagccaaa ggagaaggta tacgccgacg agatagcaaa agcctgggaa 900
   agtggagaga accttgagag agttgccaaa tactcgatgg aagatgcaaa ggcaacttat 960
   gaactcggga aagaattcct tccaatggaa attcagcttt caagattagt tggacaacct 1020
   ttatgggatg tttcaaggtc aagcacaggg aaccttgtag agtggttctt acttaggaaa 1080
   gcctacgaaa gaaacgaagt agctccaaac aagccaagtg aagaggagta tcaaagaagg 1140
   ctcagggaga gctacacagg tggattcgtt aaagagccag aaaaggggtt gtgggaaaac 1200
   atagtatacc tagattttag agccctatat ccctcgatta taattaccca caatgtttct 1260
   cccgatactc taaatcttga gggatgcaag aactatgata tcgctcctca agtaggccac 1320
   aagttctgca aggacatccc tggttttata ccaagtctct tgggacattt gttagaggaa 1380
   agacaaaaga ttaagacaaa aatgaaggaa actcaagatc ctatagaaaa aatactcctt 1440
   gactatagac aaaaagcgat aaaactctta gcaaattctt tctacggata ttatggctat 1500
   gcaaaagcaa gatggtactg taaggagtgt gctgagagcg ttactgcctg gggaagaaag 1560
   tacatcgagt tagtatggaa ggagctcgaa gaaaagtttg gatttaaagt cctctacatt 1620
   gacactgatg gtctctatgc aactatccca ggaggagaaa gtgaggaaat aaagaaaaag 1680
   gctctagaat ttgtaaaata cataaattca aagctccctg gactgctaga gcttgaatat 1740
   gaagggtttt ataagagggg attcttcgtt acgaagaaga ggtatgcagt aatagatgaa 1800
   gaaggaaaag tcattactcg tggtttagag atagttagga gagattggag tgaaattgca 1860
   aaagaaactc aagctagagt tttggagaca atactaaaac acggagatgt tgaagaagct 1920
   gtgagaatag taaaagaagt aatacaaaag cttgccaatt atgaaattcc accagagaag 1980
   ctcgcaatat atgagcagat aacaagacca ttacatgagt ataaggcgat aggtcctcac 2040
   gtagctgttg caaagaaact agctgctaaa ggagttaaaa taaagccagg aatggtaatt 2100
   ggatacatag tacttagagg cgatggtcca attagcaata gggcaattct agctgaggaa 2160
   tacgatccca aaaagcacaa gtatgacgca gaatattaca ttgagaacca ggttcttcca 2220
   gcggtactta ggatattgga gggatttgga tacagaaagg aagacctcag ataccaaaag 2280
   acaagacaag tcggcctaac ttcctggctt aacattaaaa aatccggtac ccatagccag 2340
   aagcaactac cacctgtgaa ggtcagggac ccgactacag gcaaggaggt cgagctaacg 2400
   ccaatcaaag tgtggaagct atcgccgagg gggaggaggg gcgtcaagat aggtctcttc 2460
   aagagccccg agacgggcaa gtacttcagg gccaaggtgc ccgacgacta ccccgagacc 2520
   gggtaa                                                            2526
<210> 28
   <211> 1854
   <212> DNA
   <213> Artificial
<220>
   <223> cloned construct
<400> 28
   atgagttcgg gtaaaaaacc agtaaaagta aaaacaccag ctggtaaaga ggctgaattg 60
   gttccagaaa aagtatgggc attagcacca aagggtagaa aaggtgtaaa gataggttta 120
   tttaaagatc cagaaactgg gaaatacttc agacataagc taccagatga ttatccaata 180
   ggtacccata tgggtctgct gcacgaattc ggtctgctgg aatctccgaa agcgctggaa 240
   gaagcgccgt ggccgccgcc ggaaggtgcg ttcgttggtt tcgttctgtc tcgtaaagaa 300
   ccgatgtggg cggacctgct ggcgctggcg gcggcgcgtg gtggtcgtgt tcaccgtgcg 360
   ccggaacctt ataaagccct cagggacctg aaggaggcgc gggggcttct cgccaaagac 420
   ctgagcgttc tggccctgag ggaaggcctt ggcctcccgc ccggcgacga ccccatgctc 480
   ctcgcctacc tcctggaccc ttccaacacc acccccgagg gggtggcccg gcgctacggc 540
   ggggagtgga cggaggaggc gggggagcgg gccgcccttt ccgagaggct cttcgccaac 600
   ctgtggggga ggcttgaggg ggaggagagg ctcctttggc tttaccggga ggtggagagg 660
   cccctttccg ctgtcctggc ccacatggag gccacggggg tgcgcctgga cgtggcctat 720
   ctcagggcct tgtccctgga ggtggccgag gagatcgccc gcctcgaggc cgaggtcttc 780
   cgcctggccg gccacccctt caacctcaac tcccgggacc agctggaaag ggtcctcttt 840
   gacgagctag ggcttcccgc catcggcaag acggagaaga ccggcaagcg ctccaccagc 900
   gccgccgtcc tggaggccct ccgcgaggcc caccccatcg tggagaagat cctgcagtac 960
   cgggagctca ccaagctgaa gagcacctac attgacccct tgccggacct catccacccc 1020
   aggacgggcc gcctccacac ccgcttcaac cagacggcca cggccacggg caggctaagt 1080
   agctccgatc ccaacctcca gaacatcccc gtccgcaccc cgcttgggca gaggatccgc 1140
   cgggccttca tcgccgagga ggggtggcta ttggtggccc tggactatag ccagatagag 1200
   ctcagggtgc tggcccacct ctccggcgac gagaacctga tccgggtctt ccaggagggg 1260
   cgggacatcc acacggagac cgccagctgg atgttcggcg tcccccggga ggccgtggac 1320
   cccctgatgc gccgggcggc caagaccatc aacttcgggg tcctctacgg catgtcggcc 1380
   caccgcctct cccaggagct agccatccct tacgaggagg cccaggcctt cattgagcgc 1440
   tactttcaga gcttccccaa ggtgcgggcc tggattgaga agaccctgga ggagggcagg 1500
   aggcgggggt acgtggagac cctcttcggc cgccgccgct acgtgccaga cctagaggcc 1560
   cgggtgaaga gcgtgcggga ggcggccgag cgcatggcct tcaacatgcc cgtccagggc 1620
   accgccgccg acctcatgaa gctggctatg gtgaagctct tccccaggct ggaggaaatg 1680
   ggggccagga tgctccttca ggtccacgac gagctggtcc tcgaggcccc aaaagagagg 1740
   gcggaggccg tggcccggct ggccaaggag gtcatggagg gggtgtatcc cctggccgtg 1800
   cccctggagg tggaggtggg gataggggag gactggctct ccgccaagga gtga 1854
<210> 29
   <211> 2694
   <212> DNA
   <213> Artificial
<220>
   <223> cloned construct
<400> 29
   atgagttcgg gtaaaaaacc agtaaaagta aaaacaccag ctggtaaaga ggctgaattg 60
   gttccagaaa aagtatgggc attagcacca aagggtagaa aaggtgtaaa gataggttta 120
   tttaaagatc cagaaactgg gaaatacttc agacataagc taccagatga ttatccaata 180
   ggtggtgtta ccatgaattc ggggatgctg cccctctttg agcccaaggg ccgggtcctc 240
   ctggtggacg gccaccacct ggcctaccgc accttccacg ccctgaaggg cctcaccacc 300
   agccgggggg agccggtgca ggcggtctac ggcttcgcca agagcctcct caaggccctc 360
   aaggaggacg gggacgcggt gatcgtggtc tttgacgcca aggccccctc cttccgccac 420
   gaggcctacg gggggtacaa ggcgggccgg gcccccacgc cggaggactt tccccggcaa 480
   ctcgccctca tcaaggagct ggtggacctc ctggggctgg cgcgcctcga ggtcccgggc 540
   tacgaggcgg acgacgtcct ggccagcctg gccaagaagg cggaaaagga gggctacgag 600
   gtccgcatcc tcaccgccga caaagacctt taccagctcc tttccgaccg catccacgtc 660
   ctccaccccg aggggtacct catcaccccg gcctggcttt gggaaaagta cggcctgagg 720
   cccgaccagt gggccgacta ccgggccctg accggggacg agtccgacaa ccttcccggg 780
   gtcaagggca tcggggagaa gacggcgagg aagcttctgg aggagtgggg gagcctggaa 840
   gccctcctca agaacctgga ccggctgaag cccgccatcc gggagaagat cctggcccac 900
   atggacgatc tgaagctctc ctgggacctg gccaaggtgc gcaccgacct gcccctggag 960
   gtggacttcg ccaaaaggcg ggagcccgac cgggagaggc ttagggcctt tctggagagg 1020
   cttgagtttg gcagcctcct ccacgagttc ggccttctgg aaagccccaa ggccctggag 1080
   gaggccccct ggcccccgcc ggaaggggcc ttcgtgggct ttgtgctttc ccgcaaggag 1140
   cccatgtggg ccgatcttct ggccctggcc gccgccaggg ggggccgggt ccaccgggcc 1200
   cccgagcctt ataaagccct cagggacctg aaggaggcgc gggggcttct cgccaaagac 1260
   ctgagcgttc tggccctgag ggaaggcctt ggcctcccgc ccggcgacga ccccatgctc 1320
   ctcgcctacc tcctggaccc ttccaacacc acccccgagg gggtggcccg gcgctacggc 1380
   ggggagtgga cggaggaggc gggggagcgg gccgcccttt ccgagaggct cttcgccaac     1440
   ctgtggggga ggcttgaggg ggaggagagg ctcctttggc tttaccggga ggtggagagg     1500
   cccctttccg ctgtcctggc ccacatggag gccacggggg tgcgcctgga cgtggcctat 1560
   ctcagggcct tgtccctgga ggtggccgag gagatcgccc gcctcgaggc cgaggtcttc 1620
   cgcctggccg gccacccctt caacctcaac tcccgggacc agctggaaag ggtcctcttt 1680
   gacgagctag ggcttcccgc catcggcaag acggagaaga ccggcaagcg ctccaccagc 1740
   gccgccgtcc tggaggccct ccgcgaggcc caccccatcg tggagaagat cctgcagtac 1800
   cgggagctca ccaagctgaa gagcacctac attgacccct tgccggacct catccacccc 1860
   aggacgggcc gcctccacac ccgcttcaac cagacggcca cggccacggg caggctaagt 1920
   agctccgatc ccaacctcca gaacatcccc gtccgcaccc cgcttgggca gaggatccgc 1980
   cgggccttca tcgccgagga ggggtggcta ttggtggccc tggactatag ccagatagag 2040
   ctcagggtgc tggcccacct ctccggcgac gagaacctga tccgggtctt ccaggagggg 2100
   cgggacatcc acacggagac cgccagctgg atgttcggcg tcccccggga ggccgtggac 2160
   cccctgatgc gccgggcggc caagaccatc aacttcgggg tcctctacgg catgtcggcc 2220
   caccgcctct cccaggagct agccatccct tacgaggagg cccaggcctt cattgagcgc     2280
   tactttcaga gcttccccaa ggtgcgggcc tggattgaga agaccctgga ggagggcagg     2340
   aggcgggggt acgtggagac cctcttcggc cgccgccgct acgtgccaga cctagaggcc     2400
   cgggtgaaga gcgtgcggga ggcggccgag cgcatggcct tcaacatgcc cgtccagggc     2460
   accgccgccg acctcatgaa gctggctatg gtgaagctct tccccaggct ggaggaaatg     2520
   ggggccagga tgctccttca ggtccacgac gagctggtcc tcgaggcccc aaaagagagg     2580
   gcggaggccg tggcccggct ggccaaggag gtcatggagg gggtgtatcc cctggccgtg     2640
   cccctggagg tggaggtggg gataggggag gactggctct ccgccaagga gtga           2694
<210> 30
   <211> 2517
   <212> DNA
   <213> Artificial
<220>
   <223> cloned construct
<400> 30
<210> 31
   <211> 1851
   <212> DNA
   <213> Artificial
<220>
   <223> cloned construct
<400> 31
   atggaagagg tcttagatcg tgaatacgaa gtggaatacg gcgggagaaa ataccggcta 60
   aagccagtta aagcatgggt tctccagccc cctggcaaac caggtgtcgt catagccctc 120
   tttaaactac cagatggaaa aactattagg aaggtgataa tgaaattgcc gcctagcgtt 180
   acccatatgg gtctgctgca cgaattcggt ctgctggaat ctccgaaagc gctggaagaa 240
   gcgccgtggc cgccgccgga aggtgcgttc gttggtttcg ttctgtctcg taaagaaccg 300
   atgtgggcgg acctgctggc gctggcggcg gcgcgtggtg gtcgtgttca ccgtgcgccg 360
   gaaccttata aagccctcag ggacctgaag gaggcgcggg ggcttctcgc caaagacctg 420
   agcgttctgg ccctgaggga aggccttggc ctcccgcccg gcgacgaccc catgctcctc 480
   gcctacctcc tggacccttc caacaccacc cccgaggggg tggcccggcg ctacggcggg 540
   gagtggacgg aggaggcggg ggagcgggcc gccctttccg agaggctctt cgccaacctg 600
   tgggggaggc ttgaggggga ggagaggctc ctttggcttt accgggaggt ggagaggccc 660
   ctttccgctg tcctggccca catggaggcc acgggggtgc gcctggacgt ggcctatctc 720
   agggccttgt ccctggaggt ggccgaggag atcgcccgcc tcgaggccga ggtcttccgc 780
   ctggccggcc accccttcaa cctcaactcc cgggaccagc tggaaagggt cctctttgac 840
   gagctagggc ttcccgccat cggcaagacg gagaagaccg gcaagcgctc caccagcgcc 900
   gccgtcctgg aggccctccg cgaggcccac cccatcgtgg agaagatcct gcagtaccgg 960
   gagctcacca agctgaagag cacctacatt gaccccttgc cggacctcat ccaccccagg 1020
   acgggccgcc tccacacccg cttcaaccag acggccacgg ccacgggcag gctaagtagc 1080
   tccgatccca acctccagaa catccccgtc cgcaccccgc ttgggcagag gatccgccgg 1140
   gccttcatcg ccgaggaggg gtggctattg gtggccctgg actatagcca gatagagctc 1200
   agggtgctgg cccacctctc cggcgacgag aacctgatcc gggtcttcca ggaggggcgg 1260
   gacatccaca cggagaccgc cagctggatg ttcggcgtcc cccgggaggc cgtggacccc 1320
   ctgatgcgcc gggcggccaa gaccatcaac ttcggggtcc tctacggcat gtcggcccac 1380
   cgcctctccc aggagctagc catcccttac gaggaggccc aggccttcat tgagcgctac 1440
   tttcagagct tccccaaggt gcgggcctgg attgagaaga ccctggagga gggcaggagg 1500
   cgggggtacg tggagaccct cttcggccgc cgccgctacg tgccagacct agaggcccgg 1560
   gtgaagagcg tgcgggaggc ggccgagcgc atggccttca acatgcccgt ccagggcacc 1620
   gccgccgacc tcatgaagct ggctatggtg aagctcttcc ccaggctgga ggaaatgggg 1680
   gccaggatgc tccttcaggt ccacgacgag ctggtcctcg aggccccaaa agagagggcg 1740
   gaggccgtgg cccggctggc caaggaggtc atggaggggg tgtatcccct ggccgtgccc 1800
   ctggaggtgg aggtggggat aggggaggac tggctctccg ccaaggagtg a 1851
<210> 32
   <211> 2700
   <212> DNA
   <213> Artificial
<220>
   <223> cloned construct
<400> 32
   atggcgtgtg agaagcctgt taaggttcgt gaccctacta ctggtaagga ggtagagctg 60
   gtaccaatca aggtgtggca gctagcaccc aggggtagga agggcgtcaa gataggccta 120
   ttcaagagcc ccgaaacagg caagtacttc agagccaagg taccagacga ctacccaatc 180
   tgcagcggtg gtgttaccat gaattcgggg atgctgcccc tctttgagcc caagggccgg 240
   gtcctcctgg tggacggcca ccacctggcc taccgcacct tccacgccct gaagggcctc 300
   accaccagcc ggggggagcc ggtgcaggcg gtctacggct tcgccaagag cctcctcaag 360
   gccctcaagg aggacgggga cgcggtgatc gtggtctttg acgccaaggc cccctccttc 420
   cgccacgagg cctacggggg gtacaaggcg ggccgggccc ccacgccgga ggactttccc 480
   cggcaactcg ccctcatcaa ggagctggtg gacctcctgg ggctggcgcg cctcgaggtc 540
   ccgggctacg aggcggacga cgtcctggcc agcctggcca agaaggcgga aaaggagggc 600
   tacgaggtcc gcatcctcac cgccgacaaa gacctttacc agctcctttc cgaccgcatc 660
   cacgtcctcc accccgaggg gtacctcatc accccggcct ggctttggga aaagtacggc 720
   ctgaggcccg accagtgggc cgactaccgg gccctgaccg gggacgagtc cgacaacctt 780
   cccggggtca agggcatcgg ggagaagacg gcgaggaagc ttctggagga gtgggggagc 840
   ctggaagccc tcctcaagaa cctggaccgg ctgaagcccg ccatccggga gaagatcctg 900
   gcccacatgg acgatctgaa gctctcctgg gacctggcca aggtgcgcac cgacctgccc 960
   ctggaggtgg acttcgccaa aaggcgggag cccgaccggg agaggcttag ggcctttctg 1020
   gagaggcttg agtttggcag cctcctccac gagttcggcc ttctggaaag ccccaaggcc 1080
   ctggaggagg ccccctggcc cccgccggaa ggggccttcg tgggctttgt gctttcccgc 1140
   aaggagccca tgtgggccga tcttctggcc ctggccgccg ccaggggggg ccgggtccac 1200
   cgggcccccg agccttataa agccctcagg gacctgaagg aggcgcgggg gcttctcgcc 1260
   aaagacctga gcgttctggc cctgagggaa ggccttggcc tcccgcccgg cgacgacccc 1320
   atgctcctcg cctacctcct ggacccttcc aacaccaccc ccgagggggt ggcccggcgc 1380
   tacggcgggg agtggacgga ggaggcgggg gagcgggccg ccctttccga gaggctcttc 1440
   gccaacctgt gggggaggct tgagggggag gagaggctcc tttggcttta ccgggaggtg 1500
   gagaggcccc tttccgctgt cctggcccac atggaggcca cgggggtgcg cctggacgtg 1560
   gcctatctca gggccttgtc cctggaggtg gccgaggaga tcgcccgcct cgaggccgag 1620
   gtcttccgcc tggccggcca ccccttcaac ctcaactccc gggaccagct ggaaagggtc 1680
   ctctttgacg agctagggct tcccgccatc ggcaagacgg agaagaccgg caagcgctcc 1740
   accagcgccg ccgtcctgga ggccctccgc gaggcccacc ccatcgtgga gaagatcctg 1800
   cagtaccggg agctcaccaa gctgaagagc acctacattg accccttgcc ggacctcatc 1860
   caccccagga cgggccgcct ccacacccgc ttcaaccaga cggccacggc cacgggcagg 1920
   ctaagtagct ccgatcccaa cctccagaac atccccgtcc gcaccccgct tgggcagagg 1980
   atccgccggg ccttcatcgc cgaggagggg tggctattgg tggccctgga ctatagccag 2040
   atagagctca gggtgctggc ccacctctcc ggcgacgaga acctgatccg ggtcttccag 2100
   gaggggcggg acatccacac ggagaccgcc agctggatgt tcggcgtccc ccgggaggcc 2160
   gtggaccccc tgatgcgccg ggcggccaag accatcaact tcggggtcct ctacggcatg 2220
   tcggcccacc gcctctccca ggagctagcc atcccttacg aggaggccca ggccttcatt 2280
   gagcgctact ttcagagctt ccccaaggtg cgggcctgga ttgagaagac cctggaggag 2340
   ggcaggaggc gggggtacgt ggagaccctc ttcggccgcc gccgctacgt gccagaccta 2400
   gaggcccggg tgaagagcgt gcgggaggcg gccgagcgca tggccttcaa catgcccgtc 2460
   cagggcaccg ccgccgacct catgaagctg gctatggtga agctcttccc caggctggag 2520
   gaaatggggg ccaggatgct ccttcaggtc cacgacgagc tggtcctcga ggccccaaaa 2580
   gagagggcgg aggccgtggc ccggctggcc aaggaggtca tggagggggt gtatcccctg 2640
   gccgtgcccc tggaggtgga ggtggggata ggggaggact ggctctccgc caaggagtga 2700
<210> 33
   <211> 2523
   <212> DNA
   <213> Artificial
<220>
   <223> cloned construct
<400> 33
   atgattttag atgtggatta cataactgaa gaaggaaaac ctgttattag gctattcaaa 60
   aaagagaacg gaaaatttaa gatagagcat gatagaactt ttagaccata catttacgct     120
   cttctcaggg atgattcaaa gattgaagaa gttaagaaaa taacggggga aaggcatgga     180
   aagattgtga gaattgttga tgtagagaag gttgagaaaa agtttctcgg caagcctatt 240
   accgtgtgga aactttattt ggaacatccc caagatgttc ccactattag agaaaaagtt 300
   agagaacatc cagcagttgt ggacatcttc gaatacgata ttccatttgc aaagagatac 360
   ctcatcgaca aaggcctaat accaatggag ggggaagaag agctaaagat tcttgccttc 420
   gatatagaaa ccctctatca cgaaggagaa gagtttggaa aaggcccaat tataatgatt 480
   agttatgcag atgaaaatga agcaaaggtg attacttgga aaaacataga tcttccatac 540
   gttgaggttg tatcaagcga gagagagatg ataaagagat ttctcaggat tatcagggag 600
   aaggatcctg acattatagt tacttataat ggagactcat tcgacttccc atatttagcg 660
   aaaagggcag aaaaacttgg gattaaatta accattggaa gagatggaag cgagcccaag 720
   atgcagagaa taggcgatat gacggctgta gaagtcaagg gaagaataca tttcgacttg 780
   tatcatgtaa taacaaggac aataaatctc ccaacataca cactagaggc tgtatatgaa 840
   gcaatttttg gaaagccaaa ggagaaggta tacgccgacg agatagcaaa agcctgggaa 900
   agtggagaga accttgagag agttgccaaa tactcgatgg aagatgcaaa ggcaacttat 960
   gaactcggga aagaattcct tccaatggaa attcagcttt caagattagt tggacaacct 1020
   ttatgggatg tttcaaggtc aagcacaggg aaccttgtag agtggttctt acttaggaaa 1080
   gcctacgaaa gaaacgaagt agctccaaac aagccaagtg aagaggagta tcaaagaagg 1140
   ctcagggaga gctacacagg tggattcgtt aaagagccag aaaaggggtt gtgggaaaac 1200
   atagtatacc tagattttag agccctatat ccctcgatta taattaccca caatgtttct 1260
   cccgatactc taaatcttga gggatgcaag aactatgata tcgctcctca agtaggccac 1320
   aagttctgca aggacatccc tggttttata ccaagtctct tgggacattt gttagaggaa 1380
   agacaaaaga ttaagacaaa aatgaaggaa actcaagatc ctatagaaaa aatactcctt 1440
   gactatagac aaaaagcgat aaaactctta gcaaattctt tctacggata ttatggctat      1500
   gcaaaagcaa gatggtactg taaggagtgt gctgagagcg ttactgcctg gggaagaaag      1560
   tacatcgagt tagtatggaa ggagctcgaa gaaaagtttg gatttaaagt cctctacatt      1620
   gacactgatg gtctctatgc aactatccca ggaggagaaa gtgaggaaat aaagaaaaag      1680
   gctctagaat ttgtaaaata cataaattca aagctccctg gactgctaga gcttgaatat      1740
   gaagggtttt ataagagggg attcttcgtt acgaagaaga ggtatgcagt aatagatgaa      1800
   gaaggaaaag tcattactcg tggtttagag atagttagga gagattggag tgaaattgca      1860
   aaagaaactc aagctagagt tttggagaca atactaaaac acggagatgt tgaagaagct      1920
   gtgagaatag taaaagaagt aatacaaaag cttgccaatt atgaaattcc accagagaag      1980
   ctcgcaatat atgagcagat aacaagacca ttacatgagt ataaggcgat aggtcctcac      2040
   gtagctgttg caaagaaact agctgctaaa ggagttaaaa taaagccagg aatggtaatt      2100
   ggatacatag tacttagagg cgatggtcca attagcaata gggcaattct agctgaggaa      2160
   tacgatccca aaaagcacaa gtatgacgca gaatattaca ttgagaacca ggttcttcca      2220
   gcggtactta ggatattgga gggatttgga tacagaaagg aagacctcag ataccaaaag      2280
   acaagacaag tcggcctaac ttcctggctt aacattaaaa aatccggtac ccatatggcg      2340
   tgtgagaagc ctgttaaggt tcgtgaccct actactggta aggaggtaga gctggtacca      2400
   atcaaggtgt ggcagctagc acccaggggt aggaagggcg tcaagatagg cctattcaag      2460
   agccccgaaa caggcaagta cttcagagcc aaggtaccag acgactaccc aatctgcagc      2520
   taa                                                                    2523
<210> 34
   <211> 30
   <212> DNA
   <213> Artificial
<220>
   <223> Primer sequence
<400> 34
   gatatccata tgagccagaa gcaactacca 30
<210> 35
   <211> 38
   <212> DNA
   <213> Artificial
<220>
   <223> Primer sequence
<400> 35
   gaattccata tgggtacccc cggtctcggg gtagtcgt 38
<210> 36
   <211> 29
   <212> DNA
   <213> Artificial
<220>
   <223> Primer sequence
<400> 36
   gatatccata tgagttcggg taaaaaacc 29
<210> 37
   <211> 38
   <212> DNA
   <213> Artificial
<220>
   <223> Primer sequence
<400> 37
   gaattccata tgggtaccta ttggataatc atctggta 38
<210> 38
   <211> 30
   <212> DNA
   <213> Artificial
<220>
   <223> Primer sequence
<400> 38
   gatatccata tggaagaggt cttagatcgt 30
<210> 39
   <211> 38
   <212> DNA
   <213> Artificial
<220>
   <223> Priemr sequence
<400> 39
   gaattccata tgggtaacgc taggcggcaa tttcatta 38
<210> 40
   <211> 45
   <212> DNA
   <213> Artificial
<220>
   <223> Primer sequence
<400> 40
   atccccgaat tcatggtaac accacccccg gtctcggggt agtcg 45
<210> 41
   <211> 4
   <212> PRT
   <213> Artificial
<220>
   <223> Linker peptide
<400> 41
   Gly Gly Val Thr
   1
<210> 42
   <211> 45
   <212> DNA
   <213> Artificial
<220>
   <223> Primer sequence
<400> 42
   atccccgaat tcatggtaac accacctatt ggataatcat ctggt 45
<210> 43
   <211> 30
   <212> DNA
   <213> Artificial
<220>
   <223> Primer sequence
<400> 43
   gatatccata tggcgtgtga gaagcctgtt 30
<210> 44
   <211> 45
   <212> DNA
   <213> Artificial
<220>
   <223> Primer sequence
<400> 44
   atccccgaat tcatggtaac accaccgctg cagattgggt agtcg 45
<210> 45
   <211> 30
   <212> DNA
   <213> Artificial
<220>
   <223> Primer sequence
<400> 45
   gaattcggta cccatagcca gaagcaacta 30
<210> 46
   <211> 30
   <212> DNA
   <213> Artificial
<220>
   <223> Primer sequence
<400> 46
   gaattcgtcg acttacccgg tctcggggta 30
<210> 47
   <211> 30
   <212> DNA
   <213> Artificial
<220>
   <223> Primer sequence
<400> 47
   gaattcggta cccatatgag ttcgggtaaa 30
<210> 48
   <211> 30
   <212> DNA
   <213> Artificial
<220>
   <223> Primer sequence
<400> 48
   gaattcgtcg acttatattg gataatcatc 30
<210> 49
   <211> 30
   <212> DNA
   <213> Artificial
<220>
   <223> Primer sequence
<400> 49
   gaattcggta cccatatggc gtgtgagaag 30
<210> 50
   <211> 30
   <212> DNA
   <213> Artificial
<220>
   <223> Primer sequence
<400> 50
   gaattcgtcg acttagctgc agattgggta 30
<210> 51
   <211> 20
   <212> DNA
   <213> Artificial
<220>
   <223> Primer sequence
<400> 51
   cctgctctgc cgcttcacgc 20
<210> 52
   <211> 28
   <212> DNA
   <213> Artificial
<220>
   <223> Primer sequence
<400> 52
   tccggataaa aacgtcgatg acatttgc 28
<210> 53
   <211> 28
   <212> DNA
   <213> Artificial
<220>
   <223> Primer sequence
<400> 53
   gatgacgcat cctcacgata atatccgg 28
<210> 54
   <211> 24
   <212> DNA
   <213> Artificial
<220>
   <223> Primer sequence
<400> 54
   ccatgattca gtgtgcccgt ctgg 24
<210> 55
   <211> 23
   <212> DNA
   <213> Artificial
<220>
   <223> Primer sequence
<400> 55
   cgaacgtcgc gcagagaaac agg 23
<210> 56
   <211> 23
   <212> DNA
   <213> Artificial
<220>
   <223> Primer sequence
<400> 56
   gcctcgttgc gtttgtttgc acg 23
<210> 57
   <211> 27
   <212> DNA
   <213> Artificial
<220>
   <223> Primer sequence
<400> 57
   gcacagaagc tattatgcgt ccccagg 27
<210> 58
   <211> 26
   <212> DNA
   <213> Artificial
<220>
   <223> Primer sequence
<400> 58
   tcttcctcgt gcatcgagct attcgg 26
<210> 59
   <211> 60
   <212> PRT
   <213> Sulfolobus shibatae
<400> 59
   Met Ser Ser Gly Lys Lys Ala Val Lys Val Lys Thr Pro Ala Gly Lys
   1               5                   10                  15
   Glu Ala Glu Leu Val Pro Glu Lys Val Trp Ala Leu Ala Pro Lys Gly
               20                  25                  30
   Arg Lys Gly Val Lys Ile Gly Leu Phe Lys Asp Pro Glu Thr Gly Lys
           35                  40                  45
   Tyr Phe Arg His Lys Leu Pro Asp Asp Tyr Pro Ile
       50                  55                  60
<210> 60
   <211> 57
   <212> PRT
   <213> Sulfolobus tokodaii
<400> 60
   Met Ala Glu Lys Lys Val Lys Val Lys Thr Pro Ser Gly Lys Glu Ala
   1               5                   10                  15
   Glu Leu Ala Pro Glu Lys Val Trp Val Leu Ala Pro Lys Gly Arg Lys
               20                  25                  30
   Gly Val Lys Ile Gly Leu Phe Lys Asp Pro Glu Thr Gly Lys Tyr Phe
           35                  40                  45
   Arg His Lys Leu Pro Asp Asp Tyr Pro
       50                  55
<210> 61
   <211> 45
   <212> PRT
   <213> Artificial
<220>
   <223> Consensus sequence
<220>
   <221> misc_feature
   <222> (1)..(1)
   <223> Xaa can be Lys, Arg or Glu
<220>
   <221> misc_feature
   <222> (2)..(4)
   <223> Xaa can be any naturally occurring amino acid
<220>
   <221> misc_feature
   <222> (5)..(5)
   <223> Xaa can be any naturally occurring amino acid or no amino acid
<220>
   <221> misc_feature
   <222> (7)..(10)
   <223> Xaa can be any naturally occurring amino acid
<220>
   <221> misc_feature
   <222> (11)..(11)
   <223> Xaa can be Leu or no amino acid
<220>
   <221> misc_feature
   <222> (12)..(12)
   <223> Xaa can be any naturally occurring amino acid
<220>
   <221> misc_feature
   <222> (14)..(14)
   <223> Xaa can be any naturally occurring amino acid
<220>
   <221> misc_feature
   <222> (16)..(16)
   <223> Xaa can be Ala, val or Thr
<220>
   <221> misc_feature
   <222> (18)..(18)
   <223> Xaa can be any naturally occurring amino acid
<220>
   <221> misc_feature
   <222> (20)..(20)
   <223> xaa can be any naturally occurring amino acid
<220>
   <221> misc_feature
   <222> (22)..(22)
   <223> Xaa can be any naturally occurring amino acid
<220>
   <221> misc_feature
   <222> (24)..(24)
   <223> Xaa can be Lys or Arg
<220>
   <221> misc_feature
   <222> (25)..(25)
   <223> Xaa can be any naturally occurring amino acid
<220>
   <221> misc_feature
   <222> (28)..(28)
   <223> xaa can be any naturally occurring amino acid
<220>
   <221> misc_feature
   <222> (29)..(29)
   <223> Xaa can be Ile or Val
<220>
   <221> misc-feature
   <222> (30)..(30)
   <223> Xaa can be Gly or Ala
<220>
   <221> misc_feature
   <222> (33)..(33)
   <223> Xaa can be Lys, Arg or Gln
<220>
   <221> misc_feature
   <222> (34)..(34)
   <223> xaa can be any naturally occurring amino acid
<220>
   <221> misc_feature
   <222> (36)..(36)
   <223> Xaa can be Asp, Asn or Glu
<220>
   <221> misc_feature
   <222> (37)..(37)
   <223> xaa can be any naturally occurring amino acid or no amino acid
<220>
   <221> misc_feature
   <222> (39)..(39)
   <223> xaa can be Lys or His
<220>
   <221> misc_feature
   <222> (40)..(40)
   <223> xaa can be any naturally occurring amino acid
<220>
   <221> misc_feature
   <222> (41)..(41)
   <223> Xaa can be Ile, Val or Phe
<220>
   <221> misc_feature
   <222> (43)..(44)
   <223> xaa can be any naturally occurring amino acid
<220>
   <221> misc_feature
   <222> (45)..(45)
   <223> Xaa can be Ile, Val or Leu
<400> 61
   Xaa Xaa Xaa Xaa Xaa Gly Xaa Xaa Xaa Xaa Xaa Xaa Pro Xaa Lys Xaa
   1               5                   10                  15
   Trp Xaa Leu Xaa Pro Xaa Gly Xaa Xaa Gly Val Xaa Xaa Xaa Leu Phe
               20                  25                  30
   Xaa Xaa Pro Xaa Xaa Gly Xaa Xaa Xaa Arg Xaa Xaa Xaa
           35                  40                  45
<210> 62
   <211> 28
   <212> DNA
   <213> Artificial
<220>
   <223> Primer sequence
<400> 62
   ccactgtaaa gctaacttag cattaacc 28
<210> 63
   <211> 28
   <212> DNA
   <213> Artificial
<220>
   <223> Primer sequence
<400> 63
   gtgatgagga atagtgtaag gagtatgg 28
<210> 64
   <211> 29
   <212> DNA
   <213> Artificial
<220>
   <223> PCR probe sequence
<400> 64
   ccaacacctc tttacagtga aatgcccca 29